# EUROPÄISCHE PATENTSCHRIFT

(11) **EP 2 570 036 B1**
(45) Veröffentlichungstag und Bekanntmachung des Hinweises auf die Patenterteilung: **18.06.2014**
(21) Anmeldenummer: 11181511.4
(22) Anmeldetag: 15.09.2011
(51) Int. Cl.: A23L 1/226, A23L 1/22, C07C 59/48, C07C 59/64, C07D 311/00

(54) **Verwendung bestimmter Neoflavonoide zur Verstärkung und/oder Erzeugung eines süßen sensorischen Eindruckes**
Use of particular neoflavanoids for reinforcing and/or generating a sweet sensory sensation
Utilisation de néoflavonoïdes précis pour le renforcement et/ou la production d'une impression sensorielle sucrée

(43) Veröffentlichungstag der Anmeldung: 20.03.2013
(73) Patentinhaber: Symrise AG, 37603 Holzminden (DE)
(72) Erfinder: Backes, Dr. Michael, 37603 Holzminden (DE); Vössing, Tobias, 37688 Beverungen (DE); Ley, Dr. Jakob Peter, 37603 Holzminden (DE); Paetz, Susanne, 37671 Höxter (DE)
(74) Vertreter: Eisenführ Speiser

(56) Entgegenhaltungen:
- EP-A1- 2 298 084
- DE-A1- 2 701 280
- US-A1- 2008 176 912
- US-A1- 2008 305 052
- US-A1- 2010 233 102
- ROELENS F ET AL: "Regioselective synthesis and estrogenicity of (+/-)-8-alkyl-5,7-dihydroxy-4-(4-hydroxyph enyl)-3,4-dihydrocoumarins", EUROPEAN JOURNAL OF MEDICINAL CHEMISTRY, EDITIONS SCIENTIFIQUE ELSEVIER, PARIS, FR, Bd. 40, Nr. 10, 1. Oktober 2005 (2005-10-01), Seiten 1042-1051, XP027857667, ISSN: 0223-5234 [gefunden am 2005-10-01]

## Beschreibung

Die vorliegende Erfindung betrifft primär die Verwendung einer oder mehrerer Neoflavonoide der nachfolgend definierten Formel **(I)** und/oder eines oder mehrerer physiologisch akzeptabler Salze eines oder mehrerer Neoflavonoide der nachfolgend definierten Formel **(I)** zur Erzeugung eines süßen sensorischen Eindruckes (d.h eines Süßeindruckes) in einer oral konsumierbaren Zubereitung oder zur Verstärkung des süßen sensorischen Eindruckes einer oral konsumierbaren Zubereitung umfassend mindestens einen weiteren süß schmeckenden Stoff. Ferner betrifft die vorliegende Erfindung bestimmte Halbfertigwaren und bestimmte oral konsumierbare Zubereitungen enthaltend ein oder mehrere Neoflavonoide der Formel **(I)** und/oder ein oder mehrere physiologisch akzeptable Salze eines oder mehrerer Neoflavonoide der Formel **(I).** Schließlich betrifft die Erfindung auch ein Verfahren zum Herstellen einer oral konsumierbaren Zubereitung sowie ein Verfahren zum Erzeugen und/oder Verstärken eines süßen sensorischen Eindruckes (in) einer oral konsumierbaren Zubereitung.

Nahrungs- oder Genussmittel, die einen hohen Zuckergehalt (vor allem Sucrose (= Saccharose), Lactose, Glucose oder Fructose oder deren Mischungen) aufweisen, werden in der Regel von Verbrauchern auf Grund der Süße stark präferiert. Auf der anderen Seite ist allgemein bekannt, dass ein hoher Gehalt an leicht verstoffwechselbaren Kohlenhydraten den Blutzuckerspiegel stark ansteigen lässt, zur Bildung von Fettdepots führt und letztendlich zu gesundheitlichen Problemen wie Übergewicht, Fettleibigkeit, Insulinresistenz, Altersdiabetes und deren Folgeerkrankungen führen kann. Insbesondere kommt erschwerend hinzu, dass viele der oben genannten Kohlenhydrate zusätzlich die Zahngesundheit beeinträchtigen können, da sie von bestimmten Bakteriensorten in der Mundhöhle zu beispielsweise Milchsäure abgebaut werden und den Zahnschmelz der juvenilen oder adulten Zähne angreifen können (Karies).

Daher ist es schon lange ein Ziel, den Zuckergehalt von Nahrungs- und/oder Genussmitteln so weit wie möglich zu reduzieren, wobei bevorzugtes Ziel ist, diese Reduktion mit möglichst geringer Verringerung des Süßeindruckes zu erreichen. Eine entsprechende Maßnahme besteht im Einsatz von Süßstoffen: das sind chemisch einheitliche Substanzen, die selbst keinen oder nur einen sehr geringen kalorischen Brennwert haben und gleichzeitig einen starken süßen Geschmackseindruck verursachen; die Stoffe sind in der Regel nicht-kariogen (eine Übersicht ist z.B. zu finden in Journal of the American Dietetic Association 2004, 104 (2), 255-275). Die sogenannten Bulk-Süßstoffe wie Sorbitol, Mannitol oder andere Zuckeralkohole sind zwar teilweise ausgezeichnete Süßungsmittel und können auch die übrigen nahrungsmitteltechnischen Eigenschaften von Zuckern teilweise ersetzen, führen aber bei zu häufiger Aufnahme bei einem Anteil der Bevölkerung zu osmotisch bedingten Verdauungsproblemen. Die nicht-nutritiven, hochintensiven Süßstoffe sind durch ihre geringe Einsatzkonzentration gut geeignet, Süße in Nahrungsmittel zu bringen. Eine Reihe dieser Süßstoffe sind jedoch nicht natürlichen Ursprungs (Sucralose, Cyclamat, Acesulfam K, Saccharin, Aspartam), zeigen geschmackliche Probleme durch im Vergleich zu Zucker unähnliche Zeit-Intensitätsprofile (z.B. Sucralose, Stevioside, Cyclamat), einen bitteren und/oder adstringierenden Nachgeschmack (z.B. Acesulfam K, Saccharin, Stevioside, Rebaudioside), ausgeprägte zusätzliche Aromaeindrücke (z.B. Glycerrhyzinsäureammoniumsalz). Einige der Süßstoffe sind gegenüber Hitze nicht besonders stabil (z.B. Thaumatin, Brazzein, Monellin), sind nicht in allen Anwendungen stabil (z.B. Aspartam) und sind teilweise sehr langanhaltend in ihrer süßen Wirkung (starker süßer Nachgeschmack, z.B. Saccharin, Sucralose, Stevioside, Rebaudioside, Neotam, Advantam, Superaspartam).

Daher ist es wünschenswert (Süß)Stoffe zu finden, die einen intensiven, dem Rohrzucker ähnlichen Süßgeschmack aufweisen bzw. vermitteln und darüberhinaus vorzugsweise in oral konsumierbaren Zubereitungen stabil und/oder breit anwendbar sind.

Eine andere Möglichkeit den kalorischen Gehalt von Nahrungsmitteln oder Getränken zu senken - ohne den Einsatz von nicht-nutritiven Süßstoffen - besteht darin, den Zuckergehalt von Nahrungs- und/oder Genussmitteln zu reduzieren und sensorisch schwach oder nicht wahrnehmbare Stoffe zuzusetzen, die die Süße mittelbar oder unmittelbar verstärken, wie z.B. in WO 2005/041684 beschrieben.

In EP 1 291 342 werden solche Stoffe natürlicher Herkunft (Pyridinium-Betaine) beschrieben; allerdings wird durch sie nicht selektiv der süße Geschmack, sondern auch andere Geschmacksrichtungen wie Umami oder Salzigkeit beeinflusst. Zudem sind die offenbarten Stoffe nur mit hohem Aufwand zu reinigen und/oder schwierig synthetisch herzustellen.

In WO 2007/014879 A1 wird der Einsatz von Hesperetin und in WO 2007/107596 A1 wird Phloretin als Verstärker des süßen Geschmacks von Zucker-reduzierten, der Ernährung oder dem Genuss dienenden Zubereitungen empfohlen. Bisweilen nachteilig ist allerdings bei Einsatz von Hesperetin und Phloretin die vergleichsweise schwach ausgeprägte Süßverstärkung in Nahrungs- und Genussmitteln, die hohe Anteile an Proteinen, insbesondere denaturierte Proteine oder Polysaccharide enthalten wie z.B. Joghurt-Produkte. Hesperetin zeigt zudem den Nachteil, in sehr sauren und carbonisierten Anwendungen wie Limonaden oder Cola-Getränken nicht ausreichend wirksam zu sein.

Daher ist es weiterhin wünschenswert, eine Möglichkeit zu finden, bei gleichbleibendem Süßeindruck den Gehalt an süßen Verbindungen, insbesondere süß schmeckenden Verbindungen in oral konsumierbaren Zubereitungen senken zu können, so dass Zuckerreduzierte Zubereitungen entstehen. Insbesondere besteht ein Bedarf an Mitteln, die einen gegebenen Süßeindruck verstärken, insbesondere auch über einen rein additiven Effekt hinaus.

Dementsprechend war es die primäre Aufgabe der vorliegenden Erfindung, Stoffe (einzelne Substanzen oder Substanzgemische) zu finden, die einen Süßeindruck erzeugen können (d.h. eine Eigensüße aufweisen) und/oder den Süßeindruck anderer süßer Stoffe verstärken können.

Ferner sollten entsprechende oral konsumierbare Zubereitungen angegeben werden, bei denen der Süßeindruck durch diese Stoffe erzeugt wird oder deren Süßeindruck durch diese Stoffe verstärkt wird bei weitgehend unveränderter Konzentration an weiteren süßen Verbindungen, insbesondere süß schmeckenden Verbindungen oder bei denen bei gleichbleibendem Süßeindruck der Gehalt an weiteren süßen Geschmackseindruck erzeugenden Verbindungen verringert ist, wobei bevorzugt keine oder lediglich in geringfügigem Ausmaß negative sensorische Nebeneffekte auftreten sollten.

Insbesondere sollten die gesuchten Süßeindruck vermittelnden oder süßeindruckverstärkenden Stoffe vorzugsweise bereits in geringen Konzentrationen wirksam sein. Ferner sollten die gesuchten Stoffe möglichst breit einsetzbar sein, d.h. in viele verschiedene oral anwendbare Applikations- und Produktformen eingearbeitet werden können und entsprechend mit vielen verschiedenen oral konsumierbaren Grund-, Hilfs-, Träger-, Zusatz- und/oder Wirkstoffen kombinierbar und verträglich sein.

Die primäre Aufgabe der vorliegenden Erfindung wird gelöst durch die Verwendung eines, zweier oder mehrerer verschiedener Verbindungen der Formel **(I)** 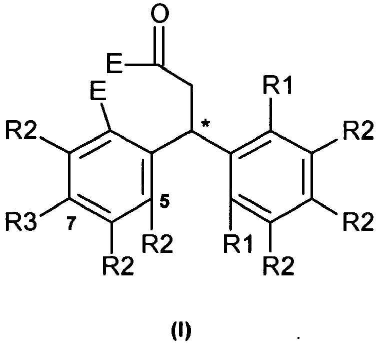 (Anmerkung: Die Ziffern "5" und "7" kennzeichnen die Positionen C5 bzw. C7 der Verbindung.)
oder
eines, zweier oder mehrerer verschiedener physiologisch akzeptabler Salze eines, zweier oder mehrerer verschiedener Verbindungen der Formel **(I),**
oder
eines Gemisches eines, zweier oder mehrerer verschiedener Verbindungen der Formel **(I)** mit einem, zwei oder mehreren verschiedenen physiologisch akzeptablen Salzen eines, zweier oder mehrerer verschiedener Verbindungen der Formel **(I),**
wobei
E entwederjeweils OH oder beide E zusammen ein O bedeuten,
R1, unabhängig von dem jeweils anderen Rest R1, Wasserstoff oder OR^{a} bedeutet, wobei R^{a} Wasserstoff, C1-C5-Alkyl oder C2-C5-Alkenyl ist,
R2, unabhängig von den anderen Resten R2, Wasserstoff oder OR^{b} bedeutet, wobei R^{b} Wasserstoff, C1-C5-Alkyl oder C2-C5-Alkenyl ist,
wobei gegebenenfalls zwei unmittelbar benachbarte Reste R1 und/oder R2 zusammen eine Gruppe OCH₂O repräsentieren,
und
R3 einen Rest OR^{x} bedeutet, wobei R^{x} C1-C5-Alkyl oder C2-C5-Alkenyl ist,
zur Erzeugung eines Süßeindruckes in einer oral konsumierbaren Zubereitung oder zum Beeinflussen der Stärke des Süßeindruckes süßer Stoffe oder Stoffgemische oder sowohl süß als auch unangenehm, vorzugsweise bitter, schmeckender Stoffe oder Stoffgemische.

Unter einem süßen Stoff bzw. einem süßen Stoffgemisch werden im Rahmen der vorliegenden Erfindung einen sensorischen Eindruck von Süße verursachenden Stoffe und Stoffgemische verstanden, insbesondere süß schmeckende Stoffe oder Stoffgemische.

Die vorliegende Erfindung betrifft vorzugsweise die Verwendung der erfindungsgemäß einzusetzenden Neoflavonoide der Formel **(I),** deren Gemische und/oder deren Salze (jeweils wie oben definiert) zur Verstärkung des süßen Geschmacks eines süß schmeckenden Stoffes, insbesondere des Süßeindruckes einer oral konsumierbaren Zubereitung umfassend mindestens einen weiteren süß schmeckenden Stoff.

Überraschenderweise wurde gefunden dass die erfindungsgemäß einzusetzenden Neoflavonoide der Formel **(I),** deren Gemische und/oder deren Salze (jeweils wie oben definiert) selbst einen Süßeindruck, beispielsweise in zum direkten Verzehr vorgesehene (d.h. geeignete) Zubereitungen, verursachen können bzw. den Süßeindruck anderer süß schmeckender Stoffe und von oral konsumierbaren Zubereitungen, die einen oder mehrere süß schmeckende Stoffe enthalten, wie z.B. einer wässrigen Sucroselösung, in signifikantem und ausgeprägtem Maße verstärken.

Überraschenderweise wurde zudem gefunden, dass die erfindungsgemäß einzusetzenden Neoflavonoide der Formel **(I),** deren Gemische und/oder deren Salze (jeweils wie oben definiert) bereits in sehr geringen Konzentrationen den süßen Geschmack bzw. die süße Geschmacksqualität von süß schmeckenden Stoffen in erfindungsgemäßen oral konsumierbaren Zubereitungen (vorzugsweise der Ernährung, der Mundpflege oder dem Genuss dienenden oder kosmetischen Zubereitungen zur Applikation im Bereich des Kopfes) erheblich verstärken können.

Die erfindungsgemäß einzusetzenden Neoflavonoide der Formel **(I),** deren Gemische und/oder deren Salze (jeweils wie oben definiert) können insbesondere zum Beeinflussen der Stärke von Geschmackseindrücken verwendet werden, wobei
der süße Geschmackseindruck eines süß schmeckenden Stoffes oder Stoffgemisches verstärkt wird
und/oder
der süße Geschmackseindruck eines sowohl süß als auch unangenehm, insbesondere bitter, schmeckenden Stoffes oder Stoffgemisches verstärkt und der unangenehme, insbesondere bittere, Geschmackseindruck des sowohl süß als auch unangenehm, insbesondere bitter, schmeckenden Stoffes oder Stoffgemisches vermindert oder maskiert wird.

In eigenen Untersuchungen hat sich zudem gezeigt, dass die Bedeutung des Restes R3 und dessen Position an C7 der Formel **(I)** wichtig für die gefundenen Wirkungen der erfindungsgemäß einzusetzenden Verbindungen ist, besonders für die süßverstärkende Wirkung, insbesondere für die süßverstärkende Wirkung eines süß schmeckenden Stoffes oder Stoffgemisches. Es konnte beobachtet werden, dass nicht erfindungsgemäß einzusetzende Verbindungen, die einen dem Rest R3 entsprechenden Rest nicht an Position C7, sondern bei ansonsten gleicher Struktur beispielsweise an Position C5 aufweisen, eine signifikant geringere süßverstärkende Wirkung zeigen als die erfindungsgemäß einzusetzenden Neoflavonoide der Formel **(I)** (siehe hierzu auch das nachfolgende Anwendungsbeispiel 1a-1).

Es wurde ferner in eigenen Untersuchungen gefunden, dass die erfindungsgemäß einzusetzenden Neoflavonoide der Formel **(I),** deren Gemische und/oder deren Salze (jeweils wie oben definiert) zusätzlich unangenehme Geschmacksnoten verändern und maskieren können, und insbesondere bittermaskierende Eigenschaften aufweisen (siehe hierzu auch das nachfolgende Anwendungsbeispiel 1b).

Unter Maskieren oder Maskierung wird im Rahmen des vorliegenden Textes eine Reduzierung, d.h. eine Verminderung, bzw. eine vollständige Unterdrückung verstanden.

Das Verändern oder Maskieren des unangenehmen Geschmackseindruckes bedeutet damit im Ergebnis regelmäßig eine Geschmacksverbesserung, insbesondere in Bezug auf bittere, adstringierende und/oder metallische Geschmackseindrücke.

Die Konfiguration an dem chiralen Kohlenstoffatom der Verbindungen der Formel **(I)** (d.h. an der mit einem "*" markierten Position im obigen Formelbild **(**I**)**) kann dabei (R) oder (S) sein. Dies gilt auch für die nachfolgenden Ausführungen und nachfolgend dargestellten Strukturformeln der erfindungsgemäß einzusetzenden Verbindungen.

Die Verbindungen der Formel **(I)** können dabei in bevorzugten Ausgestaltungen als reine Enantiomere oder als Mischungen von Enantiomeren in jedem beliebigen Verhältnis zueinander miteinander kombiniert werden. In einer bevorzugten Ausgestaltung werden die Verbindungen der Formel **(I)** in Form von racemischen Gemischen, d.h. als Racemate eingesetzt.

Vorzugsweise bedeuten R1 und R2, unabhängig von den jeweils anderen Rest R1 und R2, Wasserstoff, Hydroxy oder einen Rest ausgewählt aus der Gruppe bestehend aus 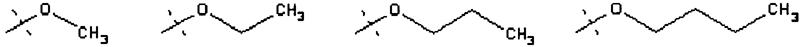 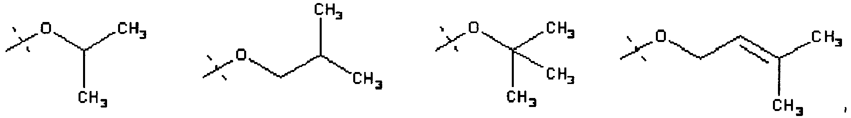 wobei die gestrichelte Linie die Bindung markiert, welche den Rest mit dem in der Formel **(I)** benachbarten C-Atom verknüpft.

Vorzugsweise bedeutet R3 einen Rest ausgewählt aus der Gruppe bestehend aus 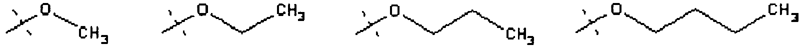 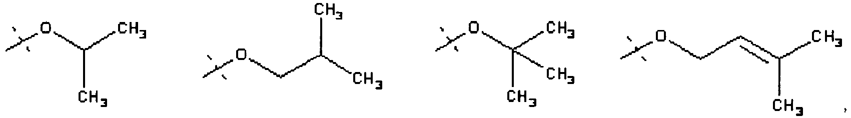 wobei die gestrichelte Linie die Bindung markiert, welche den Rest mit dem in der Formel **(I)** benachbarten C-Atom verknüpft.

Vorzugsweise gilt für die Verbindungen der Formel **(I):**
R1 bedeutet H oder OH,
   und'
R3 ist ausgewählt aus der Gruppe bestehend aus

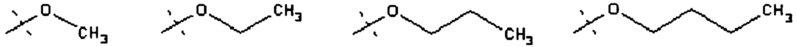

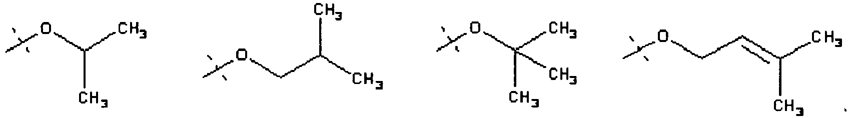

Erfindungsgemäß bevorzugt ist die oben genannte Verwendung, wobei ein, zwei, mehrere oder sämtliche der eingesetzten Verbindungen jeweils ausgewählt sind aus der Gruppe bestehend aus den Verbindungen der Formel **(I)** und deren physiologisch akzeptablen Salzen, wobei
E jeweils OH oder beide E zusammen Sauerstoff bedeuten,
R1, unabhängig von dem jeweils anderen Rest R1, Wasserstoff oder Hydroxy bedeutet,
R2, unabhängig von den anderen Resten R2, Wasserstoff, Hydroxy, Methoxy, Ethoxy, n-Propoxy, iso-Propoxy, n-Butoxy, iso-Butoxy, tert-Butoxy oder OR^{b} bedeutet, wobei R^{b} C5-Alkenyl ist, dabei wiederum bevorzugt bedeutet R^{b} Prenyl,
wobei gegebenenfalls zwei unmittelbar benachbarte Reste R2 zusammen eine Gruppe OCH₂O repräsentieren,
R3 Methoxy, Ethoxy, n-Propoxy, iso-Propoxy, n-Butoxy, iso-Butoxy, tert-Butoxy oder OR^{c} bedeutet, wobei R^{c} C5-Alkenyl ist, dabei wiederum bevorzugt bedeutet R^{c} Prenyl,
wobei vorzugsweise ein oder mehrere der Reste R1 oder R2 eine Hydroxygruppe bedeuten.

Die erfindungsgemäß einzusetzenden Verbindungen der Formel **(I)** können je nach Bedeutung von E folgenden Strukturformeln **(I-A)** oder **(I-B)** entsprechen, wobei R1, R2 und R3 jeweils die oben angegebene Bedeutung haben:

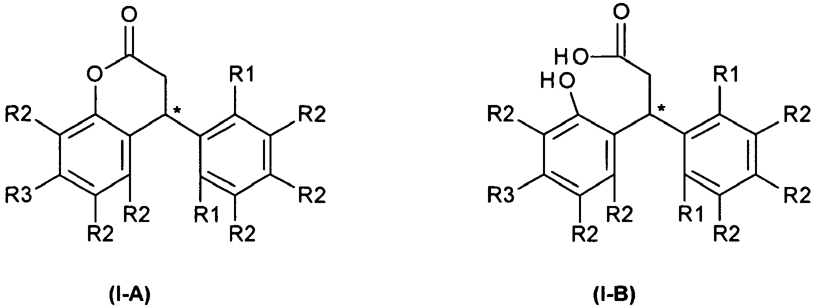

In Abhängigkeit des pH-Wertes kann der Lactonring der Verbindung der Formel **(I-A)** geöffnet werden und die Verbindung der Formel **(I-A)** im Gleichgewicht mit der entsprechenden "offenkettigen" Verbindung der Formel **(I-B)** vorliegen, wie nachfolgend schematisch gezeigt, wobei M⁺ ein (vorzugsweise physiologisch akzeptables) Gegenkation bedeutet (und wobei das Gegenkation bevorzugt die nachfolgend angegebene Bedeutung hat):

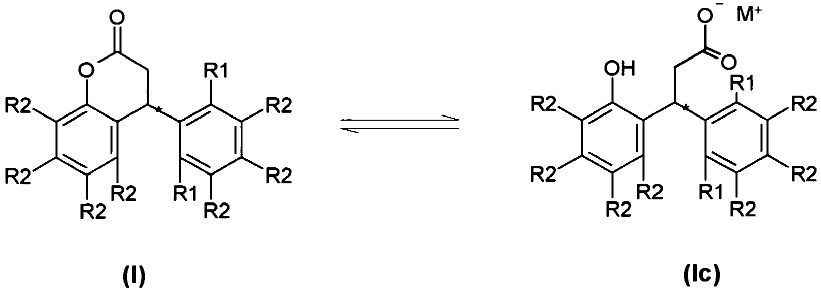

Für den Fall, dass mindestens einer der beiden Reste R1 in Formel **(I)** eine Hydroxygruppe bedeutet, kann zumeist in Abhängigkeit von der (Lebensmittel)Matrix und deren pH-Wert - insbesondere in Medien oder Matrices mit schwach saurem pH-Wert - ein Gleichgewicht zwischen den Substanzen der Formel **(I-A1)** und **(I-A2)** beobachtet werden.

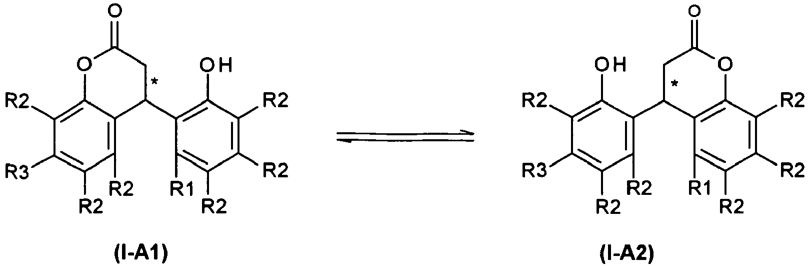

Ein weiterer Aspekt der vorliegenden Erfindung betrifft die Verwendung eines Substanzgemisches umfassend eine oder mehrere Verbindungen der Formel **(I-A1)** und eine oder mehrere Verbindungen der Formel **(I-A2),** und/oder deren physiologisch akzeptablen Salze.

In einer besonders bevorzugten Ausgestaltung gilt für die Verbindungen der Formel **(I)** und **(I-A):**
E jeweils OH oder beide E zusammen Sauerstoff bedeuten,
R1 bedeutet H,
R2 ist, unabhängig von den anderen Resten R2, ausgewählt aus der Gruppe bestehend aus Wasserstoff, Hydroxy, Methoxy, Ethoxy, n-Propoxy, iso-Propoxy,
wobei gegebenenfalls zwei unmittelbar benachbarte Reste R2 zusammen eine Gruppe OCH₂O repräsentieren,
R3 ist ausgewählt aus der Gruppe bestehend aus Methoxy, Ethoxy, n-Propoxy und iso-Propoxy,
und wobei in manchen bevorzugten Verbindungen der Formel (I) bzw. (I - A) ein oder mehrere der Reste R2 eine Hydroxygruppe bedeuten.

Erfindungsgemäß in manchen Fällen bevorzugt ist die Verwendung einer oder mehrerer Verbindungen der Formeln **(1) - (4)**, deren physiologisch akzeptablen Salze und/oder Gemische. 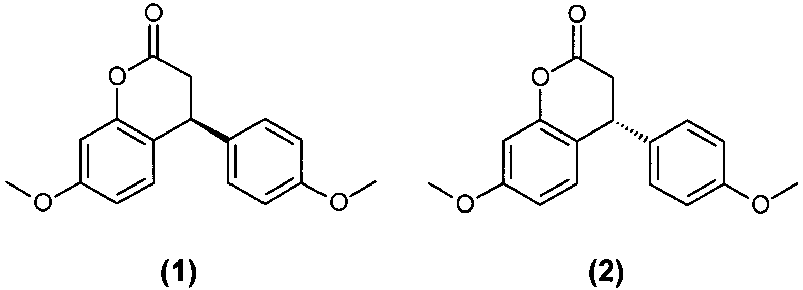 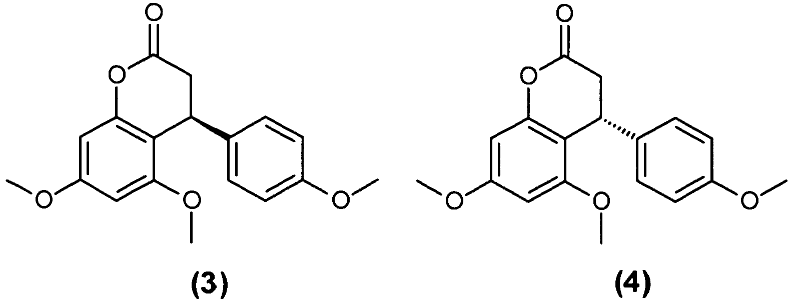
**(1)** (4*S*)-7-Methoxy-4-(4-methoxyphenyl)chroman-2-on
**(2)** (4*R*)-7-Methoxy-4-(4-methoxyphenyl)chroman-2-on
**(3)** (4*S*)-5,7-Dimethoxy-4-(4-methoxyphenyl)chroman-2-on
**(4)** (4*R*)-5,7-Dimethoxy-4-(4-methooyphenyl)chroman-2-on

Für den Fall, dass im Einzelfall ein Widerspruch zwischen dem vorstehend angegebenen chemischen Nomenklatur und der jeweils dargestellten Strukturformel der erfindungsgemäß bevorzugt einzusetzenden Verbindungen der Formeln **(1)** bis **(4)** bestehen sollte, gilt die Strukturformel.

Erfindungsgemäß bevorzugt eingesetzt werden daneben generell Verbindungen der Formel **(I),** die jeweils ausgewählt sind aus der Gruppe bestehend aus Verbindungen der Formel **(II)** 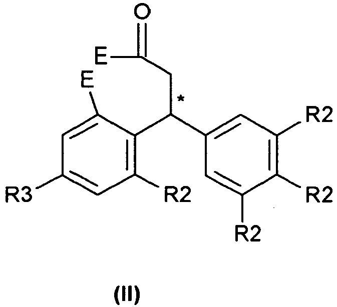 wobei
E jeweils OH oder beide E zusammen Sauerstoff bedeuten,
R2, unabhängig von den anderen Resten R2, Wasserstoff, Hydroxy, Methoxy, Ethoxy, n-Propoxy oder iso-Propoxy bedeutet, vorzugsweise H, OH, OCH₃ oder OCH₂CH₃,
wobei gegebenenfalls zwei unmittelbar benachbarte Reste R2 zusammen eine Gruppe OCH₂O repräsentieren,
R3 Methoxy, Ethoxy, n-Propoxy, iso-Propoxy, n-Butoxy, iso-Butoxy, tert-Butoxy oder OR^{c} bedeutet, wobei R^{c} C5-Alkenyl ist, dabei wiederum bevorzugt bedeutet R^{c} Prenyl,
und deren physiologisch akzeptablen Salzen,
wobei vorzugsweise ein oder mehrere der Reste R2 eine Hydroxygruppe bedeuten.

Weiter bevorzugt ist die Verwendung von Verbindungen der Formel **(II-A),** 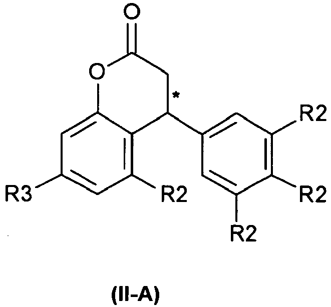 oder
- eines Salzes einer Verbindung der Formel **(II-A)**
   oder
- einer Mischung aus zwei oder mehr unterschiedlichen Verbindungen der Formel **(II-A)**, zwei oder mehr unterschiedlichen Salzen von Verbindungen der Formel (II-**A)** oder einer oder mehreren unterschiedlichen Verbindungen der Formel **(II-A)** und einem oder mehreren unterschiedlichen Salzen von einer oder mehreren unterschiedlichen Verbindungen der Formel **(II-A),**
wobei
R2, unabhängig von den anderen Resten R2, Wasserstoff, Hydroxy, Methoxy, Ethoxy, n-Propoxy oder iso-Propoxy bedeutet, vorzugsweise H, OH, OCH₃ oder OCH₂CH₃,
wobei gegebenenfalls zwei unmittelbar benachbarte Reste R2 zusammen eine Gruppe OCH₂O repräsentieren,
R3 Methoxy, Ethoxy, n-Propoxy, iso-Propoxy, n-Butoxy, iso-Butoxy, tert-Butoxy oder OR^{c} bedeutet, wobei R^{c} C5-Alkenyl ist, dabei wiederum bevorzugt bedeutet R^{c} Prenyl, besonders bevorzugt Methoxy, Ethoxy und n-Propoxy bedeutet,
wobei vorzugsweise ein oder mehrere Reste R2 eine Hydroxygruppe bedeuten.

Die Konfiguration an dem chiralen Kohlenstoffatom (d.h. an der mit einem "*" markierten Position in den obigen Formelbildern **(II)** und **(II-A)**) kann dabei jeweils (R) oder (S) sein.

Bevorzugt weist eine Verbindung der Formel **(II)** bzw. eine Verbindung der Formel **(II-A)** insgesamt ein, zwei, drei oder vier Hydroxygruppen auf, besonders bevorzugt insgesamt ein oder zwei Hydroxygruppen.

Erfindungsgemäß besonders bevorzugt ist dabei die Verwendung einer oder mehrerer Verbindungen der Formeln **(5) - (26)** und/oder deren physiologisch akzeptablen Salze 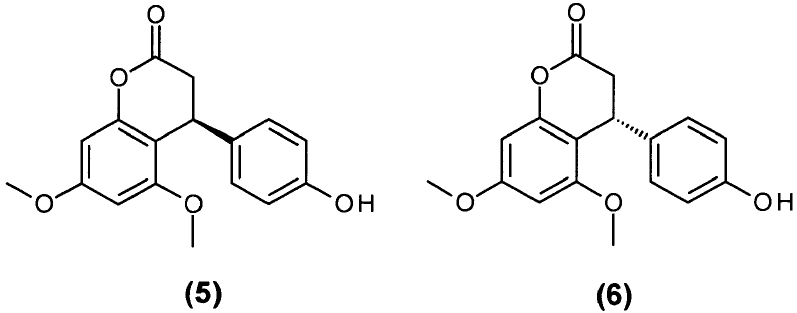 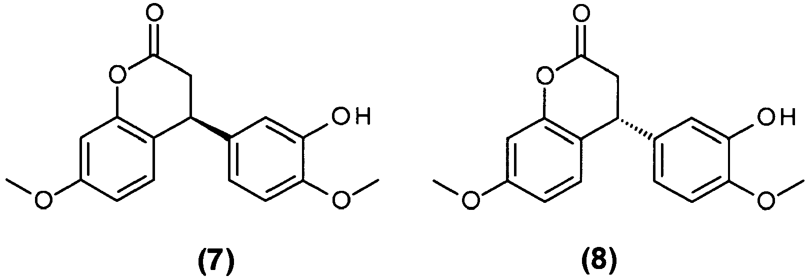 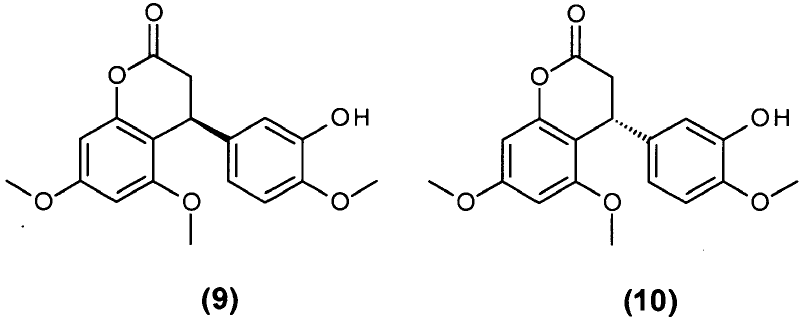 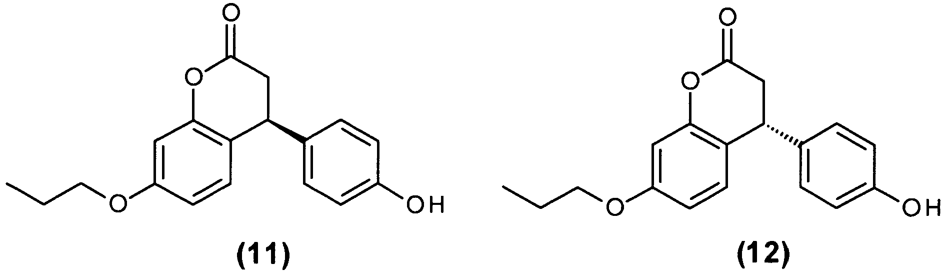 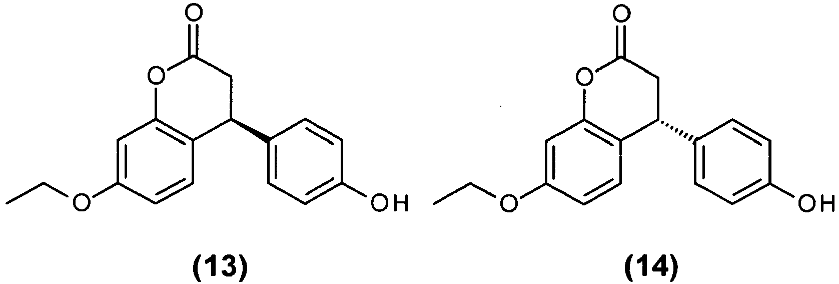 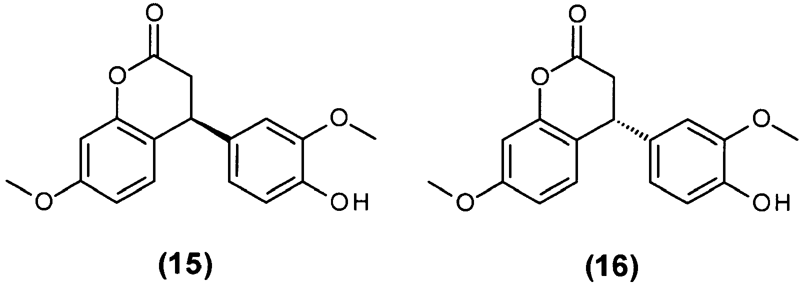 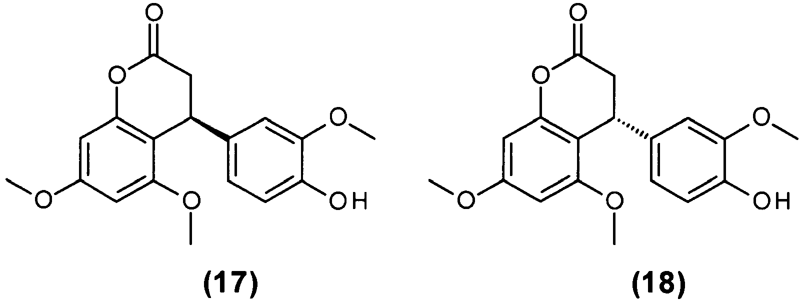 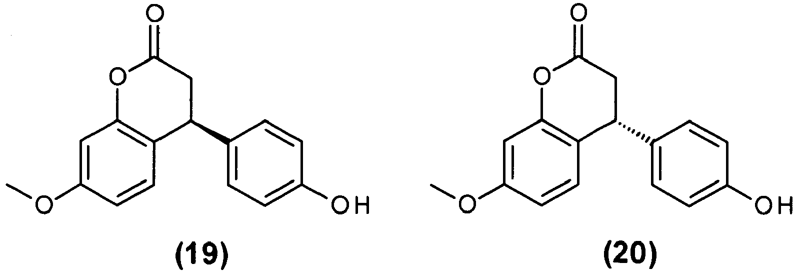 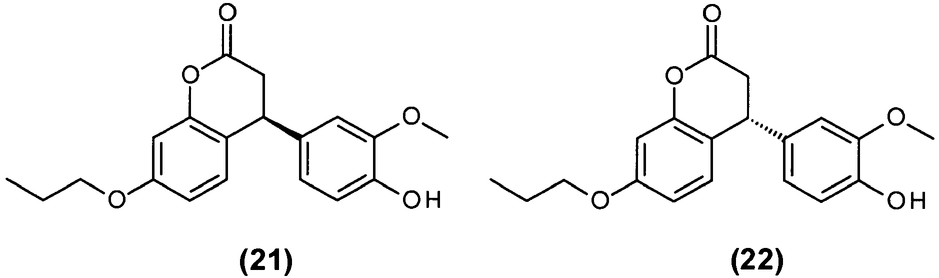 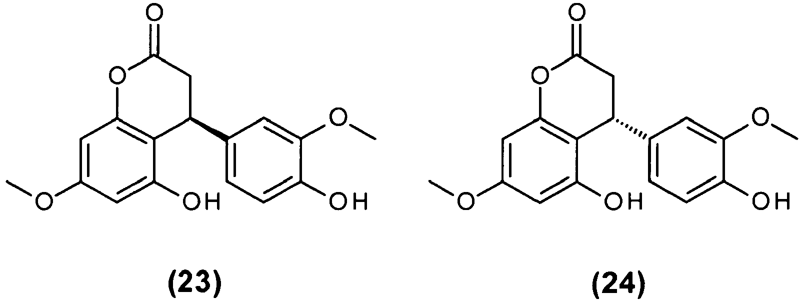 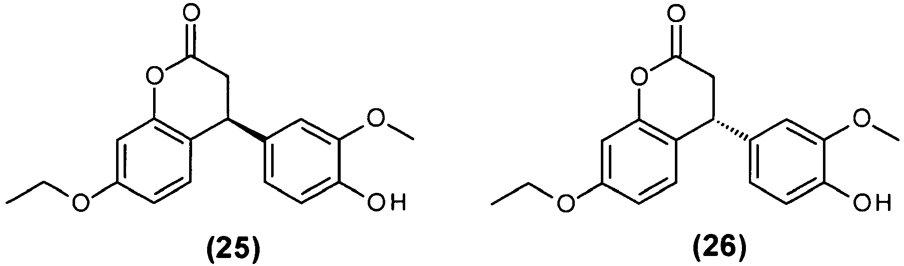
**(5)** (4*S*)-5,7-Dimethoxy-4-(4-methoxyphenyl)chroman-2-on
**(6)** (4*R*)-5,7-Dimethoxy-4-(4-methoxyphenyl)chroman-2-on
**(7)** (4*S*)-4-(3-Hydroxy-4-methoxy-phenyl)-7-methoxy-chroman-2-on
**(8)** (4*R*)-4-(3-Hydroxy-4-methoxy-phenyl)-7-methoxy-chroman-2-on
**(9)** (4*S*)-4-(3-Hydroxy-4-methoxy-phenyl)-5,7-dimethoxy-chroman-2-on
**(10)** (4*R*)-4-(3-Hydroxy-4-methoxy-phenyl)-5,7-dimethoxy-chroman-2-on
**(11)** (4*S*)-4-(4-Hydroxyphenyl)-7-propoxy-chroman-2-on
**(12)** (4*R*)-4-(4-Hydroxyphenyl)-7-propoxy-chroman-2-on
**(13)** (4*S*)-7-Ethoxy-4-(4-hydroxyphenyl)chroman-2-on
**(14)** (4*R*)-7-Ethoxy-4-(4-hydroxyphenyl)chroman-2-on
**(15)** (4*S*)-4-(4-Hydroxy-3-methoxy-phenyl)-7-methoxy-chroman-2-on
**(16)** (4*R*)-4-(4-Hydroxy-3-methoxy-phenyl)-7-methoxy-chroman-2-on
**(17)** (4*S*)-4-(4-Hydroxy-3-methoxy-phenyl)-5,7-dimethoxy-chroman-2-on
**(18)** (4*R*)-4-(4-Hydroxy-3-methoxy-phenyl)-5,7-dimethoxy-chroman-2-on
**(19)** (4*S*)-4-(4-Hydroxyphenyl)-7-methoxy-chroman-2-on
**(20)** (4*R*)-4-(4-Hydroxyphenyl)-7-methoxy-chroman-2-on
**(21)** (4*S*)-4-(4-Hydroxy-3-methoxy-phenyl)-7-propoxy-chroman-2-on
**(22)** (4*R*)-4-(4-Hydroxy-3-methoxy-phenyl)-7-propoxy-chroman-2-on
**(23)** (4*S*)-5-Hydroxy-4-(4-hydroxy-3-methoxy-phenyl)-7-methoxy-chroman-2-on
**(24)** (4*R*)-5-Hydroxy-4-(4-hydroxy-3-methoxy-phenyl)-7-methoxy-chroman-2-on
**(25)** (4*S*)-7-Ethoxy-4-(4-hydroxy-3-methoxy-phenyl)chroman-2-on
**(26)** (4*R*)-7-Ethoxy-4-(4-hydroxy-3-methoxy-phenyl)chroman-2-on

Verschiedene Neoflavonoide der Formel **(I)** können auch in bestimmten Pflanzen oder Pflanzenteilen vorkommen.

Ebenfalls sind die unter die allgemeine Formel **(I)** fallenden Neoflavonoide **(27), (28), (29)** und **(30)** welche in *Polygonum Perfoliatum* gefunden wurden (siehe Planta Medica 1999, 65, 671-673; Chin. J. Appl. Environ. Biol. 2009, 15, 615-620) sowie deren physiologisch akzeptablen Salze im Rahmen der vorliegenden Erfindung bevorzugt. 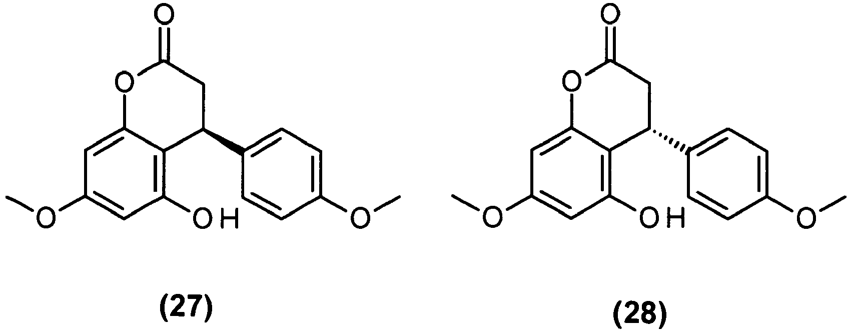 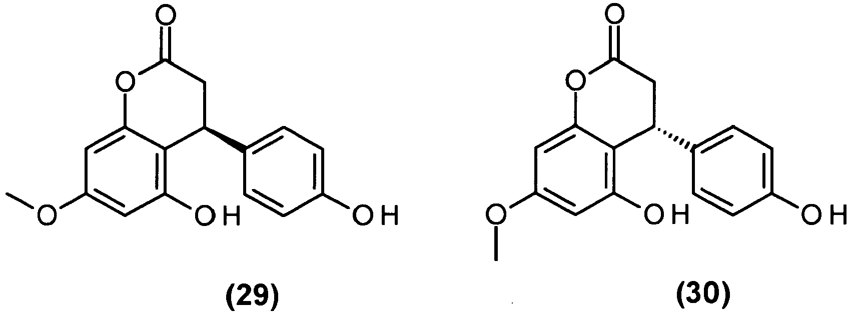
**(27)** (4*S*)-5-Hydroxy-7-methoxy-4-(4-methoxyphenyl)chroman-2-on
**(28)** (4*R*)-5-Hydroxy-7-methoxy-4-(4-methoxyphenyl)chroman-2-on
**(29)** (4*S*)-5-Hydroxy-4-(4-hydroxyphenyl)-7-methoxy-chroman-2-on
**(30)** (4*R*)-5-Hydroxy-4-(4-hydroxyphenyl)-7-methoxy-chroman-2-on

Ein oder mehrere der erfindungsgemäß zu verwendenden Neoflavonoide der Formel **(I)** können, sofern sie in der Natur vorkommen, auch in Form von pflanzlichen Extrakten, insbesondere in Form von pflanzlichen Extrakten aus *Polygonum Perfoliatum,* erfindungsgemäß verwendet werden.

Für den Fall, dass im Einzelfall ein Widerspruch zwischen der vorstehend angegebenen chemischen Nomenklatur und der jeweils dargestellten Strukturformel der erfindungsgemäß bevorzugt einzusetzenden Verbindungen der Formeln **(5)** bis **(30)** bestehen sollte, gilt die Strukturformel.

Erfindungsgemäß bevorzugt sind Verbindungen **(1)** bis **(30),** weiter bevorzugt sind Verbindungen **(5)** bis **(30),** sowie deren oben definierten Gemische und Salze, wobei diese jeweils als racemische Gemische, Einzelstoffe oder in jedem beliebigen Verhältnis zueinander kombiniert werden können.

Die Erfindung betrifft somit in einer bevorzugten Ausgestaltung die Verwendung einer Verbindung der Formeln **(1) - (30),** vorzugsweise einer Verbindung der Formeln **(5) - (30),**
oder
eines Salzes einer Verbindung der Formeln **(1) - (30),** vorzugsweise einer Verbindung der Formeln **(5) - (30),**
oder
einer Mischung aus zwei oder mehr unterschiedlichen Verbindungen der Formeln **(1) - (30),** vorzugsweise einer Verbindung der Formeln **(5) - (30),**
oder
einer Mischung aus zwei oder mehr unterschiedlichen Salzen von Verbindungen der Formeln **(1) - (30),** vorzugsweise einer Verbindung der Formeln **(5) - (30),**
oder
einer Mischung einer oder mehreren unterschiedlichen Verbindungen der Formeln **(1) - (30),** vorzugsweise einer Verbindung der Formeln **(5) - (30),** und einem oder mehreren unterschiedlichen Salzen von einer oder mehreren unterschiedlichen Verbindungen der Formeln **(1) - (30),** vorzugsweise einer Verbindung der Formeln **(5) - (30),**
zur Erzeugung eines Süßeindruckes in einer oral konsumierbaren Zubereitung oder zum Beeinflussen der Stärke des Süßeindruckes süßer Stoffe oder Stoffgemische (insbesondere süß schmeckender Stoffe oder Stoffgemische) oder sowohl süß als auch unangenehm, vorzugsweise bitter, schmeckender Stoffe oder Stoffgemische, insbesondere zur Verstärkung des Süßeindruckes einer oral konsumierbaren Zubereitung umfassend mindestens einen weiteren süß schmeckenden Stoff.

In einer bevorzugten Ausführungsform der vorliegenden Erfindung bedeutet R3 Methoxy. Dies gilt für sämtliche Aspekte der vorliegenden Erfindung, insbesondere für die erfindungsgemäß einzusetzenden Verbindungen der Formeln **(I), (I-A), (I-A1), (I-A2), (I-B), (II)** und **(II-A).** Besonders bevorzugt einzusetzende Verbindungen der Formel **(II-A)** sind daher die Verbindungen **(5), (6), (7), (8), (9), (10), (15), (16), (17), (18), (19), (20), (23), (24), (27), (28), (29)** und **(30).**

Die erfindungsgemäß einzusetzenden Verbindungen der Formeln **(I), (I-A), (I-A1), (I-A2), (I-B), (II)** und **(II-A)** können bevorzugt als ein-, oder (insbesondere im Falle der Anwesenheit mehrerer Hydroxygruppen) mehrwertige Anionen vorliegen, wobei als Gegenkation die einfach positiv geladenen Kationen der ersten Haupt- und Nebengruppe, das Ammoniumion (NH₄⁺), ein Trialkylammoniumion, die zweiwertig geladenen Kationen der zweiten Nebengruppe, sowie die dreiwertigen Kationen der 3. Haupt- und Nebengruppe dienen. Bevorzugt ist ein, mehrere oder sämtliche Gegenkationen ausgewählt aus der Gruppe bestehend aus Na⁺, K⁺, NH₄⁺, Ca²⁺, Mg²⁺, Al³⁺ und Zn²⁺.

Dem Chemiker sind mehrere Wege bekannt die Verbindungen der Formel **(I)** herzustellen. Besonders bevorzugt ist die säurekatalysierte Umsetzung (klassisch z.B. unter Verwendung von Schwefelsäure oder Salzsäure) von Phenolderivaten der nachfolgenden Formel **(A)** mit Zimtsäurederivate der nachfolgenden Formel **(B)** oder der entsprechenden Ester der nachfolgenden Formel **(C)** zu den Verbindungen der Formel **(I)** (wie oben definiert), wie in dem nachfolgenden Schema skizziert: 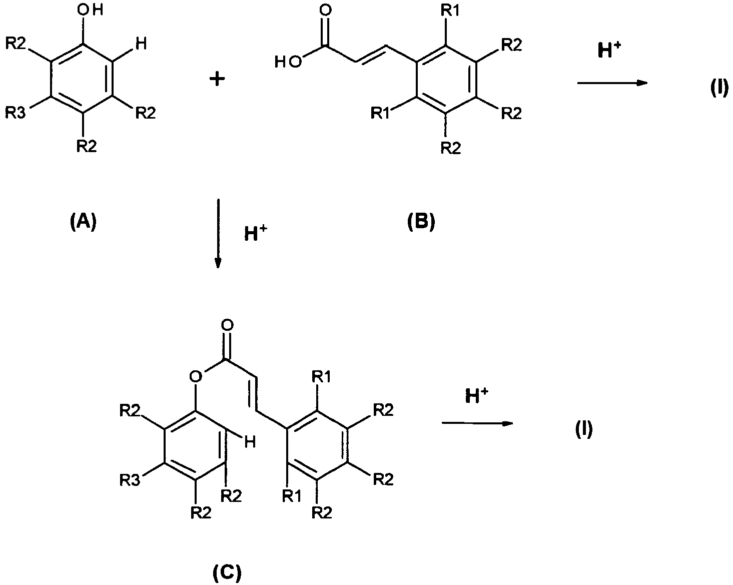 wobei jeweils die Reste R1, R2 und R3 jeweils die oben bezüglich der Formel **(I)** angegebene Bedeutung haben.

Literaturbekannt ist beispielsweise die Synthese von Verbindungen der Formel **(I)** ausgehend von 5-Methoxyresorcinol wie in Synth. Commun. 2006, 36(8) 1117-1122 beschrieben. Denkbar ist auch eine Verwendung von Montmorillonit K-10 als Katalysator analog der in Synthesis 2001, 15, 2247-2254 beschrieben Syntheseroute.

Des Weiteren können die erfindungsgemäß einzusetzenden Neoflavanoide auch in Anlehnung an die in Synth. Commun. 1987, 17(6) 723-727 beschriebene Methode hergestellt werden, wie nachfolgend anhand der Reaktion von Meldrumsäure **(E)** mit einem entsprechenden Ether des Phloroglycinols **(D)** (wobei R3 die oben angegebene Bedeutung hat) unter Zugabe eines Aldehyds (z.B. para-Hydroxybenzaldehyd **(F))** in Gegenwart von Pyridin zur Herstellung eines racemischen Gemisches erfindungsgemäß einzusetzender Verbindungen schematisch dargestellt.

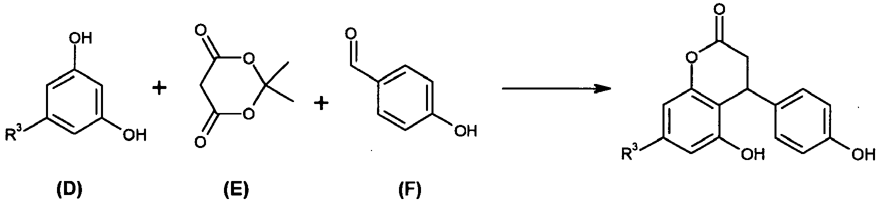

Die vorliegende Erfindung betrifft auch die Verwendung der erfindungsgemäß einzusetzenden Neoflavonoide der Formel **(I), (I-A), (I-A1), (I-A2), (I-B), (II)** und **(II-A),** deren Gemische und/oder deren Salze (jeweils wie oben definiert) in einer Zubereitung ausgewählt aus der Gruppe bestehend aus
(1) der Ernährung, der Nahrungsergänzung, der Mundpflege oder dem Genuss dienenden Zubereitungen,
(2) kosmetischen Zubereitungen, insbesondere zur Applikation im Bereich des Kopfes,
(3) zur oralen Aufnahme bestimmten pharmazeutischen Zubereitungen,
(4) Halbfertigwaren, vorzugsweise Aromakompositionen, zur Herstellung einer der in (1) bis (3) genannten Zubereitungen.

Halbfertigwaren im Rahmen der vorliegenden Erfindung liegen insbesondere Aroma- und/oder Geschmacksstoffkompositionen oder als Würzmischung vor.

Sofern die erfindungsgemäß einzusetzenden Verbindungen natürlich vorkommen, können diese auch in Form eines pflanzlichen Extraktes eingesetzt werden.

Entsprechend betrifft die vorliegende Erfindung ebenfalls die Verwendung eines aus pflanzlichem Material mittels Extraktion erhältlichen oder erhaltenen Produktes umfassend
ein, zwei oder mehrere verschiedene Verbindungen der Formel **(I)** wie oben definiert,
ein, zwei oder mehrere verschiedene physiologisch akzeptable Salze eines, zweier oder mehrerer verschiedener Verbindungen der Formel **(I)** wie oben definiert,
oder
ein Gemisch eines, zweier oder mehrerer verschiedener Verbindungen der Formel **(I)** wie oben definiert mit einem, zwei oder mehreren verschiedenen physiologisch akzeptablen Salzen eines, zweier oder mehrerer verschiedener Verbindungen der Formel **(I)** wie oben definiert,
zur Erzeugung eines Süßeindruckes in einer oral konsumierbaren Zubereitung oder zum Beeinflussen der Stärke des Süßeindruckes süßer Stoffe oder Stoffgemische (insbesondere süß schmeckender und/oder süß riechender Stoffe oder Stoffgemische) oder sowohl süß als auch unangenehm, vorzugsweise bitter, schmeckender Stoffe oder Stoffgemische, insbesondere zur Verstärkung des Süßeindruckes einer oral konsumierbaren Zubereitung umfassend mindestens einen weiteren süß schmeckenden Stoff.

Ein solcher erfindungsgemäß einzusetzender pflanzlicher Extrakt enthält vorzugsweise einen Gesamtanteil von 50 Gew.-% oder mehr, bevorzugt von 80 Gew.-% oder mehr, besonders bevorzugt von 90 Gew.-% oder mehr an Verbindungen der Formel **(I)** und deren Salzen, jeweils bezogen auf die Trockenmasse des pflanzlichen Extraktes, wobei die Verbindungen der Formel **(I)** und/oder deren Salze entweder als Einzelstoffe vorliegen oder in jedem beliebigen Verhältnis miteinander kombiniert vorliegen können.

Unter dem Begriff "Trockenmasse" im weiteren Sinne wird die wasser- und extraktionsmittelfreie Masse eines erfindungsgemäß einzusetzendenen Extraktes verstanden. Eine solche Masse kann beispielsweise erhalten werden, indem nach Beendung des oder der Extraktionsschritte das Extraktionsmittel durch destillative oder andere evaporative Verfahren vollständig entfernt wird (einschließlich des Wassers, welches gegebenenfalls aus dem pflanzlichen Material stammt).

Unter dem Begriff "Trockenmasse" im engeren Sinne wird die Gesamtmasse aller Feststoffe des Extraktes verstanden, bezogen auf 20°C bei 1013 mbar.

Im Rahmen des vorliegenden Textes bezieht sich der Zustand "fest" bzw. der Begriff "Feststoff" auf 20°C bei 1013 mbar.

Die Erfindung betrifft ferner Halbfertigwaren umfassend oder bestehend aus den folgenden Komponenten:
(a) eine oder mehrere Verbindungen der Formel **(I)** und/oder deren Salze wie oben definiert, vorzugsweise in einer der als bevorzugt bzw. besonders bevorzugt bezeichneten Ausgestaltungen,
   sowie
   einen, zwei, drei oder mehrere Stoffe ausgewählt aus der Gruppe bestehend aus den Komponenten (b) und (c)
(b) einen oder mehrere süß schmeckende Stoffe,
   und/oder
(c) einen oder mehrere sowohl süß als auch unangenehm, insbesondere bitter,
   schmeckende Stoffe,
   sowie
(d) gegebenenfalls einen oder mehrere zum Verzehr geeignete Trägerstoffe,
(e) gegebenenfalls eine oder mehrere weitere Substanzen zum Maskieren oder Vermindern eines unangenehmen, insbesondere eines bitteren Geschmackeindruckes,
   und
(f) gegebenenfalls eine oder mehrere weitere Substanzen zum Verstärken eines süßen Geschmackeindruckes,
wobei die Komponenten (b)-(f) weder eine Verbindung der Formel **(I)** noch ein Salz einer Verbindung der Formel **(I)** sind bzw. enthalten.

Erfindungsgemäße Halbfertigwaren, vorzugsweise erfindungsgemäße Aromakompositionen, und erfindungsgemäße oral konsumierbare Zubereitungen können neben den erfindungsgemäß einzusetzenden Stoffen der Formel **(I)** und/oder deren Salzen (wie oben definiert) ein oder mehrere verschiedene (natürliche oder nichtnatürliche) Aromastoffe und/oder Reaktionsaromen, Aromazubereitungen, weitere Geschmacksstoffe, weitere geschmacksmodulierende Stoffe, Prekursoren, sonstige Aromastoffe, Zusatzstoffe, Süßungs-, Färbungs- und Säuerungsmittel, Stabilisatoren oder Lösungsmittel, Hilfs- und Trägerstoffe enthalten.

Erfindungsgemäß bevorzugt ist eine Halbfertigware, die zusätzlich ein, zwei, drei, vier, fünf, sechs, sieben, acht, neun, zehn oder mehrere weitere süß schmeckende und/oder süß riechende Stoffe umfasst, wobei diese weiteren süß schmeckenden und/oder süß riechenden Stoffe vorzugsweise ausgewählt sind aus den nachfolgend definierten Gruppen (b1) bis (b5).

Eine erfindungsgemäß bevorzugte Halbfertigware umfasst eine Gesamtmenge an Verbindungen der Formel **(I)** und deren physiologisch akzeptablen Salzen wie oben definiert (Komponente (a)) im Bereich von 0,0001 bis 90 Gew.-%, bevorzugt im Bereich von 0,001 bis 50 Gew.-%, weiter bevorzugt im Bereich von 0,01 bis 40 Gew.-%, besonders bevorzugt im Bereich von 0,1 bis 20 Gew.-%, am meisten bevorzugt im Bereich von 0,2 bis 10 Gew.-%, bezogen auf das Gesamtgewicht der Halbfertigware.

Erfindungsgemäße Halbfertigwaren können zur Verstärkung des süßen sensorischen Eindruckes, insbesondere des süßen Geschmackseindruckes, von oral konsumierbaren Fertigwaren (d.h. von zum direkten Verzehr vorgesehenen Zubereitungen) dienen, die unter Verwendung der erfindungsgemäßen Halbfertigware hergestellt werden.

Eine bevorzugte erfindungsgemäße Halbfertigware enthält einen oder mehrere zum Verzehr geeignete Trägerstoffe (Komponente (d)) ausgewählt aus der Gruppe bestehend aus Ethanol, Isopropanol, Glycerin, 1,2-Propylenglycol, Diacetin, Triacetin, verzehrbaren Fetten, verzehrbaren fetten Ölen, Maltodextrin, Gummi Arabicum, Siliciumdioxid und deren Mischungen.

Eine weiter bevorzugte erfindungsgemäße Halbfertigware enthält einen oder mehrere zum Verzehr geeignete Trägerstoffe ausgewählt aus der Gruppe bestehend aus Glycerin, 1,2-Propylenglycol, Diacetin, Triacetin, verzehrbaren fetten Ölen, Maltodextrin, Gummi Arabicum, Siliciumdioxid und deren Mischungen.

In einer bevorzugten Ausführungsform ist eine erfindungsgemäße Halbfertigware dadurch gekennzeichnet, dass sie sprühgetrocknet ist.

In einer bevorzugten Ausführungsform ist eine erfindungsgemäße Halbfertigware dadurch gekennzeichnet, dass sie einen oder mehrere zum Verzehr geeignete feste Trägerstoffe umfasst, d.h. Bestandteil (d) einer erfindungsgemäßen Halbfertigware einen oder mehrere zum Verzehr geeignete feste Trägerstoffe umfasst oder daraus besteht.

Erfindungsgemäße Halbfertigware in Form von Aromakompositionen im Sinne der vorliegenden Erfindung enthalten neben einer oder mehreren Verbindungen der Formel **(I)** und/oder ein oder mehreren deren physiologisch akzeptablen Salze dieser Verbindungen zumindest (i) ein, zwei, drei, vier, fünf, sechs, sieben, acht, neun, zehn oder mehrere (weitere) Aromastoffe (vorzugsweise ausgewählt aus der nachfolgend definierten Gruppe (A)) und/oder (ii) ein, zwei, drei, vier, fünf, sechs, sieben oder mehrere (weitere) Geschmacksstoffe.

Eine besonders bevorzugt erfindungsgemäße Halbfertigware ist dadurch gekennzeichnet, dass die Halbfertigware

zwei, drei, vier, fünf, sechs, sieben, acht, neun, zehn oder mehr weitere Aromastoffe enthält mit einem Molgewicht von größer als 120 g/mol, vorzugsweise mit einem Molgewicht von größer als 125 g/mol, bevorzugt mit einem Molgewicht im Bereich von 125 g/mol bis 220 g/mol, insbesondere bevorzugt mit einem Molgewicht im Bereich von 130 g/mol bis 210 g/mol.

Eine bevorzugte erfindungsgemäße Halbfertigware enthält vorzugsweise kein Hexan und kein Methylenchlorid, und bevorzugt kein Hexan, kein Methylenchlorid, kein Aceton und kein Methanol.

Die Erfindung betrifft auch eine Halbfertigware, vorzugsweise in Form einer Aromakomposition, zur Herstellung einer erfindungsgemäßen Zubereitung (wie oben definiert), enthaltend
- ein, zwei oder mehrere verschiedene Verbindungen der Formel **(I)** wie oben definiert, oder
- ein, zwei oder mehrere verschiedene physiologisch akzeptable Salze eines, zweier oder mehrerer verschiedener Verbindungen der Formel **(I)** wie oben definiert, oder
- ein Gemisch eines, zweier oder mehrerer verschiedener Verbindungen der Formel **(I)** wie oben definiert mit einem, zwei oder mehreren verschiedenen physiologisch akzeptablen Salzen eines, zweier oder mehrerer verschiedener Verbindungen der Formel **(I)** wie oben definiert,
und
einen, zwei, drei, vier, fünf, sechs, sieben, acht, neun, zehn oder mehr weitere Aromastoffe enthält mit einem Molgewicht von größer als 90 g/mol, vorzugsweise von größer als 100 g/mol, bevorzugt mit einem Molgewicht im Bereich von 110 g/mol bis 300 g/mol, weiter bevorzugt mit einem Molgewicht im Bereich von 120 g/mol bis 250 g/mol, besonders bevorzugt mit einem Molgewicht im Bereich von 125 g/mol bis 220 g/mol, insbesondere bevorzugt mit einem Molgewicht im Bereich von 130 g/mol bis 210 g/mol,
sowie vorzugsweise einen oder mehrere zum Verzehr geeignete Trägerstoffe, vorzugsweise ausgewählt aus der Gruppe bestehend aus Ethanol, Isopropanol, Glycerin, 1,2-Propylenglycol, Diacetin, Triacetin, Maltodextrin, Gummi Arabicum, Siliciumdioxid und deren Mischungen.

Die oben beschriebenen Ausgestaltungen, insbesondere die oben als bevorzugt bzw. besonders bevorzugt gekennzeichneten Ausgestaltungen, bezüglich der erfindungsgemäßen Verwendung der Verbindungen der Formeln **(I), (I-A), (I-A1), (I-A2), (I-B), (II)** und **(II-A)** gelten entsprechend für eine erfindungsgemäße Halbfertigware, insbesondere für eine bevorzugte bzw. besonders bevorzugte erfindungsgemäße Halbfertigware.

Die vorliegende Erfindung betrifft auch eine Zubereitung, vorzugsweise oral konsumierbare Zubereitung, ausgewählt aus der Gruppe bestehend aus
(1) der Ernährung, der Nahrungsergänzung, der Mundpflege oder dem Genuss dienenden Zubereitungen,
(2) kosmetischen Zubereitungen, insbesondere zur Applikation im Bereich des Kopfes,
(3) zur oralen Aufnahme bestimmten pharmazeutischen Zubereitungen,
umfassend
(i) eine oder mehrere Verbindungen der Formel **(I)** und/oder deren Salze wie oben definiert,
   und
(ii) einen oder mehrere weitere süß schmeckende Stoffe, einen oder mehrere süß riechende Stoffe und/oder einen oder mehrere sowohl süß als auch unangenehm, insbesondere bitter, schmeckende Stoffe,
wobei die Menge an Bestandteil (i) in der Zubereitung ausreicht, den Süßeindruck des bzw. der süß schmeckenden und/oder süß riechenden Stoffe des Bestandteils (ii) sensorisch zu verstärken.

Eine bevorzugte erfindungsgemäße Zubereitung, vorzugsweise eine erfindungsgemäß oral konsumierbare Zubereitung, umfasst eine Halbfertigware wie oben definiert, vorzugsweise in einer der als bevorzugt bzw. besonders bevorzugten bezeichneten Ausgestaltungen.

Eine bevorzugte erfindungsgemäße oral konsumierbare Zubereitung (woe oben definiert) ist ausgewählt aus der Gruppe bestehend aus
(1) der Ernährung, der Nahrungsergänzung, der Mundpflege oder dem Genuss dienenden Zubereitungen,
(2) kosmetischen Zubereitungen zur Applikation im Bereich des Kopfes,
(3) zur oralen Aufnahme bestimmten pharmazeutischen Zubereitungen,
wobei die oral konsumierbare Zubereitung (1), (2) oder (3) bezogen auf ihr Gesamtgewicht 0,1 mg/kg (entsprechend 0,1 ppm) bis 1 Gew.-%, vorzugsweise im Bereich von 0,5 bis 1000 mg/kg, bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg, an Verbindungen der Formel **(I)** und/oder deren Salzen wie oben definiert umfasst.

Zur Bestimmung des Süßeindruckes einer erfindungsgemäß einzusetzenden Verbindung der Formel **(I)** im Vergleich mit einer wässrigen Sucroselösung wird eine wässrige Lösung einer Verbindung der Formel **(I)** in gleicher Konzentration hergestellt, wie sie in der zu untersuchenden oral konsumierbaren Zubereitung vorliegt. Diese wässrige Lösung wird dann von einer Gruppe von wenigstens fünf Personen geschmacklich gegen eine Vergleichsreihe verschiedener Saccharosekonzentrationen in Wasser verglichen und eingeordnet. Auf diese Art wird die Sucroseäquivalenz bestimmt (angegeben in Sucrose (= Saccharose) - Äquivalenten), also die Konzentration der Sucroselösung, deren Süßeindruck äquivalent dem Süßeindruck der Konzentration der Verbindung der Formel **(I)** ist. Entsprechend wird auch vorgegangen zur Bestimmung des Süßeindruckes eines physiologisch akzeptablen Salzes der Verbindung der Formel **(I)** oder von Gemischen (wie oben definiert).

Bevorzugte Konzentrationen der Saccharosevergleichslösung sind 0; 0,25; 0,5; 0,75; 1; 1,5; 2; 3; 4 und 5 Gew.-% Saccharose in Wasser. Gegebenenfalls können auch andere Konzentrationen, insbesondere höhere Konzentrationen für die Vergleichsreihe eingesetzt werden. Die ermittelte Sucroseäquivalenz ergibt sich aus dem Mittelwert der Einzeleinstufung der einzelnen Panelisten. Bevorzugt sind die Panelisten Personen, die im Bereich des Abschmeckens (Verkostens) über Erfahrung verfügen, verschiedenen Alters und Geschlechts sowie verschiedener Herkunft.

Eine bevorzugte erfindungsgemäße Zubereitung, vorzugsweise oral konsumierbare Zubereitung, ist dadurch gekennzeichnet, dass die Gesamtmenge an Verbindungen der Formel **(I)** und/oder deren Salzen (wie oben definiert) in der Zubereitung ausreicht, um im Vergleich mit einer Zubereitung, die bei ansonsten identischer Zusammensetzung weder eine Verbindung der Formel **(I)** noch ein Salz einer Verbindung der Formel **(I)** wie oben definiert umfasst, eine Steigerung des in Sucroseäquivalenten zu bestimmenden Süßeindruckes um 10 % oder mehr, vorzugsweise um 20 % oder mehr, bevorzugt um 30% oder mehr und besonders bevorzugt 35% oder mehr zu vermitteln.

Überraschenderweise wurde ferner gefunden, dass Verbindungen der allgemeinen Formel **(I),** deren Gemische und/oder Salze (wie oben definiert) insbesondere in Form der vorstehend bzw. nachfolgend beschriebenen erfindungsgemäßen Halbfertigwaren, den Eindruck einer Vielzahl von unangenehm, insbesondere bitter schmeckenden Stoffen und oral konsumierbaren Zubereitungen, die einen oder mehrere unangenehm, insbesondere bitter schmeckende Stoffe enthalten, verringern oder sogar vollständig unterdrücken können.

Unangenehme Geschmackseindrücke verursachende Stoffe im Rahmen dieses Textes sind:
- Stoffe, die bitter, adstringierend, pappig, kalkig, staubig, trocken, mehlig, ranzig und/oder metallisch schmecken sowie
- Stoffe, die einen bitteren, adstringierenden, pappigen, kalkigen, staubigen, trockenen, mehligen, ranzigen und/oder metallischen (gegebenenfalls stark anhaltenden) Nachgeschmack aufweisen.

Die vorgenannten unangenehm schmeckenden Stoffe können weitere, nicht unangenehme Geschmacks- und/oder Geruchsqualitäten besitzen.

Erfindungsgemäß zu maskierende Bitterstoffe sind dabei vorzugsweise Xanthine (insbesondere Coffein, Theobromin, Theophyllin), phenolische Glycoside (insbesondere Salicin, Arbutin), Flavanoidglycoside (insbesondere Neohesperedin, Eriocitrin, Neoeriocitrin, Narirutin, Hesperidin, Naringin), Chalcone oder Chalconglycoside, Dihydrochalconglycoside (insbesondere Phloridzin, Trilobatin), hydrolisierbare Tannine (insbesondere Gallus- oder Ellagsäureester von Kohlenhydraten, z.B. Pentagalloylglucose), nichthydrolisierbare Tannine (insbesondere galloylierte Catechine oder Epicatechine und deren Oligomeren, z.B. Proanthyocyanidine oder Procyanidine, Thearubigenin), Flavone und deren Glycoside (insbesondere Quercetin, Quercitrin, Rutin, Taxifolin, Myricetin, Myrictrin), Kaffeesäure oder deren Ester, terpenoide Bitterstoffe (insbesondere Limonin, Nomilin, Lupolone und Humolone), metallische Salze (Kaliumchlorid, Natriumsulfat, Magnesiumsalze, Eisensalze, Aluminiumsalze, Zinksalze), pharmazeutische Wirkstoffe (z.B. Fluorchinolon-Antibiotika, Paracetamol, Aspirin, β-Lactam-Antibiotika, Ambroxol, Propylthiouracil [PROP], Guaifenesin), Aminosäuren (z.B. Leucin, Isoleucin, Valin, Tryptophan, Prolin, Histidin, Tyrosin, Lysin oder Phenylalanin), Peptide (insbesondere Peptide mit einer Aminosäure aus der Gruppe Leucin, Isoleucin, Valin, Tryptophan, Prolin oder Phenylalanin am N- oder C-Terminus).

Bitterstoffe, die erfindungsgemäß bevorzugt maskiert werden, sind ausgewählt aus der Gruppe bestehend aus Coffein, Theobromin, Chinin, Salicin, Arbutin, Neohesperedin, Eriocitrin, Neoeriocitrin, Narirutin, Hesperidin, Naringin, Phloridzin, Catechin, Epicatechin, Epigallocatechingallat (EGCG), Gallocatechin, Gallocatechin-3-gallat, Procyanidin B2, Procyanidin B5, Procyanidin C1, Thearubigenin, Rutin, Taxifolin, Myricetin, Myrictrin, Kaffeesäure oder deren Ester, Limonin und Nomilin, Aminosäuren (z.B. Leucin, Isoleucin, Valin, Tryptophan, Prolin, Histidin, Tyrosin, Lysin oder Phenylalanin), Peptide mit einer Aminosäure aus der Gruppe Leucin, Isoleucin, Valin, Tryptophan, Prolin oder Phenylalanin am N- oder C-Terminus, Kaliumchlorid, Paracetamol, Aspirin und β-Lactam-Antibiotika.

Die erfindungsgemäß einzusetzenden Verbindungen der Formel **(I)** und die physiologisch akzeptablen Salze der Verbindungen der Formel **(I)** weisen, insbesondere in den erfindungsgemäß bevorzugt bzw. besonders bevorzugt einzusetzenden Konzentrationen, in erfindungsgemäßen (vorzugsweise oral konsumierbaren) Zubereitungen, keinen nennenswerten Eigengeschmack auf, insbesondere zeigen sie in den (bevorzugten bzw. besonders bevorzugten) Einsatzkonzentrationen keine unangenehmen oder störenden Geschmacksnoten.

In einer erfindungsgemäß bevorzugten Zubereitung liegt die Gesamtmenge an Verbindungen der Formel **(I)** wie oben definiert und an physiologisch akzeptablen Salzen der Verbindungen der Formel **(I)** wie oben definiert im Bereich von 0,1 mg/kg (entsprechend 0,1 ppm) bis 1 Gew.-%, vorzugsweise im Bereich von 0,5 bis 1000 mg/kg (entsprechend 0,5 bis 1000 ppm), bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg, jeweils bezogen auf das Gesamtgewicht der Zubereitung. Diese Mengenbereiche gelten insbesondere für zum direkten Verzehr vorgesehene erfindungsgemäße oral konsumierbare Zubereitungen.

In eigenen Untersuchungen wurde ferner gefunden, dass bei deutlich höheren Gesamteinsatzkonzentrationen an Verbindungen der Formel **(I)** wie oben definiert und an physiologisch akzeptablen Salzen der Verbindungen der Formel **(I)** wie oben definiert der süßverstärkende Effekt abnimmt bzw. in geringerem Ausmaß auftritt. Auch insoweit ist die oben genannte Gesamtmenge an Verbindungen der Formel **(I)** wie oben definiert und an physiologisch akzeptablen Salzen der Verbindungen der Formel **(I)** wie oben definiert bevorzugt, insbesondere in den bevorzugten bzw. besonders bevorzugten Gesamtmengenbereichen.

Insbesondere die süßverstärkende Wirkung der erfindungsgemäß einzusetzenden Verbindungen der Formel **(I)** wird bereits bei einer Gesamtmenge an erfindungsgemäßem Verbindungen der Formel **(I)** von kleiner 0,01 Gew.-% (entsprechend 100 ppm) (ppm = Gewichtsteile pro Million), bevorzugt von kleiner 0,0050 Gew.-% (entsprechend 50 ppm) beobachtet, jeweils bezogen auf das Gesamtgewicht einer erfindungsgemäßen oral konsumierbaren Zubereitung.

Wie oben bereits ausgeführt, sind erfindungsgemäße Halbfertigwaren und erfindungsgemäße oral konsumierbare Zubereitungen bevorzugt, die eine oder mehrere der oben genannten als bevorzugt bzw. besonders bevorzugt gekennzeichneten Verbindungen der Formeln **(I)** oder deren Salze enthalten.

Die in Planta Medica 1999, 65, 671-673 und Chin. J. Appl. Environ. Biol. 2009, 15, 615-620 beschriebenen ethanolisch-wässrigen Extrakte aus der gemahlenen ganzen Pflanze *Polygonum Perfoliatum* sind, ebenso wie die dort aus den ethanolisch-wässrigen Extrakten erhaltenen Fraktionen und Substanzgemische, nicht Gegenstand der vorliegenden Erfindung.

Eine bevorzugte erfindungsgemäße Zubereitung bzw. Halbfertigware ist dadurch gekennzeichnet, dass die Zubereitung bzw. Halbfertigware kein Extrakt ist, der mittels Extraktion der gemahlenen ganzen Pflanze *Polygonum Perfoliatum* mit einem Extraktionsmittelgemisch aus Ethanol und Wasser im Massen- oder Volumenverhältnis 90 : 10 oder 95 : 5 erhältlich ist, und vorzugsweise kein Extrakt ist, der mittels Extraktion der ganzen Pflanze *Polygonum Perfoliatum* mit einem Extraktionsmittelgemisch aus Ethanol und Wasser erhältlich ist mit einem Volumenverhältnis größer als 1 : 1 erhältlich ist, und bevorzugt kein Extrakt ist, der mittels Extraktion der ganzen Pflanze *Polygonum Perfoliatum* mit einem Extraktionsmittelgemisch aus Ethanol und Wasser erhältlich ist.

Eine bevorzugte erfindungsgemäße Zubereitung bzw. Halbfertigware enthält vorzugsweise kein Hexan und kein Methylenchlorid, und bevorzugt kein Hexan, kein Methylenchlorid, kein Aceton und kein Methanol.

Eine bevorzugte erfindungsgemäße Zubereitung bzw. Halbfertigware ist keine Zubereitung bzw. Halbfertigware, die 4-Dihydroxy-5,7-dihydroxy-4-(4-hydroxyphenyl)coumarin, α-Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Saikosaponin M, Hydropiperosid, Quercetin-3-*O*-β-*D*-glucuronid-6"-butylester und Quercetin-3-*O*-β-*D*-glucuronid-6"-methylester enthält.

Hier hinsichtlich der diesen Verbindungen entsprechenden Strukturformeln sei auf die Literaturstelle Chin. J. Appl. Environ. Biol. 2009, 15, 615-620 hingewiesen, welche hinsichtlich der darin offenbarten entsprechenden Verbindungen auf dem Wege der Verweisung Bestandteil dieser Anmeldung wird.

Eine bevorzugte erfindungsgemäße Zubereitung bzw. Halbfertigware ist keine Zubereitung bzw. Halbfertigware, die 4-Dihydroxy-5,7-dihydroxy-4-(4-hydroxyphenyl)coumarin, α-Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5 und Bonarosid im Massenverhältnis von 4 : 9 : 8 : 37 : 30 : 24 : 14 : 5 : 12 : 41 enthält.

Sofern eine erfindungsgemäße Zubereitung bzw. Halbfertigware 4-Dihydroxy-5,7-dihydroxy-4-(4-hydroxyphenyl)coumarin, enthält, gelten eine, mehrere oder sämtliche der folgenden Bedingungen:
- das Gewichtsverhältnis von 4-Dihydroxy-5,7-dihydroxy-4-(4-hydroxyphenyl)coumarin zu Quercetin ist ungleich 4 : 30,
- das Gewichtsverhältnis von 4-Dihydroxy-5,7-dihydroxy-4-(4-hydroxyphenyl)coumarin zu Cucurbitacin IIa ist ungleich 4 : 24,
- das Gewichtsverhältnis von 4-Dihydroxy-5,7-dihydroxy-4-(4-hydroxyphenyl)coumarin zu Cucurbitacin U ist ungleich 4 : 14,
- das Gewichtsverhältnis von 4-Dihydroxy-5,7-dihydroxy-4-(4-hydroxyphenyl)coumarin zu 7'-Dihydroxymatairesinol ist ungleich 4 : 8.

Sofern eine erfindungsgemäße Zubereitung bzw. Halbfertigware 4-Dihydroxy-5,7-dihydroxy-4-(4-hydroxyphenyl)coumarin, enthält, beträgt das Massenverhältnis von 4-Dihydroxy-5,7-dihydroxy-4-(4-hydroxyphenyl)coumarin zu der Gesamtmasse von α-Tocopherolchinon, 7'-Dihydroxymatairesinol, (24S)-Ethylchloesta-3β,5α,6α-triol, Quercetin, Cucurbitacin IIa, Cucurbitacin U, Iotroridosid A, Pokeweedcerebrosid 5 und Bonarosid nicht 4 : 180.

Eine bevorzugte erfindungsgemäße Zubereitung bzw. Halbfertigware ist keine Zubereitung bzw. Halbfertigware, die α-Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Saikosaponin M, Hydropiperosid, Quercetin-3-O-β-D-glucuronid-6"-butylester und Quercetin-3-*O*-β-*D*-glucuronid-6"-methylester enthält.

Eine bevorzugte erfindungsgemäße Zubereitung bzw. Halbfertigware ist keine Zubereitung bzw. Halbfertigware, die α-Tocopherolchinon, (24S)-Ethylchloesta-3β,5α,6α-triol, Cucurbitacin IIa, Cucurbitacin U, lotroridosid A, Pokeweedcerebrosid 5 und Bonarosid enthält.

Eine erfindungsgemäß bevorzugte Zubereitung bzw. Halbfertigware enthält kein Cucurbitacin IIa, vorzugsweise kein Cucurbitacin IIa und kein Cucurbitacin U, und enthält bevorzugt keine Cucurbitacine.

Die oben beschriebenen Ausgestaltungen, insbesondere die oben als bevorzugt bzw. besonders bevorzugt gekennzeichneten Ausgestaltungen, bezüglich der erfindungsgemäßen Verwendung der Verbindungen der Formeln **(I), (I-A), (I-A1), (I-A2), (I-B), (II)** und **(II-A)** gelten entsprechend für eine erfindungsgemäße Zubereitung bzw. Halbfertigware, insbesondere für eine bevorzugte bzw. besonders bevorzugte erfindungsgemäße Zubereitung bzw. Halbfertigware.

### Verfahren zur Herstellung

- einer erfindungsgemäßen Halbfertigware wie vorstehend oder nachstehend definiert
   bzw.
- einer erfindungsgemäßen Zubereitung wie vorstehend oder nachstehend definiert, umfassend die Schritte
   (1) Her- oder Bereitstellen der folgenden Komponenten:
      (a) eine oder mehrere Verbindungen der Formel **(I)** und/oder deren Salze wie oben definiert,
         sowie
         ein, zwei, drei oder mehrere Stoffe ausgewählt aus der Gruppe bestehend aus den Komponenten (b) und (c)
      (b) einen oder mehrere süß schmeckende Stoffe,
         und/oder
      (c) einen oder mehrere sowohl süß als auch unangenehm, insbesondere bitter, schmeckende Stoffe,
         sowie
      (d) gegebenenfalls einen oder mehrere zum Verzehr geeignete Trägerstoffe,
      (e) gegebenenfalls eine oder mehrere weitere Substanzen zum Maskieren oder Vermindern eines unangenehmen, insbesondere eines bitteren Geschmackeindruckes,
         und
      (f) gegebenenfalls eine oder mehrere weitere Substanzen zum Verstärken eines süßen Geschmackeindruckes,
      wobei die Komponenten (b)-(f) weder eine Verbindung der Formel **(I)** noch ein Salz einer Verbindung der Formel **(I)** sind bzw. enthalten,
      und
   (2) Mischen der Komponente (a) mit einer oder mehreren der Komponenten (b) und/oder (c) sowie gegebenenfalls (d) sowie gegebenenfalls Komponente (e) und gegebenenfalls Komponente (f).

In einem weiteren Aspekt betrifft die vorliegende Erfindung ein Verfahren zum Beeinflussen der Stärke eines sensorischen Eindruckes, insbesondere des Süßeindruckes, eines süß schmeckenden und/oder süß riechenden Stoffes oder Stoffgemisches oder eines sowohl süß als auch unangenehm, insbesondere bitter, schmeckenden Stoffes oder Stoffgemisches, mit folgendem Schritt:
Mischen von Komponente (a)
   (a) eine oder mehrere Verbindungen der Formel **(I)** und/oder deren Salze wie oben definiert
      mit Komponente (b) und/oder (c)
   (b) einen oder mehrere süß schmeckende Stoffe,
      und/oder
   (c) einen oder mehrere sowohl süß als auch unangenehm, insbesondere bitter, schmeckende Stoffe,
      sowie
   (d) gegebenenfalls einen oder mehrere zum Verzehr geeignete Trägerstoffe,
   (e) gegebenenfalls eine oder mehrere weitere Substanzen zum Maskieren oder Vermindern eines unangenehmen, insbesondere eines bitteren Geschmackeindruckes, und
   (f) gegebenenfalls eine oder mehrere weitere Substanzen zum Verstärken eines süßen Geschmackeindruckes,
      wobei die Komponenten (b)-(f) weder eine Verbindung der Formel **(I)** noch ein Salz einer Verbindung der Formel **(I)** sind bzw. enthalten, und
wobei die Gesamtmenge an Komponente (a) in der Mischung ausreicht, um die Stärke des sensorischen Eindruckes, insbesondere des Süßeindruckes, des oder der süß schmeckenden Stoffe der Komponente (b) bzw. des oder der sowohl süß als auch unangenehm, insbesondere bitter, schmeckenden Stoffe der Komponente (c) zu beeinflussen.

Die vorliegende Erfindung betrifft auch eine Verfahren zum (a) Vermitteln eines süßen Geschmackseindruckes und/oder Verstärken eines süßen Geschmackseindruckes eines, zweier oder mehrerer süß schmeckender Stoffe und/oder (b) Herstellen einer erfindungsgemäßen oral konsumierbaren Zubereitung, vorzugsweise in einer der als bevorzugt gekennzeichneten Ausgestaltungen, mit folgenden Schritten:
a) Bereitstellen einer erfindungsgemäß einzusetzenden Verbindung, vorzugsweise in einer der als bevorzugt gekennzeichneten Ausgestaltungen, bzw. eines physiologisch akzeptablen Salzes einer erfindungsgemäß einzusetzenden Verbindung, vorzugsweise in einer der als bevorzugt gekennzeichneten Ausgestaltungen, einer erfindungsgemäßen Halbfertigware, vorzugsweise in einer der als bevorzugt gekennzeichneten Ausgestaltungen, und/oder eines erfindungsgemäß einzusetzenden Extraktes, vorzugsweise in einer der als bevorzugt gekennzeichneten Ausgestaltungen,
b) Bereitstellen einer oral konsumierbaren Zubereitung, vorzugsweise umfassend einen, zwei oder mehrere weitere süß schmeckende Stoffe,
c) in Kontakt bringen oder Vermischen der in Schritt a) und b) bereitgestellten Bestandteile.

Bevorzugt sind erfindungsgemäße oral zu konsumierende Zubereitungen, umfassend eine oder mehrere Verbindungen der Formel **(I)** und/oder ein oder mehrere physiologisch akzeptable Salze einer Verbindung der Formel **(I),** in einer Gesamtmenge, die ausreicht, um in der oral zu konsumierenden Zubereitungen eine Süßwahrnehmung zu erzeugen, die mindestens derjenigen einer Vergleichszubereitung entspricht, welche aus einer 2 Gew.-%igen Lösung von Saccharose in Wasser besteht.

Bevorzugt sind erfindungsgemäße oral zu konsumierende Zubereitungen, umfassend eine oder mehrere der Verbindungen der Formel **(I)** bzw. ein oder mehrere physiologisch akzeptable Salze einer erfindungsgemäß einzusetzenden Verbindung der Formel **(I)** sowie mindestens einen weiteren süß schmeckenden Stoff, wobei dieser in einer Konzentration vorliegt, die ausreicht, um in der oral zu konsumierenden Zubereitungen eine Süßwahrnehmung zu erzeugen, die mindestens derjenigen einer Vergleichszubereitung entspricht, welche aus einer 2 Gew.-%igen Lösung von Saccharose in Wasser besteht.

Gegenstand der vorliegenden Erfindung ist auch ein Verfahren zum Verstärken des beim Verkosten (Abschmecken) entstehenden Süßeindruckes einer oral konsumierbaren Zubereitung, umfassend die Schritte:
a) Bereitstellen einer oral konsumierbaren Zubereitung, umfassend einen oder mehrere weitere süß schmeckende Stoffe die gemeinsam einen Süßeindruck vermittelt, der gleich dem oder stärker als der einer wässrigen Sucroselösung mit einer Konzentration von 2 Gew.-% Sucrose ist,
b) Bereitstellen einer oder mehrerer Verbindungen der Formel **(I),** deren Salze oder Gemische (wie oben definiert),
c) Mischen der in Schritt a) und b) bereitgestellten Bestandteile.

Die Erfindung betrifft auch ein Verfahren zum Verstärken des süßen Geschmacks eines süß schmeckenden Stoffes mit folgendem Schritt:
- Mischen eines süß schmeckenden Stoffes mit einer Menge einer oder mehrerer Verbindungen der Formel **(I),** deren Salze oder Gemische (wie oben definiert), die ausreicht, den süßen Geschmackseindruck des bzw. der süß schmeckenden Stoffe sensorisch zu verstärken,
wobei vorzugsweise die Gesamtmenge an Komponente (a) in der Mischung ausreicht,
den süßen Geschmackseindruck eines süß schmeckenden Stoffes oder Stoffgemisches zu verstärken, vorzugsweise synergistisch zu verstärken,
und/oder
den süßen Geschmackseindruck eines sowohl süß als auch unangenehm, insbesondere bitter schmeckenden Stoffes oder Stoffgemisches synergistisch zu verstärken und den unangenehmen, insbesondere bitteren, Geschmackseindruck des sowohl süß als auch unangenehm, insbesondere bitter, schmeckenden Stoffes oder Stoffgemisches zu vermindern oder zu maskieren.

Weitere süß schmeckende Stoffe im Sinne der Erfindung können natürlich vorkommende, süß schmeckende Stoffe oder Pflanzenextrakte oder aber auch synthetische süß schmeckende Stoffe sein.

Natürlich vorkommende süß schmeckende Stoffe (inklusive Pflanzenextrakte) können beispielsweise sein süß schmeckende Kohlenhydrate (z.B. Saccharose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrine), Zuckeralkohole (z.B. Erythritol, Threitol, Arabitol, Ribitol, Xylitol, Sorbitol, Mannitol, Maltitol, Isomaltit, Dulcitol, Lactitol), Proteine (z.B. Miraculin, Pentaidin, Monellin, Thaumatin, Curculin, Brazzein, Mabinlin), D-Aminosäuren (z.B. D-Phenylalanin, D-Tryptophan) oder aus natürlichen Quellen gewonnenen Extrakte oder Fraktionen, enthaltend diese Aminosäuren und/oder Proteine, Neohesperidindihydrochalkon, Naringindihydrochalkon, Steviolgylcoside, Stevioside, Steviolbiosid, Rebaudioside, Rebaudiosid A, Rebaudiosid B, Rebaudiosid C, Rebaudiosid D, Rebaudiosid E, Rebaudiosid F, Rebaudiosid G, Rebaudiosid H, Dulcoside, Rubusosid, Suavioside A, Suavioside B, Suavioside G, Suavioside H, Suavioside 1, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside 1, Oslandin, Polypodosid A, Strogin 1, Strogin, 2 Strogin 4, Selligueanin A, Dihydroquercetin-3-acetat, Perillartin, Telosmosid A₁₅, Periandrin I-V, Pterocaryoside, Cyclocaryoside, Mukurozioside, trans-Anethol, trans-Cinnamaldehyd, Bryoside, Bryonoside, Bryonodulcoside, Carnosifloside, Scandenoside, Gypenoside, Trilobatin, Phloridzin, Dihydroflavanole, Hematoxylin, Cyanin, Chlorogensäure, Albiziasaponin, Telosmoside, Gaudichaudiosid, Mogroside, Mogrosid V, Hernandulcine, Monatin, Glycyrrhetinsäure und deren Derivate insbesondere Glycyrrhizin (bevorzugt als Ammoniumsalz), und Phyllodulcin, wobei im Falle der natürlich vorkommenden Süßstoffe auch Extrakte oder angereicherte Fraktionen dieser Extrakte verwendet werden können, z.B. Thaumatococcus-Extrakte (Katemfestaude), Extrakte aus *Stevia* ssp. (insbesondere *Stevia rebaudiana*), Swingle-Extrakt (*Momordica* bzw. *Siratia grosvenorii,* Luo-Han-Guo), Extrakte aus *Glycerrhyzia* ssp. (insb. *Glycerrhyzia glabra), Rubus* ssp. (insbesondere *Rubus suavissimus*), Citrus-Extrakte, Extrakte aus *Lippia dulcis,* Extrakte aus *Mycetia balansae* (welche vorzugsweise Balansin A und/oder Balansin B enthalten), wie in der europäischen Patentanmeldung mit der Anmeldenummer 11168468.4 offenbart (Symrise), die hinsichtlich dieser Verbindung auf dem Wege der Verweisung Bestandteil dieser Anmeldung wird.

Erfindungsgemäß bevorzugt ist eine Halbfertigware bzw. eine oral konsumierbare Zubereitung, die zusätzlich ein, zwei, drei, vier, fünf, sechs, sieben, acht, neun, zehn oder mehrere weitere süße Stoffe umfasst, ausgewählt aus den folgenden Gruppen (b1) bis (b5):
(b1) süße Aromastoffe, wobei vorzugsweise ein, zwei, drei, vier, fünf oder mehrere der süßen Aromastoffe ausgewählt sind aus der Gruppe bestehend aus Vanillin, Ethylvanillin, 2-Hydrox-4-methoxybenzaldehyd, Ethylvanillinisobutyrat (= 3-Ethoxy-4-isobutyryloxybenzaldehyd), Furaneol^{®} (2,5-Dimethyl-4-hydroxy-3(2H)-furanon) und dessen Abkömmlinge (vorzugsweise Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und dessen Abkömmlinge (vorzugsweise Ethylmaltol), Cumarin, gamma-Lactone (vorzugsweise gamma-Undecalacton, gamma-Nonalacton), delta-Lactone (vorzugsweise 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Fruchtester und Fruchtlactone (vorzugsweise Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd;
(b2) Kohlenhydrate ausgewählt aus der Gruppe bestehend aus Saccharose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrine und pflanzliche Zubereitungen enthaltend einen oder mehrere der genannten Kohlenhydrate, vorzugsweise in einem Anteil von zumindest 5 Gew. %, bevorzugt zumindest 15 Gew.-%, wobei die Kohlenhydrate auch als natürlich vorkommende oder künstlich hergestellte Mischung vorliegen können, dabei insbesondere als Honig, Invertzuckersirup oder hochangereicherter Fructose-Sirup aus Maisstärke, und den physiologisch akzeptablen Salzen dieser Kohlenhydrate, insbesondere den Natrium-, Kalium-, Calcium oder Ammoniumsalzen;
(b3) Zuckeralkohole, vorzugsweise natürlich vorkommende Zuckeralkohole ausgewählt aus der Gruppe bestehend aus Glycerin, Erythritol, Threitol, Arabitol, Ribitol, Xylitol, Sorbitol, Mannitol, Maltitol, Isomaltit, Dulcitol, Lactitol, und den physiologisch akzeptablen Salzen dieser Zuckeralkohole, insbesondere den Natrium-, Kalium-, Calcium oder Ammoniumsalzen;
(b4) natürlich vorkommende Süßstoffe, vorzugsweise ausgewählt aus der Gruppe bestehend aus
(b4-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(b4-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside 1, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen;
(b4-3) Extrakte oder angereicherte Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra*), Extrakten aus *Rubus* ssp. (insbesondere *Rubus suavissimus*), Citrus-Extrakten und Extrakten aus *Lippia dulcis*; Extrakte aus *Mycetia balansae;*
(b5) synthetische süß schmeckende Stoffe, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate.

Die Aromastoffe der obigen Gruppe (b1) sind Aromastoffe, die einen süßen Geruchseindruck verursachen. Die Aromastoffe der obigen Gruppe (b1) schmecken zwar nicht süß im engeren Sinne, können aber einen süßen Geschmack im weiteren Sinne (inklusive Geruchswahrnehmung) suggerieren.

In einer bevorzugten Ausgestaltung liegt die Gesamtmenge an Verbindungen der Formel **(I)** und die Gesamtmenge an süß schmeckenden Stoffe der oben definierten Gruppen (b4) und (b5) im Bereich von 0,001 bis 1 Gew.-%, bevorzugt im Bereich von 0,001 bis 0,5 Gew.-%, weiter bevorzugt im Bereich von 0,003 bis 0,1 Gew.-%, bezogen auf die Gesamtmasse der zum direkten Verzehr vorgesehenen oral konsumierbaren Zubereitung.

Erfindungsgemäß bevorzugt ist eine Halbfertigware bzw. eine oral konsumierbare Zubereitung, wobei der oder die sowohl süß als auch unangenehm schmeckenden Stoffe der Komponente (c) ausgewählt sind aus der Gruppe bestehend aus aus Steviolglycosiden (insbesondere Steviosid und Rebaudiosid A), Rubusosid, Dulcoside, Mogroside, Phyllodulcin, Glycyrrhetinsäure, Extrakte aus Stevia ssp. (insbesondere Stevia rebaudiana), Luo Han Guo, Rubus suavissimus, Hydrangea dulcis, Glycyrrhyza glabra, Magap, Natriumcyclamat, Acesulfam K, Neohesperidindihydrochalkon, Naringindihydrochalkon, Saccharin, Saccharin-Natriumsalz, Aspartam, Superaspartam, Neotam, Alitam, Sucralose, Lugduname, Carrelame, Sucrononate und Sucrooctate.

Eine bevorzugte erfindungsgemäße oral konsumierbare Zubereitung umfasst neben einer erfindungsgemäß einzusetzenden Verbindung, neben einem physiologisch akzeptablen Salz bzw. neben einem erfindungsgemäß einzusetzenden Gemisch (wie oben definiert) zusätzlich einen oder mehrere weitere Stoffe zum Verstärken eines süßen Geschmackseindruckes.

Weitere Stoffe zum Verstärken des süßen Geschmackseindruckes - ohne die vorliegende Erfindung darauf zu beschränken - vorzugsweise ausgewählt aus der Gruppe bestehend aus Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenylalkandionen, wie zum Beispiel Isogingerdion-[2] (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); Divanilline (insbesondere solche wie beschrieben in WO 2004/078302, die bezüglich der darin offenbarten entsprechenden Verbindungen auf dem Wege der Verweisung Bestandteil dieser Anmeldung wird), Hesperetin wie in der WO 2007/014879 offenbart, die hinsichtlich dieser Verbindungen auf dem Wege der Verweisung Bestandteil dieser Anmeldung wird, 4-Hydroxydihydrochalkonen, dabei insbesondere Phloretin, wie in der EP 1 998 636 B1 offenbart, die hinsichtlich dieser Verbindungen auf dem Wege der Verweisung Bestandteil dieser Anmeldung wird, oder Propenylphenylglycosiden (Chavicolglycosiden) wie in EP 1 955 601 A1 beschrieben, die hinsichtlich dieser Verbindungen auf dem Wege der Verweisung Bestandteil dieser Anmeldung wird, Hydroxyflavane wie in EP 2 253 226 A1 offenbart, die hinsichtlich dieser Verbindungen auf dem Wege der Verweisung Bestandteil dieser Anmeldung wird, bestimmte Extrakte aus *Hydrangea macrophylla* wie in EP 2 298 084 A1 offenbart, die hinsichtlich dieser Verbindungen auf dem Wege der Verweisung Bestandteil dieser Anmeldung wird, 1-(2,4-Dihydroxyphenyl)-3-(3-hydroxy-4-methoxyphenyl)-propan-1-on wie in EP 2 353 403 A1 offenbart, die hinsichtlich dieser Verbindung auf dem Wege der Verweisung Bestandteil dieser Anmeldung wird und Triterpene und Triterpenglycoside (Balansine) sowie Extrakte aus *Mycetia balansae* (vorzugsweise enthaltend Balansin A und/oder Balansin B) wie in der europäischen Patentanmeldung mit der Anmeldenummer 11168468.4 offenbart (Symrise), die hinsichtlich dieser Verbindung auf dem Wege der Verweisung Bestandteil dieser Anmeldung wird.

Dabei sind bevorzugte den süßen Geschmack verstärkende Stoffe insbesondere Hesperetin, Phloretin, 3',7-Dihydroxy-4'-methoxyflavan und (S)-3',7-Dihydroxy-4'-methoxyflavan, bestimmte Hydrangea-Extrakte oder Phyllodulcin, sowie 1-(2,4-Dihydroxyphenyl)-3-(3-hydroxy-4-methoxyphenyl)-propan-1-on.

Mit den Stoffen der letztgenannten Gruppen lassen sich nochmals verstärkende Effekte hinsichtlich des Süßeindruckes der erfindungsgemäßen Zubereitungen erzielen.

Bevorzugt ist ferner eine erfindungsgemäße Zubereitung, umfassend einen oder mehrere weitere aromatisierende Pflanzenextrakte, Aroma-, Hilfs- oder Trägerstoffe.

Selbstverständlich umfassen bevorzugte erfindungsgemäße Zubereitungen auch weitere übliche Lebensmittelbestandteile.

In einer bevorzugten Ausgestaltung umfasst eine erfindungsgemäße oral konsumierbare Zubereitung als süß schmeckenden Stoff bzw. süß schmeckende Stoffe einen oder mehrere süß schmeckende Zucker, wobei die Menge an Neofiavonoiden der Formel **(I),** deren Salze und/oder deren Gemische (jeweils wie oben definiert) ausreicht, um im Vergleich mit einer Zubereitung, die bei ansonsten identischer Zusammensetzung weder eine Verbindung der Formel **(I)** noch ein Salz einer Verbindung der Formel **(I)** enthält, aber zumindest die 1,05-fache Menge an Zucker umfasst, den gleichen oder einen verstärkten Süßeindruck zu vermitteln.

Ganz besonders bevorzugt ist eine zum direkten Verzehr vorgesehene erfindungsgemäße oral konsumierbare Zubereitung, wobei die Gesamtmenge der Verbindungen der Formel **(I)** im Bereich von 3 ppm bis 250 ppm liegt, bevorzugt im Bereich von 5 ppm bis 175 ppm, besonders bevorzugt im Bereich von 10 bis 95 ppm, bezogen auf das Gesamtgewicht der Zubereitung.

Bevorzugt ist eine erfindungsgemäße oral konsumierbare Zubereitung, wobei diese ausgewählt ist aus der Gruppe bestehend aus pharmazeutischer Zubereitung, der Mundpflege dienende Zubereitung, der Ernährung oder dem Genuss dienende flüssige und feste Lebensmittelzubereitung, aber auch kosmetische Zubereitungen zur Applikation im Bereich des Kopfes sein, die mit der Mundhöhle in Kontakt kommen können.

Oral konsumierbare Zubereitungen können bevorzugt süß schmeckende, der Ernährung, der Nahrungsergänzung, der Mundpflege oder dem Genuss dienende Zubereitungen, kosmetische Zubereitungen, vorzugsweise zur Applikation im Bereich des Kopfes, oder orale pharmazeutische Zubereitungen (d.h. zur oralen Aufnahme bestimmte pharmazeutische Zubereitungen) sein.

Eine bevorzugt erfindungsgemäße Halbfertigware bzw. Zubereitung ist dadurch gekennzeichnet, dass die Halbfertigware bzw. Zubereitung
(i) einen, zwei, drei, vier, fünf, sechs, sieben, acht, neun, zehn oder mehr weitere Aromastoffe enthält mit einem Molgewicht von größer als 90 g/mol, vorzugsweise von größer als 100 g/mol, bevorzugt mit einem Molgewicht im Bereich von 110 g/mol bis 300 g/mol, weiter bevorzugt mit einem Molgewicht im Bereich von 120 g/mol bis 250 g/mol, besonders bevorzugt mit einem Molgewicht im Bereich von 125 g/mol bis 220 g/mol, insbesondere bevorzugt mit einem Molgewicht im Bereich von 130 g/mol bis 210 g/mol,
   und/oder
(ii) die Halbfertigware bzw. Zubereitung eine, zwei, drei, vier, fünf, sechs, sieben, acht oder sämtliche der folgenden Substanzen nicht enthält:
   Tocopherolchinon, 7'-Dihydroxymatairesinol, (24S)-Ethylchloesta-3β,5α,6α-triol, Quercetin, Cucurbitacin IIa, Cucurbitacin U, lotroridosid A, Pokeweedcerebrosid 5 und Bonarosid,
   und/oder
(iii) die Halbfertigware bzw. Zubereitung keine der folgenden Substanzen enthält:
   (24S)-Ethylchloesta-3β,5α,6α-triol, lotroridosid A, Pokeweedcerebrosid 5, Bonarosid, Heloniosid A, Heloniosid B, Lapathosid D, Vanicosid B, Vanicosid C, Vanicosid F, Asteryunnanosid, Hydropiperosid,
   und/oder
(iv) die Halbfertigware bzw. Zubereitung frei von frischen oder getrockneten Pflanzenteilen, insbesondere frei von Blättern und Blatteilen, von *Persicaria perfoliata* (= *Polygonum perfoliatum*) ist.

Eine besonders bevorzugt erfindungsgemäße Halbfertigware bzw. Zubereitung ist dadurch gekennzeichnet, dass die Halbfertigware bzw. Zubereitung
(i) zwei, drei, vier, fünf, sechs, sieben, acht, neun, zehn oder mehr weitere Aromastoffe enthält mit einem Molgewicht von größer als 120 g/mol, vorzugsweise mit einem Molgewicht von größer als 125 g/mol, bevorzugt mit einem Molgewicht im Bereich von 125 g/mol bis 220 g/mol, insbesondere bevorzugt mit einem Molgewicht im Bereich von 130 g/mol bis 210 g/mol.

Vorzugsweise gelten zumindest zwei der vorstehend definierten Bedingungen, bevorzugt Bedingung (i) und (iv) oder Bedingung (i) und (iii).

Vorzugsweise gelten zumindest drei der vorstehend definierten Bedingungen, bevorzugt Bedingung (i), (ii) und (iv) oder Bedingung (i), (ii) und (iii), oder sämtliche der Bedingungen (i) bis (iv).

Im Rahmen der vorliegenden Erfindung bevorzugt einzusetzende (weitere) Aromastoffe (als Bestandteil einer erfindungsgemäßen Halbfertigware bzw. Zubereitung) werden vorzugsweise gewählt aus der folgenden Gruppe (A) bestehend aus:
Acetophenon, Allylcapronat, alpha-lonon, beta-lonon, Anisaldehyd, Anisylacetat, Anisylformiat, Benzaldehyd, Benzothiazol, Benzylacetat, Benzylalkohol, Benzylbenzoat, beta-lonon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion®), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al, Phenylacetaldehyd, 2-Methyl-3-(methylthio)furan, 2-Methyl-3-furanthiol, Bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

In einer bevorzugten Ausgestaltung liegt die Gesamtmenge einer erfindungsgemäßen Halbfertigware, vorzugsweise einer erfindungsgemäßen Aromakomposition, vorzugsweise enthaltend ein, zwei, drei, vier, fünf oder mehrere der Aromastoffe aus der oben definierten Gruppe (b1) bzw. der Gruppe (A), im Bereich von 0,01 bis 2 Gew.-%, bevorzugt im Bereich von 0,01 bis 1 Gew.-%, weiter bevorzugt im Bereich von 0,01 bis 0,5 Gew.-%, besonders bevorzugt im Bereich von 0,01 bis 0,2 Gew.-%, jeweils bezogen auf die Gesamtmasse der zum direkten Verzehr vorgesehenen oral konsumierbaren Zubereitung.

Die der Ernährung oder dem Genuss dienenden flüssige und feste Lebensmittelzubereitung im Sinne der Erfindung sind z.B. Backwaren (z.B. Brot, Trockenkekse, Kuchen, sonstiges Gebäck), Süßwaren im engeren Sinne (z.B. Schokoladen, Schokoladenriegelprodukte, sonstige Riegelprodukte, Fruchtgummi, Hart- und Weichkaramellen, Kaugummi), alkoholische oder nicht-alkoholische Getränke (z.B. Kaffee, Tee, Wein, weinhaltige Getränke, Bier, bierhaltige Getränke, Liköre, Schnäpse, Weinbrände, fruchthaltige Limonaden, isotonische Getränke, Erfrischungsgetränke, Nektare, Obst- und Gemüsesäfte, Frucht- oder Gemüsesaftzubereitungen), Instantgetränke (z.B. Instant-Kakao-Getränke, Instant-Tee-Getränke, Instant-Kaffeegetränke), Fleischprodukte (z.B. Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (z.B. Frühstückscerealien, Müsliriegel, vorgegarte Fertigreis-Produkte), Milchprodukte (z.B. Milchgetränke, Milcheis, Joghurt, Kefir, Frischkäse, Weichkäse, Hartkäse, Trockenmilchpulver, Molke, Butter, Buttermilch, teilweise oder ganz hydrolisierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fruchtzubereitungen (z.B. Konfitüren, Fruchteis, Fruchtsoßen, Fruchtfüllungen), Gemüsezubereitungen (z.B. Ketchup, Soßen, Trockengemüse, Tiefkühlgemüse, vorgegarte Gemüse, in Essig eingelegte Gemüse, eingekochte Gemüse), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Remoulade, Dressings, Würzzubereitungen), sonstige Fertiggerichte und Suppen (z.B. Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden.

Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen als Nahrungsergänzungsmittel vorliegen.

Der Mundpflege dienende Zubereitungen (Mundhygieneprodukte) im Sinne der vorliegenden Erfindung sind insbesondere Mund- und/oder Zahnpflegemittel wie Zahnpasten, Zahngele, Zahnpulver, Mundwässer, Kaugummis und andere Mundpflegemittel.

Orale pharmazeutische Zubereitungen im Sinne der Erfindung sind Zubereitungen, die z.B. in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen und als verschreibungspflichtige, apothekenpflichtige oder sonstige Arzneimittel oder als Nahrungsergänzungsmittel verwendet werden.

Kosmetische Zubereitungen zur Applikation im Bereich des Kopfes sind insbesondere solche, die selbst bei sachgemäßer Auftragung auf die Haut mit der Mundhöhle in Kontakt treten können, also beispielsweise - wie bereits erwähnt - kosmetische Zubereitungen zur Applikation im Bereich des Kopfes wie Seifen, andere Reinigungs- oder Pflegemittel für den Gesichtsbereich, Gesichtscremes, -lotionen oder -salben, Sonnenschutzmittel, Bartreinigungs- oder -pflegemittel, Rasierschäume, -seifen oder -gele, Lippenstifte oder andere Lippenkosmetika oder Lippenpflegemittel.

Weitere übliche Wirk-, Grund-, Hilfs- und Zusatzstoffe für der Ernährung, der Mundpflege oder dem Genuss dienende, kosmetische oder orale pharmazeutische Zubereitungen können in Mengen von bis zu 99,9999999 Gew.-% enthalten sein, bezogen auf das Gesamtgewicht der Zubereitung. Ferner können die Zubereitungen Wasser in einer Menge bis zu 99,99 Gew.-% enthalten, vorzugsweise 5 bis 90 Gew.-%, bezogen auf das Gesamtgewicht der Zubereitung.

Die erfindungsgemäßen Zubereitungen enthaltend Verbindungen der Formel **(I)** werden gemäß einer bevorzugten Ausgestaltung hergestellt, indem die Verbindungen der Formel **(I)** bevorzugt als Aromakomposition in Form eines Gemisches mit einem festen oder flüssigen Trägerstoff in eine oral konsumierbare Basis-Zubereitung eingearbeitet werden. Vorteilhafterweise können als Lösung vorliegende erfindungsgemäße Zubereitungen auch durch Sprühtrocknung in eine feste Zubereitung überführt werden.

Gemäß einer weiteren bevorzugten Ausführungsform werden zur Herstellung erfindungsgemäßer oral konsumierbarer Zubereitungen die Verbindungen der Formel **(I)** oder diese enthaltende Aromakompositionen auch vorher in Emulsionen, in Liposomen, z.B. ausgehend von Phosphatidylcholin, in Microsphären, in Nanosphären oder auch in Kapseln, Granulaten oder Extrudaten aus einer für Lebens- und Genussmittel geeigneten Matrix, z.B. aus Stärke, Stärkederivaten, Cellulose oder Cellulosederivaten (z.B. Hydroxypropylcellulose), anderen Polysacchariden (z.B. Alginat), natürlichen Fetten, natürlichen Wachsen (z.B. Bienenwachs, Carnaubawachs) oder aus Proteinen, z.B. Gelatine, eingearbeitet.

In einem weiteren bevorzugten Herstellungsverfahren werden die Verbindungen der Formel **(I)** oder diese enthaltende Aromakompositionen vorher mit einem oder mehreren geeigneten Komplexbildnern, beispielsweise mit Cyclodextrinen oder Cyclodextrinderivaten, bevorzugt α- oder β-Cyclodextrin, komplexiert und in dieser komplexierten Form eingesetzt.

Besonders bevorzugt ist eine erfindungsgemäße oral konsumierbare Zubereitung, bei der die Matrix so gewählt wird, dass die Verbindungen der Formel **(I)** verzögert von der Matrix freigegeben werden, so dass man eine langanhaltende Wirkung erhält.

Als weitere Bestandteile für erfindungsgemäße oral konsumierbare Zubereitungen können übliche Grund-, Hilfs- und Zusatzstoffe für Nahrungs- oder Genussmittel verwendet werden, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Früchte, Kräuter, Nüsse, Gemüse- oder Fruchtsäfte oder -pasten oder deren Gemische), verdauliche oder nicht verdauliche, nicht süße Kohlenhydrate (z.B. Dextrine, Amylose, Amylopektin, Inulin, Xylane, Cellulose), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutahthion), native oder prozessierte Proteine (z.B. Gelatine), Enzyme (z.B. Peptidasen), Nukleinsäuren, Nucleotide, andere Geschmackskorrigentien für unangenehme Geschmackseindrücke, Geschmacksmodulatoren für weitere, in der Regel nicht unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carageenan, Alginat), Konservierungsstoffe (z.B. Benzoesäure, Sorbinsäure), Antioxidantien (z.B. Tocopherol, Ascorbinsäure), Chelatoren (z.B. Citronensäure), organische oder anorganische Säuerungsmittel (z.B. Äpfelsäure, Essigsäure, Citronensäure, Weinsäure, Phosphorsäure), zusätzliche Bitterstoffe (z.B. Chinin, Coffein, Limonin, Amarogentin, Humolone, Lupolone, Catechine, Tannine), mineralische Salze (z.B. Natriumchlorid, Kaliumchlorid, Magnesiumchlorid, Natriumphosphate), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigentien.

Zahnpflegemittel (als Beispiel für der Mundpflege dienende Zubereitungen) umfassen im allgemeinen ein abrasives System (Schleif- oder Poliermittel), wie z.B. Kieselsäuren, Calciumcarbonate, Calciumphosphate, Alumiuniumoxide und/oder Hydroxylapatite, oberflächenaktive Substanzen wie z.B. Natriumlaurylsulfat, Natriumlaurylsarcosinat und/oder Cocamidopropylbetain, Feuchthaltemitteln wie z.B. Glycerin und/oder Sorbit, Verdickungsmittel, wie z.B. Carboxymethylcellulose, Polyethylenglycole, Carrageenan und/oder Laponite^{®}, Geschmackskorrigentien für unangenehme Geschmackseindrücke, Geschmackskorrigentien für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Kühlwirkstoffen wie z.B. Menthol, Mentholderivate (z.B. L-Menthol, L-Menthyllactat, L-Menthylalkylcarbonate, Menthonketale, Menthancarbonsäureamide), 2,2,2-Trialkylessigsäureamiden (z.B. 2,2-Diisopropylpropionsäuremethylamid), Icilin-Derivate, Stabilisatoren und aktive Wirkstoffe, wie z.B. Natriumfluorid, Natriummonofluorphosphat, Zinndifluorid, quartären Ammoniumfluoriden, Zinkcitrat, Zinksulfat, Zinnpyrophosphat, Zinndichlorid, Mischungen verschiedener Pyrophosphate, Triclosan, Cetylpyridiniumchlorid, Aluminiumlactat, Kaliumcitrat, Kaliumnitrat, Kaliumchlorid, Strontiumchlorid, Wasserstoffperoxid, Aromen und/oder Natriumbicarbonat oder Geruchskorrigentien.

Kaugummis (als weiteres Beispiel für der Mundpflege oder dem Genuss dienende Zubereitungen), umfassen im allgemeinen eine Kaugummibase, d.h. eine beim Kauen plastisch werdende Kaumasse, andere Geschmackskorrigentien für unangenehme Geschmackseindrücke, Geschmacksmodulatoren für weitere, in der Regel nicht unangenehme Geschmackseindrücke, geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, weitere Aromen und Stabilisatoren oder Geruchskorrigentien.

Als Bestandteile für erfindungsgemäße orale pharmazeutische Zubereitungen können alle üblicherweise weiteren Wirk-, Grund-, Hilfs- und Zusatzstoffe für orale pharmazeutische Zubereitungen verwendet werden. Als Wirkstoffe können insbesondere auch unangenehm schmeckende oral formulierbare pharmazeutische Wirkstoffe verwendet werden. Die Wirk-, Grund-, Hilfs- und Zusatzstoffe können in an sich bekannter Weise in die oralen Applikationsformen überführt werden. Dies geschieht regelmäßig unter Verwendung inerter, nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) und Geruchskorrigentien sowie nicht den bitteren Geschmack betreffende Geschmackskorrigentien.

Bevorzugt können die erfindungsgemäßen oral konsumierbare Zubereitungen auch weitere Aromastoffe enthalten, um den Geschmack und/oder Geruch der Zubereitung abzurunden und zu verfeinern. Geeignete Aromakompositionen enthalten z.B. synthetische, natürliche oder naturidentische Aroma-, Riech- und/oder Geschmacksstoffe sowie geeignete Hilfs- und Trägerstoffe. Als besonders vorteilhaft wird dabei angesehen, dass ein eventuell vorhandener bitterer oder metallischer Geschmackseindruck, der von in den erfindungsgemäßen Zubereitungen enthaltenen Aroma-, Riech- und/oder Geschmacksstoffen ausgeht, vermindert oder unterdrückt werden kann und damit das gesamte Aroma- oder Geschmacksprofil verbessert wird.

Wie bereits ausgeführt, betrifft ein Aspekt der vorliegenden Erfindung die Verwendung einer erfindungsgemäß einzusetzenden Verbindung der Formel **(I),** eines erfindungsgemäß einzusetzenden Salzes oder eines erfindungsgemäß einzusetzenden Gemisches (jeweils wie oben definiert), oder einer erfindungsgemäßen Halbfertigware (wie oben definiert)
- zur Erzielung eines süßen Geschmackseindruckes,
- zur Verstärkung eines süßen Geschmackseindruckes,
   und/oder
- als Geschmackskorrigenz.

In einer besonders bevorzugten Ausführung der Erfindung umfasst eine erfindungsgemäße Zubereitung neben einer Verbindung der Formel **(I),** einem physiologisch akzeptablen Salz einer Verbindung der Formel **(I),** einem erfindungsgemäß einzusetzenden Gemisch (jeweils wie oben definiert) zusätzlich ein oder mehrere weitere Geschmackskorrigentien, d.h. eine oder mehrere weitere Substanzen, die nicht der Formel **(I)** oder deren Salz entsprechen, wobei das Geschmackskorrigenz geeignet ist bzw. die Geschmackskorrigentien geeignet sind zum
- Verändern oder Maskieren (Maskieren bedeutet dabei Vermindern oder vollständig Unterdrücken) des unangenehmen Geschmackseindruckes eines oder mehrerer unangenehm schmeckender Stoffe, oder
- Verstärken eines angenehmen Geschmackseindruckes, vorzugsweise eines weiteren Geschmackseindruckes zusätzlich zu einem süßen Geschmackseindruck, oder
- Verstärken eines angenehm schmeckenden Stoffes, vorzugsweise eines angenehm schmeckenden Stoffes,der zusätzlich zu einem süßen Geschmackseindruck einen weiteren angenehmen Geschmackseindruck vermittelt.

Die weiteren Geschmackskorrigentien können bevorzugt aus der folgenden Liste ausgewählt werden: Nucleotide (z.B. Adenosin-5'-monophosphat, Cytidin-5'-monophosphat) oder deren pharmazeutisch akzeptablen Salze, Lactisole, Natriumsalze (z.B. Natriumchlorid, Natriumlactat, Natriumcitrat, Natriumacetat, Natriumgluconoat), Hydroxyflavanonen, wie zum Beispiel Eriodictyol, Sterubin (Eriodictyol-7-methylether), Homoeriodictyol, und deren Natrium-, Kalium-, Calcium-, Magnesium- oder Zinksalzen (insbesondere solche wie beschrieben in EP 1 258 200 A2, die bezüglich der darin offenbarten entsprechenden Verbindungen auf dem Wege der Verweisung Bestandteil dieser Anmeldung wird), Hydroxybenzoesäureamiden, wie zum Beispiel 2,4-Dihydroxybenzoesäurevanillylamid, 2,4-Dihydroxybenzoesäure-N-(4-hydroxy-3-methoxybenzyl)amid, 2,4,6-Trihydroxybenzoesäure-N-(4-hydroxy-3-methoxybenzyl)amid, 2-Hydroxy-benzoesäure-N-4-(hydroxy-3-methoxybenzyl)amid, 4-Hydroxybenzoesäure-N-(4-hydroxy-3-methoxybenzyl)amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)ethylamid, 2,4-Dihydroxybenzoesäure-N-(4-hydroxy-3-ethoxybenzyl)amid, 2,4-Dihydroxybenzoesäure-N-(3,4-dihydroxybenzyl)amid und 2-Hydroxy-5-methoxy-N-[2-(4-hydroxy-3-methoxyphenyl)ethyl]amid; 4-Hydroxybenzoesäurevanillylamid (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl)ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenylalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird); Divanillinen (insbesondere solche wie beschrieben in WO 2004/078302, die bezüglich der darin offenbarten entsprechenden Verbindungen auf dem Wege der Verweisung Bestandteil dieser Anmeldung wird) und 4-Hydroxydihydrochalconen, vorzugsweise wie beschrieben in US 2008/227867 A1, die bezüglich der darin offenbarten entsprechenden Verbindungen auf dem Wege der Verweisung Bestandteil dieser Anmeldung wird, dabei insbesondere Phloretin und Davidigenin, Aminosäuren oder Gemische von Molkeproteinen mit Lecithinen, Hesperetin wie in der WO 2007/014879 offenbart, die hinsichtlich dieser Verbindungen auf dem Wege der Verweisung Bestandteil dieser Anmeldung wird, 4-Hydroxydihydrochalkone wie in der WO 2007/107596 offenbart, die hinsichtlich dieser Verbindungen auf dem Wege der Verweisung Bestandteil dieser Anmeldung wird, oder Propenylphenylglycoside (Chavicolglycoside) wie in EP 1 955 601 A1 beschrieben, die hinsichtlich dieser Verbindungen auf dem Wege der Verweisung Bestandteil dieser Anmeldung wird, Pellitorin, insbesondere trans-Pellitorin, und davon abgeleitete Aromakompositionen wie in EP 2 008 530 A1 beschrieben, die hinsichtlich dieser Verbindungen und Aromakompositionen auf dem Wege der Verweisung Bestandteil dieser Anmeldung werden, bestimmte Extrakte aus *Rubus suavissimus* wie in US Provisional Application 61/333,435 (Symrise) und den darauf basierenden Patentanmeldungen (z.B. die europäische Patentanmeldung mit der Anmeldenummer 11165566.8 (Symrise)) beschrieben, die hinsichtlich dieser Extrakte auf dem Wege der Verweisung Bestandteil dieser Anmeldung werden, Umami - Verbindungen wie die in WO 2008/046895 A1 und EP 1 989 944 A1 beschrieben, die jeweils hinsichtlich dieser Verbindungen auf dem Wege der Verweisung Bestandteil dieser Anmeldung werden sowie Umami-Verbindungen wie beschrieben in EP 2 064 959 A1 bzw. EP 2 135 516 A1, die bezüglich der darin offenbarten entsprechenden Verbindungen auf dem Wege der Verweisung Bestandteil dieser Anmeldung werden, Phyllodulcin oder Extrakte aus *Hydrangea macrophylla* var. *thunbergii* makino und davon abgeleitete Aromakompositionen, wie beschrieben in EP 2 298 084 A1 bzw. US 2011/0076239 A1, die hinsichtlich dieser Extrakte bzw. Phyllodulcin auf dem Wege der Verweisung Bestandteil dieser Anmeldung werden, Hydroxyflavan-Derivate und davon abgeleitete Aromakompositionen, wie beschrieben in US 2010/0292175 A1, die hinsichtlich dieser Verbindungen und der davon abgeleiteten Aromakompositionen auf dem Wege der Verweisung Bestandteil dieser Anmeldung werden und bestimmte Neoflavonoide wie in der europäischen Patentanmeldung mit der Anmeldenummer EP 11 181 504.9 offenbart (Symrise).

Sofern eine erfindungsgemäße Zubereitung beispielsweise eine vergleichsweise hohe Konzentration einer erfindungsgemäß einzusetzenden Verbindung der Formel **(I),** eines physiologisch akzeptablen Salzes einer Verbindung der Formel **(I),** oder eines erfindungsgemäß einzusetzenden Gemisches enthält, kann es vorkommen, dass neben den beschriebenen gewünschten sensorischen Effekten zusätzlich unerwünschte Geschmackseindrücke auftreten. Diese unerwünschten Geschmackseindrücke können durch Geschmackskorrigenzien zumindest teilweise reduziert oder sogar vollständig unterdrückt werden. In einem solchen Fall werden die Geschmackskorrigenzien vorzugsweise ausgewählt aus der Gruppe bestehend aus Natriumsalzen (dabei vorzugsweise Natriumchlorid, Natriumlactat, Natriumcitrat, Natriumacetat, Natriumgluconoat), Homoeriodictyol oder dessen Natriumsalzen, Eriodictyol, trans-Pellitorin und Rubus-Extrakten, vorzugsweise Rubus-Extrakten wie in der europäischen Patentanmeldung mit der Anmeldenummer 11165566.8 (Symrise) beschrieben.

### Beispiele

Die nachfolgenden Beispiele dienen zur Verdeutlichung der Erfindung, ohne diese damit einzuschränken. Sofern nicht anders angegeben, beziehen sich alle Angaben auf das Gewicht.

Der im Folgenden eingesetzte Extrakt aus *Mycetia balansae* sowie die Verbindungen Balansin A und Balansin B wurden erhalten wie in der europäischen Patentanmeldung mit der Anmeldenummer 11168468.4 beschrieben (Symrise).

### Beispiele

Die Beispiele dienen zur Verdeutlichung der Erfindung, ohne diese einzuschränken. Sofern nicht anders angegeben, beziehen sich alle Angaben auf das Gewicht.

### Beispiel 1: (4S)-7-Methoxy-4-(4-methoxyphenyl)chroman-2-on (1) und (4R)-7-Methoxy-4-(4-methoxyphenyl)chroman-2-on (2)

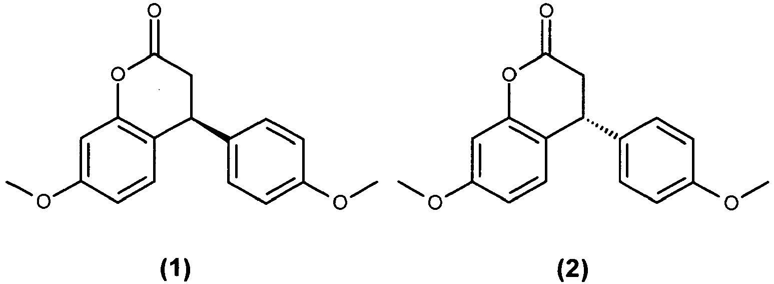

30 mmol 3-Methoxyphenol und 30 mmol 4-Methoxyzimtsäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml 5%-iger Natriumbicarbonatlösung gewaschen, über Natriumsulfat getrocknet und eingeengt. Der erhaltene Feststoff wurde aus Hexan/Aceton (80:45) umkristallisiert.

### Ausbeute: 4,2 mmol (14% der Theorie)

### Analysendaten:

**¹H-NMR (400 MHz,** CDCl₃): 2.95 (dd, J = 8.0/15.8 Hz, 1 H); 3.03 (dd, J = 5.9/15.8 Hz, 1 H); 3.79 (s, 3H); 3.80 (s, 3H); 4.24 (dd, J = 5.9/8.0 Hz, 1 H); 6,63 (dd, J = 2.6/8.5 Hz 1 H); 6.68 (d, J = 2.6 Hz, 1 H); 6.84 - 6.90 (kB, 3H); 7.04 - 7.09 (m, 2H) ppm.

**¹³C-NMR (100 MHz, CDCl₃):** 37.5 (CH₂); 39.3 (CH); 55.3 (CH₃); 55.6 (CH₃); 102.5 (CH); 110.7 (CH); 114.5 (CH); 118.1 (C); 128.6 (CH); 128.9 (CH); 132.7 (C); 152.4 (C); 158.9 (C); 160.0 (C); 167.8 (C=O) ppm.

**Massenspektrum (EI):** m/z (%) = 285 (19); 284 (M⁺, 100); 256 (29); 253 (9); 242 (17); 241 (84); 227 (16); 212 (11); 211 (56); 128 (12).

### Beispiel 2: (4S)-5,7-Dimethoxy-4-(4-methoxyphenyl)chroman-2-on (3) und (4R)-5,7-Dimethoxy-4-(4-methoxyphenyl)chroman-2-on (4)

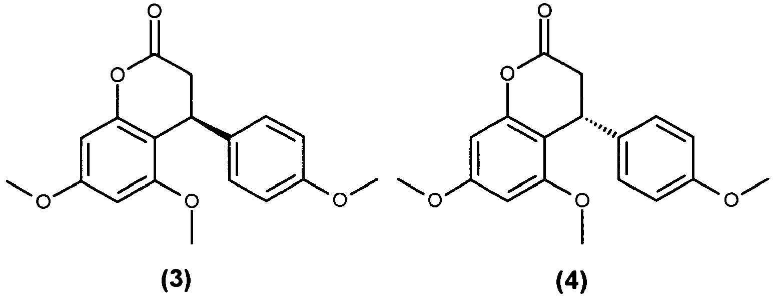

30 mmol 3,5-Dimethoxyphenol und 30 mmol 4-Methoxyzimtsäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml 5%-iger Natriumbicarbonatlösung gewaschen, über Natriumsulfat getrocknet und eingeengt. Der erhaltene Feststoff wurde aus Hexan/Aceton (5:2) umkristallisiert.

### Ausbeute: 12,0 mmol (40% der Theorie)

### Analysendaten:

**¹H-NMR (400 MHz, CDCl₃):** 2.98 (m, 2H); 3.75 (s, 6H); 3.81 (s, 3H); 4.51 (t, *J* = 4.4 Hz, 1H); 6,27 (d, *J* = 6.3 Hz, 1 H); 6.32 (d, *J* = 6.3 Hz, 1 H); 6.79 (m, 2H); 7.02 (m, 2H) ppm.

**¹³C-NMR (100 MHz, CDCl₃):** 33.7 (CH); 37.3 (CH₂); 55.2 (CH₃); 55.6 (CH₃); 55.8 (CH₃); 93.9 (CH); 95.1 (CH); 106.4 (C); 114.2 (CH); 127.8 (CH); 133.6 (C); 153.0 (C); 157.4 (C); 158.6 (C); 160.6 (C); 167.8 (C=O) ppm.

**Massenspektrum (EI):** m/z (%) = 315 (20); 314 (M^{·+}, 100); 286 (38); 272 (14); 271 (74); 257 (14); 241 (32); 207 (19); 121 (11); 69 (8).

**Beispiel 3**: (4*S*)-4-(4-Hydroxyphenyl)-5,7-dimethoxy-chroman-2-on **(5)** und (4*R*)-4-(4-Hydroxyphenyl)-5,7-dimethoxy-chroman-2-one **(6)**

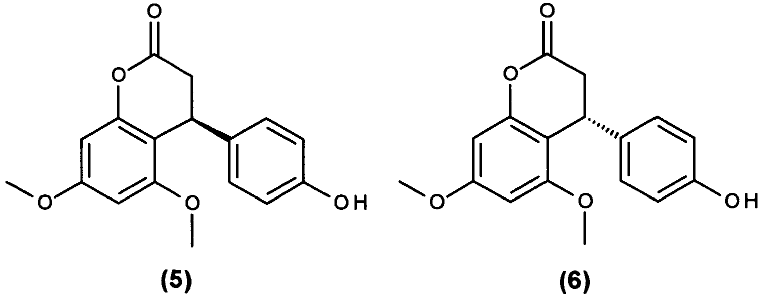

40 mmol 3,5-Dimethoxyphenol und 40 mmol 4-Hydroxyzimtsäure wurden in 70 ml 1,4-Dioxan und 1,5 ml konzentrierte Schwefelsäure vorgelegt und für 24 Stunden unter Rückfluss erhitzt. Das Reaktionsgemisch wurde auf 600 ml Wasser, wobei das Produkt ausfällt. Dar Feststoff wird abfiltriert und mit Ethanol/Wasser (1:1) gewaschen und anschließend zweimal aus Ethanol/Wasser (1:1) umkristallisiert.

### Ausbeute: 13,6 mmol (34% der Theorie)

### Analysendaten:

**¹H-NMR (400 MHz, CDCl₃):** 2.97 (m, 2H); 3.76 (s, 3H); 3.82 (s, 3H); 4.50 (t, *J* = 4.6 Hz, 1 H); 4.87 (s, 1 H); 6,28 (d, *J* = 2.3 Hz, 1 H); 6.32 (d, *J* = 2.3 Hz, 1 H); 6.71 (m, 2H); 7.97 (m, 2H) ppm.

**¹³C-NMR (100 MHz, CDCl₃):** 33.7 (CH); 37.3 (CH₂); 55.6 (CH₃); 55.8 (CH₃); 94.0 (CH); 95.2 (CH); 106.4 (C); 115.6 (CH); 128.0 (CH); 133.7 (C); 153.0 (C); 154.6 (C); 157.4 (C); 160.6 (C); 168.0 (C=O) ppm.

**Massenspektrum (EI):** m/z (%) = 301 (19); 300 (M˙⁺, 100); 272 (37); 258 (14); 257 (72); 241 (20); 207 (12); 154 (9); 153 (8); 69 (11).

**Beispiel 4:** (4*S*)-4-(3-Hydroxy-4-methoxy-phenyl)-7-methoxy-chroman-2-on **(7)** und (4*R*)-4-(3-Hydroxy-4-methoxy-phenyl)-7-methoxy-chroman-2-on **(8)**

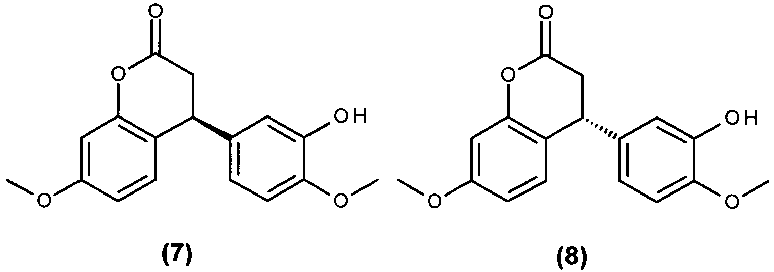

40 mmol 3-Methoxyphenol und 40 mmol Isoferulasäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml 5%-iger Natriumbicarbonatlösung gewaschen, über Natriumsulfat getrocknet und eingeengt. Das ölige Produkt wurde säulechromatographisch (Methyl-tert-butylether/ Hexan, 1:1) aufgereinigt und der so erhaltene Feststoff aus Ethanol/Wasser (1:1) umkristallisiert.

### Ausbeute: 4,1 mmol (10% der Theorie)

### Analysendaten:

**¹H-NMR (400 MHz, CDCl₃):** 2.94 (dd, *J* = 7.7/15.8 Hz, 1 H); 3.02 (dd, *J* = 5.9/15.8 Hz, 1 H); 3.80 (s, 3H); 3.86 (s, 3H); 4.18 (m, 1 H); 5.70 (s, 1 H); 6.59 - 6.67 (kB, 3H); 6.71 (d, *J* = 2.2 Hz, 1 H); 6,79 (d, *J* = 8.3 Hz, 1 H); 6.89 (m, 1 H) ppm.

**¹³C-NMR (100 MHz, CDCl₃):** 37.4 (CH); 39.5 (CH₂); 55.6 (CH₃); 56.0 (CH₃); 102.5 (CH); 110.7 (CH); 111.0 (CH); 113.7 (CH); 117.8 (C); 119.0 (CH); 128.9 (CH); 133.9 (C); 145.9 (C); 146.0 (C); 152.4 (C); 159.9 (C); 167.8 (C=O) ppm.

**Massenspektrum (EI):** m/z (%) = 301 (23); 300 (M^{·+}, 100); 282 (23); 267 (25); 257 (90); 243 (31); 241 (83); 227 (41); 176 (70); 133 (27).

### Beispiel 5: (4S)-4-(3-Hydroxy-4-methoxy-phenyl)-5,7-dimethoxy-chroman-2-on (9) und (4R)-4-(3-Hydroxy-4-methoxy-phenyl)-5,7-dimethoxy-chroman-2-on (10)

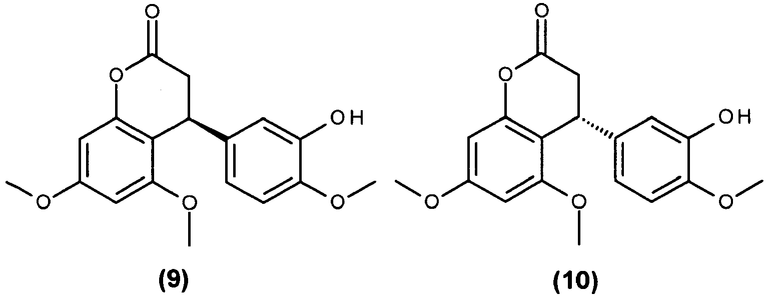

40 mmol 3,5-Dimethoxyphenol und 40 mmol Isoferulasäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml 5%-iger Natriumbicarbonatlösung gewaschen, über Natriumsulfat getrocknet und eingeengt. Der erhaltene Feststoff wurde aus Hexan/Aceton (5:2) umkristallisiert.

### Ausbeute: 7,1 mmol (18% der Theorie)

### Analysendaten:

**¹H-NMR (400 MHz, CDCl₃):** 2,94 (dd, *J* = 5.7/15.8 Hz, 1 H); 2,99 (dd, *J* = 3.4/15.8 Hz, 1 H); 3.75 (s, 3H); 3.81 (s, 3H); 3.83 (s, 3H); 4.47 (dd, *J* = 3.2/5.7 Hz, 1 H); 5.55 (s, 1 H); 6,27 (d, *J* = 2.3 Hz, 1 H); 6.31 (d, *J* = 2.3 Hz, 1 H); 6.56 (ddd, *J* = 0.6/2.2/8.3 Hz, 1 H); 6.70 (dd, *J* = 0.3/2.2 Hz, 1 H); 6.72 (d, *J* = 8.4 Hz, 1 H) ppm.

**¹³C-NMR (100 MHz, CDCl₃):** 33.9 (CH); 37.3 (CH₂); 55.6 (CH₃); 55.85 (CH₃); 55.93 (CH₃); 94.0 (CH); 95.1 (CH); 106.2 (C); 110.9 (CH); 113.4 (CH); 117.9 (CH); 134.8 (C); 145.6 (C); 145.7 (C); 153.1 (C); 157.4 (C); 160.6 (C); 167.7 (C=O) ppm.

**Massenspektrum (EI):** m/z (%) = 331 (20); 330 (M^{·+}, 100); 302 (10); 287 (41); 273 (12); 271 (17); 257 (10); 207 (11); 176 (30); 154 (38); 137 (11).

### Beispiel 6: (4S)-4-(4-Hydroxyphenyl)-7-propoxy-chroman-2-on (11) und (4R)-4-(4-Hydroxyphenyl)-7-propoxy-chroman-2-on (12)

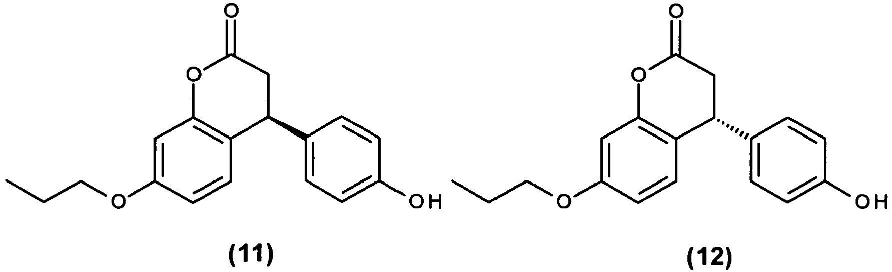

15 mmol 3-Propoxyphenol und 15 mmol 4-Hydroxyzimtsäure wurden in 70 ml 1,4-Dioxan und 1,0 ml konzentrierte Schwefelsäure vorgelegt und für 24 Stunden unter Rückfluss erhitzt. Das Reaktionsgemisch wurde auf 250 ml 5%-iger Natriumbicarbonatlösung gegossen und mit 300 ml Methyl-tert-butylether extrahiert. Die organische Phase wird anschließend nochmals mit 50 ml gesättigter Kochsalzlösung gewaschen, über Natriumsulfat getrocknet und eingeengt. Das ölige Produkt wurde säulechromatographisch (Methyl-tert-butylether/ Hexan, 1:1) aufgereinigt.

### Ausbeute: 4,3 mmol (29% der Theorie)

### Analysendaten:

**¹H-NMR (400 MHz, CDCl₃):** 1.03 (t, *J* = 7.4 Hz, 3H); 1.81 (tq, *J* = 6.6/7.4 Hz, 2H); 2.95 (dd, *J* = 7.6/15.7 Hz, 1 H); 3.03 (dd, *J* = 5.9/15.7 Hz, 1 H); 3.90 (t, *J* = 6.6 Hz, 2H); 4.22 (dd, *J* = 6.1/7.5 Hz, 1 H); 5.12 (bs, 1 H); 6.63 (dd, *J* = 2.5/8.4 Hz, 1 H); 6,66 (d, *J* = 2.5 Hz, 1 H); 6.78 (m, 2H); 6.86 (ddd, *J* = 0.4/0.9/8.4 Hz, 1 H); 7.0 (m, 2H) ppm.

**¹³C-NMR (100 MHz, CDCl₃):** 10.5 (CH₃); 22.5 (CH₂); 37.6 (CH₂); 39.3 (CH); 69.9 (CH₂); 103.0 (CH); 111.3 (CH); 115.9 (CH); 117.7 (C); 128.7 (CH); 128.8 (C); 132.9 (C); 152.3 (C); 155.0 (C); 159.5 (C); 168.1 (C=O) ppm.

**Massenspektrum (EI):** m/z (%) = 299 (19); 298 (M^{·+}, 100); 270 (13); 256 (25); 255 (51); 239 (17); 228 (25); 214 (11); 213 (39); 197 (15).

### Beispiel 7: (4S)-7-Ethoxy-4-(4-hydroxyphenyl)chroman-2-on (13) und (4R)-7-Ethoxy-4-(4-hydroxyphenyl)chroman-2-on (14)

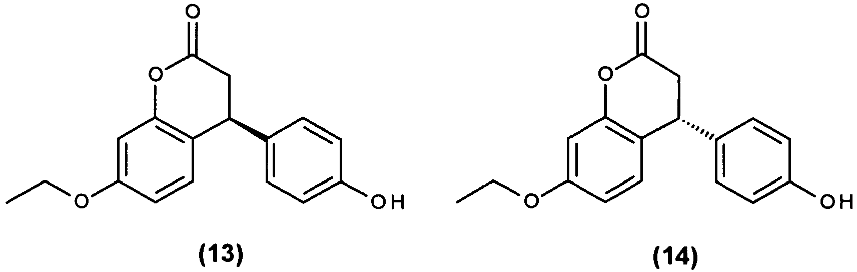

15 mmol 3-Ethoxyphenol und 15 mmol 4-Hydroxyzimtsäure wurden in 70 ml 1,4-Dioxan und 1,0 ml konzentrierte Schwefelsäure vorgelegt und für 24 Stunden unter Rückfluss erhitzt. Das Reaktionsgemisch wurde auf 250 ml 5%-iger Natriumbicarbonatlösung gegossen und mit 300 ml Methyl-tert-butylether extrahiert. Die organische Phase wird anschließend nochmals mit 50 ml gesättigter Kochsalzlösung gewaschen, über Natriumsulfat getrocknet und eingeengt. Das ölige Produkt wurde säulechromatographisch (Methyl-tert-butylether/ Hexan, 1:1) aufgereinigt.

### Ausbeute: 4,5 mmol (30% der Theorie)

### Analysendaten:

**¹H-NMR (400 MHz, CDCl₃):** 1.42 (t, *J* = 7.0 Hz, 3H); 2.94 (dd, *J* = 7.8/15.8 Hz, 1 H); 3.03 (dd, *J* = 5.9/15.8 Hz, 1 H); 4.02 (q, *J* = 7.0 Hz, 2H); 4.22 (dd, *J* = 5.9/7.9 Hz, 1 H); 4.87 (s, 1 H); 6.62 (dd, *J* = 2.5/8.4 Hz, 1 H); 6,67 (d, *J* = 2.6 Hz, 1 H); 6.79 (m, 2H); 6.86 (ddd, *J* = 0.4/0.9/8.4 Hz, 1 H); 7.01 (m, 2H) ppm.

**¹³C-NMR (100 MHz, CDCl₃):** 14.7 (CH₃); 37.6 (CH₂); 39.3 (CH); 63.9 (CH₂); 103.0 (CH); 111.3 (CH); 115.9 (CH); 117.8 (C); 128.78 (CH); 128.81 (CH); 133.0 (C); 152.4 (C); 154.9 (C); 159.3 (C); 167.9 (C=O) ppm.

**Massenspektrum (EI):** m/z (%) = 285 (19); 284 (M^{·+}, 100); 256 (26); 242 (16); 241 (74); 227 (8); 225 (23); 213 (21); 128 (9); 77 (8).

### Beispiel 8: (4S)-4-(4-Hydroxy-3-methoxy-phenyl)-7-methoxy-chroman-2-on (15) und (4R)-4-(4-Hydroxy-3-methoxy-phenyl)-7-methoxy-chroman-2-on (16)

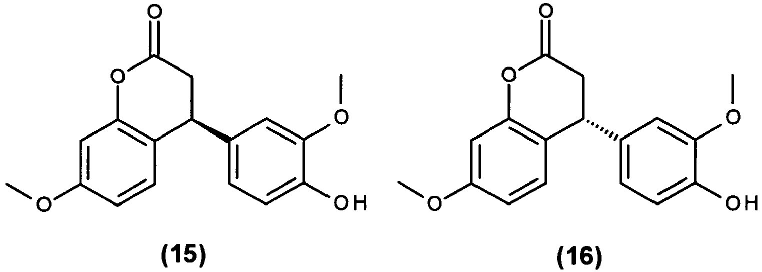

40 mmol 3-Methoxyphenol und 40 mmol Ferulasäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml gesättigter Natriumbicarbonatlösung gewaschen, über Natriumsulfat getrocknet und eingeengt. Das Rohprodukt wurde säulechromatographisch (Methyl-tert-butylether/ Hexan, 1:1) aufgereinigt.

### Ausbeute: 12,7 mmol (32% der Theorie)

### Analysendaten:

**¹H-NMR (400 MHz, CDCl₃):** 2,95 (dd, *J* = 8.1/15.6 Hz, 1 H); 3,04 (dd, *J* = 5.9/15.6 Hz, 1 H); 3.81 (s, 3H); 3.84 (d, *J* = 0.2 Hz, 3H); 4.21 (dd, *J* = 5.9/8.2 Hz, 1 H); 5.57 (d, *J* = 0.3 Hz, 1 H); 6,61 - 6.69 (kB, 4H); 6.88 (d, *J* = 8.1 Hz, 1 H); 6.89 (ddd, *J* = 0.3/0.8/8.4 Hz, 1 H) ppm.

**¹³C-NMR (100 MHz, CDCl₃):** 37.6 (CH₂); 39.9 (CH); 55.6 (CH₃); 55.9 (CH₃); 102.5 (CH); 109.7 (CH); 110.7 (CH); 114.8 (CH); 118.0 (C); 120.5 (CH); 128.9 (CH); 132.6 (C); 145.0 (C); 146.9 (C); 152.4 (C); 160.0 (C); 167.8 (C=O) ppm.

**Massenspektrum (EI):** m/z (%) = 301 (20); 300 (M^{·+}, 100); 272 (11); 267 (11); 257 (46); 243 (9); 242 (13); 241 (29); 227 (22); 176 (19).

### Beispiel 9: (4S)-4-(4-hydroxy-3-methoxy-phenyl)-5,7-dimethoxy-chroman-2-one (17) und (4R)-4-(4-Hydroxy-3-methoxy-phenyl)-5,7-dimethoxy-chroman-2-on (18)

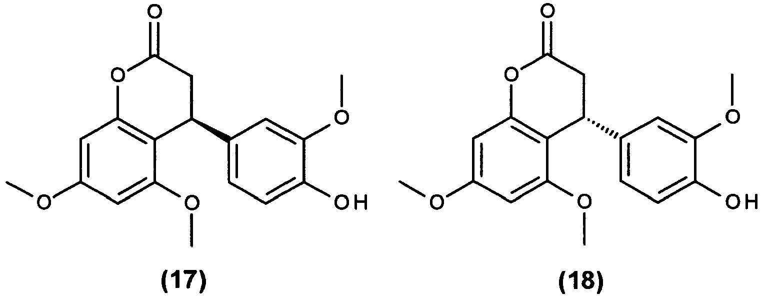

40 mmol 3,5-Dimethoxyphenol und 40 mmol Ferulasäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml 5%-iger Natriumbicarbonatlösung gewaschen, über Natriumsulfat getrocknet und eingeengt. Der erhaltene Feststoff wurde aus Methyl-tert-butylether/ Hexan umkristallisiert.

### Ausbeute: 17,4 mmol (44% der Theorie)

### Analysendaten:

**¹H-NMR (400 MHz, CDCl₃):** 2,97 (m, 2H); 3.76 (s, 3H); 3.81 (s, 3H); 3.83 (s, 3H); 4.49 (dd, *J* = 4.5/5.3 Hz, 1 H); 5.50 (d, *J* = 0.4 Hz, 1 H); 6,28 (d, *J* = 2.3 Hz, 1 H); 6.32 (d, *J* = 2.3 Hz, 1 H); 6.59 (m, 1 H); 6.62 (d, *J* = 2.1 Hz, 1 H); 6.78 (d, *J* = 8.1 Hz, 1 H) ppm.

**¹³C-NMR (100 MHz, CDCl₃):** 34.2 (CH); 37.4 (CH₂); 55.6 (CH₃); 55.82 (CH₃); 55.85 (CH₃); 94.0 (CH); 95.1 (CH); 106.4 (C); 109.2 (CH); 114.5 (CH); 119.5 (CH); 133.5 (C); 144.7 (C); 146.7 (C); 153.0 (C); 157.4 (C); 160.6 (C); 167.9 (C=O) ppm.

**Massenspektrum (EI):** m/z (%) = 331 (20); 330 (M^{·+}, 100); 302 (16); 287 (41); 272 (10); 271 (17); 257 (14); 207 (10); 176 (20); 154 (25).

### Beispiel 10: (4S)-4-(4-Hydroxyphenyl)-7-methoxy-chroman-2-on (19) und (4R)-4-(4-Hydroxyphenyl)-7-methoxy-chroman-2-on (20)

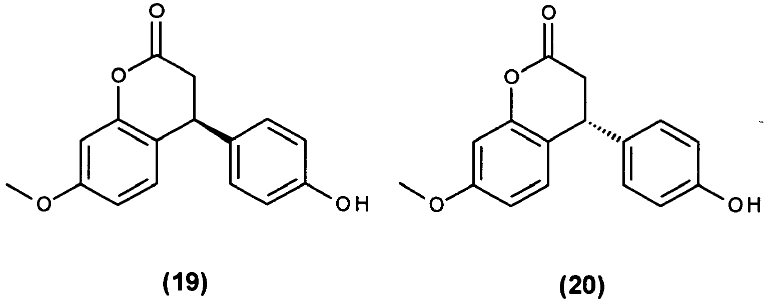

40 mmol 3-Methoxyphenol und 40 mmol 4-Hydroxyzimtsäure wurden in 70 ml 1,4-Dioxan und 2,0 ml konzentrierte Schwefelsäure vorgelegt und für 24 Stunden unter Rückfluss erhitzt. Das Reaktionsgemisch wurde auf 600 ml 5%-iger Natriumbicarbonatlösung gegossen und mit 300 ml Methyl-tert-butylether extrahiert. Die organische Phase wird anschließend nochmals mit 100 ml 5%-iger Natriumbicarbonatlösung gewaschen, über Natriumsulfat getrocknet und eingeengt. Der erhaltene Feststoff wurde aus Hexan/Aceton umkristallisiert.

### Ausbeute: 4,4 mmol (11% der Theorie)

### Analysendaten:

**¹H-NMR (400 MHz, CDCl₃):** 2,95 (dd, *J* = 7.8/15.7 Hz, 1 H); 3,03 (dd, *J* = 6.0/15.7 Hz, 1 H); 3.80 (s, 3H); 4.23 (dd, *J* = 6.2/7.3 Hz, 1 H); 4.89 (bs, 1 H); 6,64 (dd, *J* = 2.3/8.3 Hz, 1 H); 6.68 (d, *J* = 2.7 Hz, 1 H); 6.79 (m, 2H); 6.87 (ddd, *J* = 0.3/0.8/8.8 Hz, 1 H); 7.01 (m, 2H) ppm.

**¹³C-NMR (100 MHz, CDCl₃):** 37.5 (CH₂); 39.3 (CH); 55.6 (CH₃); 102.5 (CH); 110.8 (CH); 115.9 (CH); 117.9 (C); 128.8 (CH); 128.9 (CH); 132.9 (C); 152.4 (C); 154.9 (C); 160.0 (C); 167.9 (C=O) ppm.

**Massenspektrum (EI):** m/z (%) = 271 (18); 270 (M^{·+}, 100); 242 (29); 228 (20); 227 (97); 212 (9); 211 (37); 128 (9); 121 (9); 77 (10).

### Beispiel 11: (4S)-4-(4-Hydroxy-3-methoxy-phenyl)-7-propoxy-chroman-2-on (21) und (4R)-4-(4-Hydroxy-3-methoxy-phenyl)-7-propoxy-chroman-2-on (22)

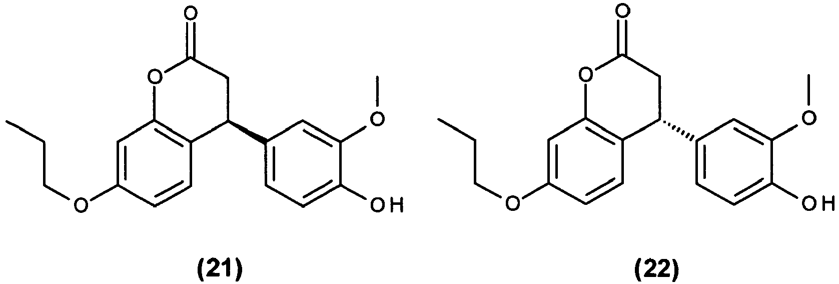

15 mmol 3-Propoxyphenol und 15 mmol Ferulasäure wurden in 70 ml 1,4-Dioxan und 1,0 ml konzentrierte Schwefelsäure vorgelegt und für 24 Stunden unter Rückfluss erhitzt. Das Reaktionsgemisch wurde auf 250 ml gesättigte Natriumbicarbonatlösung gegossen und mit 300 ml Methyl-tert-butylether extrahiert. Die organische Phase wird anschließend mit 50 ml gesättigter Kochsalzlösung gewaschen, über Natriumsulfat getrocknet und eingeengt. Das Rohprodukt wurde säulechromatographisch (Methyl-tert-butylether/ Hexan, 1:1) aufgereinigt.

### Ausbeute: 7,0 mmol (47% der Theorie)

### Analysendaten:

**¹H-NMR (400 MHz, CDCl₃):** 1.04 (t, *J* = 7.4 Hz, 3H); 1.81 (tq, *J* = 6.5/7.4 Hz, 2H); 2.95 (dd, *J* = 8.1/15.8 Hz, 1 H); 3.04 (dd, *J* = 5.9/15.8 Hz, 1 H); 3.83 (s, 3H); 3.91 (t, *J* = 6.6 Hz, 2H); 4.21 (dd, *J* = 5.8/8.2 Hz, 1 H); 5.59 (d, *J* = 0.3 Hz, 1 H); 6.61 - 6.68 (m, 4H); 6.87 (ddd, *J* = 0.4/0.9/8.4 Hz, 1 H); 6,87 (d, *J* = 8.1 Hz, 1 H) ppm.

**¹³C-NMR (100 MHz, CDCl₃):** 10.5 (CH₃); 22.5 (CH₂); 37.6 (CH₂); 39.9 (CH); 55.9 (CH₃); 69.9 (CH₂); 103.0 (CH); 109.7 (CH); 111.3 (CH); 114.8 (CH); 117.7 (C); 120.5 (CH); 128.8 (CH); 132.6 (C); 145.0 (C); 146.9 (C); 152.4 (C); 159.5 (C); 167.9 (C=O) ppm.

**Massenspektrum (EI):** m/z (%) = 329 (22); 328 (M^{·+}, 100); 286 (15); 285 (32); 269 (20); 255 (11); 243 (18); 227 (10); 213 (11); 176 (13).

### Beispiel 12: (4S)-5-hydroxy-4-(4-hydroxy-3-methoxy-phenyl)-7-methoxy-chroman-2-one (23) und (4R)-5-hydroxy-4-(4-hydroxy-3-methoxy-phenyl)-7-methoxy-chroman-2-one (24) sowie (4S)-7-hydroxy-4-(4-hydroxy-3-methoxy-phenyl)-5-methoxy-chroman-2-on (A) und (4R)-7-hydroxy-4-(4-hydroxy-3-methoxy-phenyl)-5-methoxy-chroman-2-on (B)

Eine Mischung aus 17,5 mmol *trans*-4-Hydroxyzimtsäure und 17,5 mmol 5-Methylresorcin in 40 ml 1,4-Dioxan wurde mit 0,5 ml konzentrierter Schwefelsäure versetzt und für 12 Stunden unter Rückfluss erhitzt. Nach Abkühlen wurde das Reaktionsgemisch auf 250 ml entmineralisiertes Wasser gegossen und mit 150 ml Essigsäureethylester (EE) extrahiert. Die organische Phase wurde je zweimal mit 50 ml entmineralisiertem Wasser und 50 ml 5%-iger Natriumbicarbonatlösung gewaschen und über Natriumsulfat getrocknet. Nach Entfernen des Lösungsmittels wurde das Produkt säulenchromatographisch (Laufmittel: Essigester/Hexan 2:3) an Silicagel aufgereinigt. Das Produkt bestand gemäß NMR aus zwei Isomeren, die im Verhältnis 37:63 (23/24:A/B) vorlagen. Die weitere Auftrennung wurde mittels präparativer HPLC durchgeführt (Phenomenex Luna C18 (2), 5µm, 150x21,5mm; Acetonitril/Wasser 75:25; 25 ml/min; 105 bar)

### Ausbeute: 9,8 mmol (56 % der Theorie)

### Beispiel 12a: (4S)-5-hydroxy-4-(4-hydroxy-3-methoxy-phenyl)-7-methoxy-chroman-2-one (23) und (4R)-5-hydroxy-4-(4-hydroxy-3-methoxy-phenyl)-7-methoxy-chroman-2-one (24)

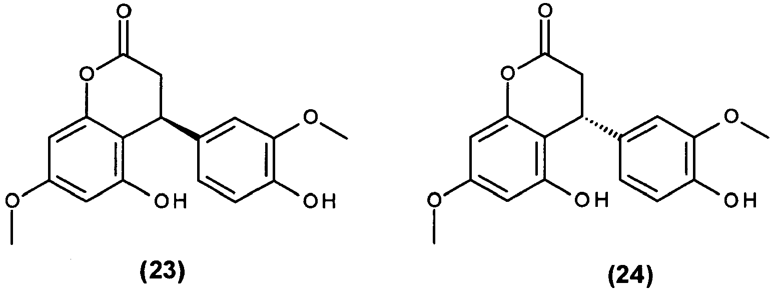

### Analysendaten:

**Retentionszeit:** 20,3 min - 21,8 min

**¹H-NMR (400 MHz, CDCl₃):** 2.97 (dd, *J* = 3.6/15.8 Hz; 1 H); 3.03 (dd, *J* = 6.1/15.8 Hz; 1 H); 3.79 (s, 3H); 3.82 (s, 3H); 4.44 (dd, *J* = 3.6/6.1 Hz, 1 H); 4.96 (bs, 1 H); 5.53 (bs, 1 H); 6.21 (d, *J* = 2.3 Hz, 1 H); 6.35 (d, *J* = 2.3 Hz, 1 H); 6.62 (d, *J* = 2.0 Hz; 1 H); 6.64 (dd, *J* = 2.0/8.6 Hz, 1 H); 6.82 (d, *J* = 8.6 Hz, 1 H) ppm.

**¹³C-NMR (100 MHz, (CD₃)₂SO):** 33.2 (CH); 37.1 (CH₂); 55.1 (CH₃); 55.5 (CH₃); 93.0 (CH); 97.5 (CH); 105.2 (C); 111.3 (CH); 115.2 (CH); 118.2 (CH); 132.7 (C); 145.3 (C); 147.5 (C); 154.0 (C); 155.3 (C); 159.5 (C); 167.8 (C=O) ppm.

**HRMS** [M-H; C₁₇H₁₅O₆]: ber.: 315,0874 gef.: 315.0902

### Beispiel 12b: (4S)-7-hydroxy-4-(4-hydroxy-3-methoxy-phenyl)-5-methoxy-chroman-2-on (A) und (4R)-7-hydroxy-4-(4-hydroxy-3-methoxy-phenyl)-5-methoxy-chroman-2-on (B)

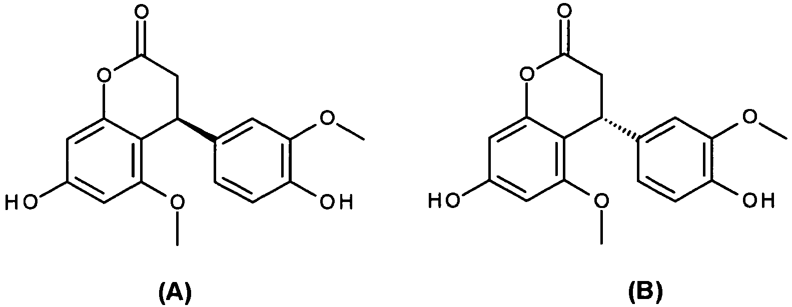

### Analysendaten:

**Retentionszeit:** 17,0 min-19,8 min

**¹H-NMR (400 MHz, CDCl₃):** 2.95 - 2.99 (kB, 2H); 3.76 (s, 3H); 3.81 (s, 3H); 4.48 (dd, *J* = 3.9/4.7 Hz, 1 H); 5.19 (bs, 1 H); 5.49 (bs, 1 H); 6.25 (d, *J* = 2.3 Hz, 1 H); 6.27 (d, *J* = 2.3 Hz, 1 H); 6.59 (dd, *J* = 2.1/7.9 Hz; 1 H); 6.61 (d, *J* = 2.1 Hz, 1 H); 6.79 (d, *J* = 7.9 Hz, 1 H) ppm.

**¹³C-NMR (100 MHz, (CD₃)₂SO):** 33.1 (CH); 38.8 - 40.1 (CH₂; verdeckt durch Lösungsmittelsignal); 55.5 (CH₃); 55.6 (CH₃); 95.4 (CH); 95.6 (CH); 104.3 (C); 111.1 (CH); 115.2 (CH); 118.1 (CH); 132.7 (C); 145.3 (C); 147.5 (C); 152.5 (C); 157.0 (C); 158.2 (C); 167.7 (C=O) ppm.

**HRMS** [M-H; C₁₇H₁₅O₆]: ber.: 315,0874 gef.: 315.0920

### Beispiel 13: (4S)-7-Ethoxy-4-(4-hydroxy-3-methoxy-phenyl)chroman-2-on (25) und (4R)-7-Ethoxy-4-(4-hydroxy-3-methoxy-phenyl)chroman-2-on (26)

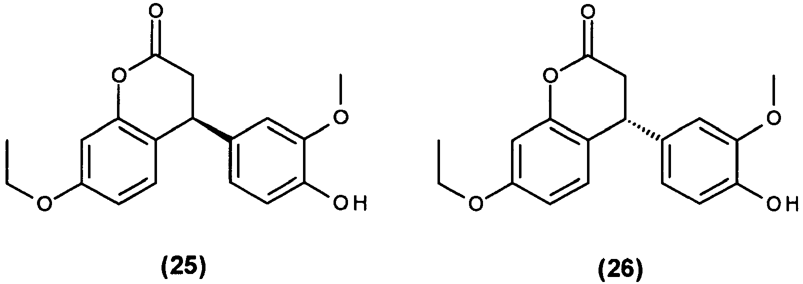

15 mmol 3-Ethoxyphenol und 15 mmol Ferulasäure wurden in 70 ml 1,4-Dioxan und 1,0 ml konzentrierte Schwefelsäure vorgelegt und für 24 Stunden unter Rückfluss erhitzt. Das Reaktionsgemisch wurde auf 250 ml gesättigte Natriumbicarbonatlösung gegossen und mit 300 ml Methyl-tert-butylether extrahiert. Die organische Phase wird anschließend mit 50 ml gesättigter Kochsalzlösung gewaschen, über Natriumsulfat getrocknet und eingeengt. Das Rohprodukt wurde säulechromatographisch (Methyl-tert-butylether/ Hexan, 1:1) aufgereinigt.

### Ausbeute: 4,3 mmol (29% der Theorie)

### Analysendaten:

**¹H-NMR (400 MHz, CDCl₃):** 1.42 (t, *J* = 7.0 Hz, 3H); 2.95 (dd, *J* = 5.9/15.6 Hz, 1 H); 3.04 (dd, *J* = 5.9/15.6 Hz, 1 H); 3.83 (s, 3H); 4.01 (q, *J* = 7.0 Hz, 2H); 4.21 (dd, *J* = 5.8/8.1 Hz, 1 H); 5.60 (bs, 1 H); 6.61 - 6.67 (m, 4H); 6.87 (ddd, *J* = 0.4/0.9/8.4 Hz, 1 H); 6,87 (d, *J* = 8.0 Hz, 1 H) ppm.

**¹³C-NMR (100 MHz, CDCl₃):** 14.7 (CH₃); 37.6 (CH₂); 39.9 (CH); 55.9 (CH₃); 63.8 (CH₂); 103.0 (CH); 109.7 (CH); 111.2 (CH); 114.8 (CH); 117.8 (C); 120.5 (CH); 128.8 (CH); 132.6 (C); 145.0 (C); 146.9 (C); 152.4 (C); 159.3 (C); 167.9 (C=O) ppm.

**Massenspektrum (EI):** m/z (%) = 315 (20); 314 (M^{·+}, 100); 286 (11); 272 (8); 271 (39); 257 (11); 255 (22); 243 (10); 241 (14); 176 (11).

### Anwendungsbeispiel 1: Untersuchungen zu der Verstärkung des Süßeindruckes bzw. zu der Bittermaskierung

### Anwendungsbeispiel 1a: Verstärkung des Süßeindruckes einer Zuckerlösung

Um die Verstärkung des Süßeindruckes zu quantifizieren, wurde die Süße einer 5 Gew.-%igen Sucroselösung (bestehend aus 5 Gew.-% Sucrose in Wasser) und einer Probe, die aus Wasser, 5 Gew.-% Sucrose und einer bestimmten (d.h. die unten jeweils angegebene) Menge an Testsubstanz bestand, von einer Expertengruppe ermittelt. Die Bewertung erfolgte auf einer Skala von 1 [nicht süß] bis 10 [extrem süß]. Die Verstärkung des Süßeindruckes (in %) wurde aus den jeweiligen Durchschnittswerten der Bewertungen der Sucroselösung und der jeweiligen zu untersuchenden Probe enthaltend die Testsubstanz berechnet. Bei den eingesetzten Testsubstanzen handelte es sich jeweils um ein racemisches Gemisch der nachfolgend angegebenen erfindungsgemäß einzusetzenden Verbindungen.

| **Substanz** | **Süß-Eindruck (1-10)** | | **% Verstärkung des Süß-Eindrucks** |
|---|---|---|---|
| | **ohne** | **mit** | |
| **(1) + (2),** Verhältnis 1:1, 50 ppm | 4,8 ± 0,9 | 5,4 ± 0,9 | 12 % (p < 0,08) |
| **(11) + (12),** Verhältnis 1:1, 25 ppm | 5,8 ± 1,1 | 6,6 ± 1,5 | 14 % (p < 0,09) |
| **(15) + (16),** Verhältnis 1:1, 50 ppm | 5,2 ± 0,9 | 6,7 ± 1,4 | 29 % (p < 0,001) |
| **(17) + (18),** Verhältnis 1:1, 50 ppm | 4,2 ± 1,4 | 7,1 ± 1,8 | 37 % (p < 0,002) |
| **(19) + (20),** Verhältnis 1:1, 50 ppm | 4,7 ± 1,1 | 5,4 ± 1,1 | 14 % (p < 0,09) |
| **(23) + (24),** Verhältnis 1:1, 50 ppm | 4,2 ± 0,8 | 7,6 ± 1,5 | 47 % (p < 0,00001) |

### Anwendungsbeispiel 1a-1: Verstärkung des Süßeindruckes einer Zuckerlösung

Um die Verstärkung des Süßeindruckes zu quantifizieren, wurde die Süße einer 5 Gew.-%igen Sucroselösung (bestehend aus 5 Gew.-% Sucrose in Wasser) und einer Probe, die aus Wasser, 5 Gew.-% Sucrose und einer bestimmten (d.h. die unten jeweils angegebene) Menge an Testsubstanz bestand, von einer Expertengruppe ermittelt. Die Bewertung erfolgte auf einer Skala von 1 [nicht süß] bis 10 [extrem süß]. Die Verstärkung des Süßeindruckes (in %) wurde aus den jeweiligen Durchschnittswerten der Bewertungen der Sucroselösung und der jeweiligen zu untersuchenden Probe enthaltend die Testsubstanz berechnet.

| **Substanz** | **Süß-Eindruck (1-10)** | | **% Verstärkung des Süß-Eindrucks** |
|---|---|---|---|
| | **ohne** | **mit** | |
| **(23) + (24),** Verhältnis 1:1, **50 ppm** | 4,2 ± 0,8 | 7,6 ± 1,5 | 47 % (p < 0,00001) |
| **(A) + (B),** Verhältnis 1:1, **50 ppm** | 5,4 ± 1,6 | 5,8 ± 1,4 | 8 % (p < 0,5) |
| Mischung nach **Beispiel 12** bestehend aus: | 5,1 ± 1,5 | 7,4 ± 1,5 | 44 % (p < 0,0002) |
| **(23) + (24),** Verhältnis 1:1, **37 ppm** | | | |
| **(A) + (B),** Verhältnis 1:1, **63 ppm** | | | |

Dieses Beispiel verdeutlicht, dass strukturell ähnliche Verbindungen mit einem entsprechenden Rest an Position C5 (z.B. Verbindungen **(A)** und **(B)**) anstelle von Position C7 (z. B. Verbindungen **(23)** und **(24)**) deutlich weniger stark süßverstärkend wirken als erfindungsgemäß einzusetzende Verbindungen.

### Anwendungsbeispiel 1b: Bitter-Reduzierung einer Bitterstofflösung

Um die Reduzierung (d.h. die Maskierung oder Verminderung) des Bitter-Eindruckes in einer Probe zu quantifizieren, wurde die Bitterkeit einer Lösung enthaltend 500 ppm Coffein oder 100 ppm Naringin von einer Expertengruppe jeweils mit einer Probe verglichen, die 500 ppm Coffein oder 100 ppm Naringin und zusätzlich die jeweils angegebene Menge einer (hinsichtlich der Fähigkeit zur Bitter-Reduzierung) zu bewertende Substanz enthielt (Bewertungsskale: 1 [nicht bitter] bis 10 [extrem bitter]). Für die Auswertung, d.h. die Berechnung der Reduktion (in %) des Bittereindruckes wurden jeweils die Durchschnittswerte der Bewertung der Expertengruppe verwendet.

| Test-Substanz | Bitterstoff | Bitter-Eindruck (1-10) | | % Reduktion des Bitter-Eindruckes (Signifikanz) |
|---|---|---|---|---|
| | | **ohne** | **mit** | |
| 25 ppm **(15) + (16)** (Verhältnis 1:1) | 500 ppm Coffein | 4,2 ± 1,6 | 3,2 + 1,4 | 23,7% (p < 0,06) |
| 50 ppm **(19) + (20)** (Verhältnis 1:1) | 500 ppm Coffein | 3,9 ± 1.1 | 3,6 + 1,5 | 9,9 % (p < 0,6) |
| 25 ppm **(15) + (16)** (Verhältnis 1:1) | 100 ppm Naringin | 4,1 ± 1,8 | 3,2 ± 1,9 | 20,5 % (p < 0,2) |
| 25 ppm **(17) + (18)** (Verhältnis 1:1) | 100 ppm Naringin | 6,1 ± 2,2 | 4,8 ± 2,5 | 9,9 % (p < 0,2) |

### Anwendungsbeispiel 2: Halbfertigwaren

| | Zubereitung (Einsatz in Gew.-%) | | | | | | | |
|---|---|---|---|---|---|---|---|---|
| Inhaltsstoff | **A** | **B** | **C** | **D** | **E** | **F** | **G** | **H** |
| "Flüssigzucker", enthaltend 80 % Sucrose | 99,85 | - | - | - | - | - | 99,80 | - |
| Verbindung **(15) + (16)** (Verhältnis 1:1) | 0,025 | 10 | | 9 | | 4 | | 7 |
| Verbindung **(23) + (24)** (Verhältnis 1:1) | 0,025 | | 8 | | 10 | 5 | 0,05 | |
| Rebaudiosid A 98 % | - | 80 | - | - | - | 25 | 0,01 | 68,5 |
| Steviosid 95 % | - | - | 70 | - | - | - | - | - |
| Balansin A | 0,05 | 5 | | - | 70 | 7 | 0,02 | 9 |
| Balansin B | - | 5 | 15 | - | - | 4 | - | 9 |
| Extrakt aus *Mycetia balansae* enthaltend 5 Gew-% Balansin A und 5 Gew.-% Balansin B, bezogen auf das Gesamtgewicht das Extraktes | - | - | - | 21 | | | | |
| Extrakt aus *Rubus suavissimus,* enthaltend 5 Gew.-% Rubusosid, bezogen auf das Gesamtgewicht des Extraktes z.B. von PlantExtrakt | - | - | - | 25 | - | 25 | 0,07 | - |
| Extrakt aus *Hydrangea dulcis* enthaltend 8 % Phyllodulcin, bezogen auf das Gesamtgewicht des Extraktes | - | - | - | 25 | - | 25 | - | - |
| Phloretin | 0,02 | - | 4 | 5 | 3,2 | 3,5 | 0,02 | 5 |
| Hesperetin | 0,02 | - | 1 | 5 | 0,8 | 1 | 0,02 | 1 |
| 3',7-Dihydroxy-4'-methoxyflavan gemäß EP 2 253 226 | 0,01 | - | 2 | 8 | - | - | 0,01 | - |
| Neohesperidindihydrochalkon | - | - | - | - | - | 0,5 | - | - |
| Homoeriodictyol-Natriumsalz | - | - | - | - | 16 | - | - | - |
| Vanillin, natürlich | - | - | - | 2 | - | - | - | - |
| Zuckerdestillat aus Rohrzucker (z.B. Treatt) | - | - | - | - | - | - | - | 0,5 |

Die Inhaltsstoffe werden in den oben angegebenen Mengenverhältnissen gemischt und können dann in dieser Form (weiter) verwendet werden. Die typische Dosierung der Zubereitungen A und G im Fertigprodukt liegt im Bereich von 7 bis 15 Gew.-%, bezogen auf das Gesamtgewicht des Fertigproduktes. Die typische Dosierung der übrigen Zubereitungen des Anwendungsbeispiels 5 liegt im Bereich von 0,01 bis 0,1 Gew.-%, bevorzugt bei 0,03 bis 0,06 Gew.-%, bezogen auf das Gesamtgewicht des Fertigproduktes.

### Anwendungsbeispiel 3: Erfindungsgemäße Gemische bzw. Halbfertigwaren

| | Zubereitung (Einsatz in Gew.-%) | | | | | | | |
|---|---|---|---|---|---|---|---|---|
| Inhaltsstoff | **A** | **B** | **C** | **D** | **E** | **F** | **G** | **H** |
| Verbindung **(17) + (18)** (Verhältnis 1:1) | 80 | 20 | | 25 | | 20 | | 15 |
| Verbindung **(23) + (24)** (Verhältnis 1:1) | 20 | 80 | 50 | | 20 | 20 | 20 | 15 |
| Balansin A | | | | 25 | | | | 5 |
| Balansin B | | | | | | | | 5 |
| Rebaudiosid A 98 % | | | 50 | 25 | | | | 20 |
| Steviosid 95 % | | | | 25 | | | | |
| Saccharin^{®}-Natriumsalz | | | | | | 20 | | |
| Cyclamat^{®} | | | | | 75 | | | |
| Acesulfam^{®} K | | | | | | 20 | | |
| Aspartam^{®} | | | | | | 20 | | |
| Neotam^{®} | | | | | 5 | | | |
| Thaumatin | | | | | | | | 20 |
| Sucralose^{®} | | | | | | | 80 | |
| Glycyrrhizin-Ammoniumsaz | | | | | | | | 20 |

Die Inhaltsstoffe werden in den oben angegebenen Mengenverhältnissen gemischt und zur Süßung von oral konsumierbaren Zubereitungen verwendet. Die typische Dosierung der Mischungen A bis H im Fertigprodukt liegt im Bereich von 0,001 bis 0,5 Gew.-%, bevorzugt im Bereich von 0,003 bis 0,1 Gew.-%, bezogen auf das Gesamtgewicht des Fertigproduktes.

### Anwendungsbeispiel 4: Halbfertigwaren (mit nicht-kalorischen Zuckern und/oder Zuckeralkoholen)

| | Zubereitung (Einsatz in Gew.-%) | | | | | | | |
|---|---|---|---|---|---|---|---|---|
| Inhaltsstoff | **A** | **B** | **C** | **D** | **E** | **F** | **G** | **H** |
| Zubereitung A aus Anwendungsbeispiel 3 | | 0,05 | | | 0,05 | | | 0,05 |
| Zubereitung C aus Anwendungsbeispiel 3 | | | 0,05 | | | 0,05 | | |
| Zubereitung H aus Anwendungsbeispiel 3 | | | | 0,05 | | | 0,05 | |
| Verbindung **(23) + (24)** (Verhältnis 1:1) | 0,05 | | | | | | | |
| Maltit | 50 | 10 | | | | | | |
| Mannit | | 10 | | | | | | |
| Sorbitol | 20 | 20 | | | | | | |
| Erythritol | 29,95 | 59,95 | 99,95 | 99,95 | | 50 | 50 | 99,95 |
| Xylitol | | | | | | | 49,95 | |
| Palatinose | | | | | 50 | | | |
| Tagatose | | | | | 49,95 | 49,95 | | |

Die Stoffe werden in den oben angegebenen Mengenverhältnissen gemischt und zur Süßung von oral konsumierbaren Zubereitungen verwendet. Die typische Dosierung der Halbfertigwaren A bis H im Fertigprodukt liegt im Bereich von 0,01 bis 80 Gew.-%, bevorzugt liegt im Bereich von 0,1 bis 50 Gew.-%, besonders bevorzugt liegt im Bereich von 0,1 bis 10 Gew.-%, jeweils bezogen auf das Gesamtgewicht des Fertigproduktes. Die Halbfertigwaren A bis H können als Süßungsmittel z.B. auch direkt für Kaffee oder Tee eingesetzt werden.

### Anwendungsbeispiel 5: Sprühgetrocknete Zubereitung als Halbfertigware zur Aromatisierung von Fertigwaren

| **Inhaltsstoff** | **Einsatz in Gew.-%** | | | | |
|---|---|---|---|---|---|
| **Zubereitung** | **A** | **B** | **C** | **D** | **E** |
| Trinkwasser | 60,8 | 60,8 | 60,8 | 60,8 | 60,8 |
| Maltodextrin aus Weizen | 24,3 | 24,3 | 24,3 | 24,3 | 24,3 |
| Gummi Arabicum | 6,1 | 6,1 | 6,1 | 6,1 | 6,1 |
| Verbindung **(17) + (18)** (Verhältnis 1:1) | 8,8 | 3,3 | 4,0 | - | - |
| Verbindung **(23) + (24)** (Verhältnis 1:1) | - | 3,3 | 1,5 | 3,3 | 4,4 |
| Hesperetin | - | 2,2 | - | - | 1,1 |
| Homoeriodictyol-Natriumsalz | - | - | - | 5,5 | 3,3 |
| Phloretin | - | - | 3,3 | - | - |

Das Trinkwasser wird in einem Behälter vorgelegt und das Maltodextrin und das Gummi Arabicum darin gelöst. Anschließend werden die Aromastoffe mit einem Turrax in die Trägerstofflösung emulgiert. Die Temperatur der Sprühlösung sollte 30°C nicht überschreiten. Das Gemisch wird dann sprühgetrocknet (Solltemperatur Eingang: 185 - 195°C, Solltemperatur Ausgang: 70 - 75°C).

### Anwendungsbeispiel 6: Lösungen der Halbfertigwaren

Die Gemische und Halbfertigwaren aus den obigen Anwendungsbeispielen 5, 6 und 7 können auch mit Wasser, Propylenglycol, Glycerin bzw. Ethanol oder bevorzugt mit Gemischen der vorgenannten Lösungsmittel (z.B. Wasser-Propylenglycol, Wasser-Glycerin, Wasser-Ethanol, Glycerin-Ethanol, Glycerin-Propylenglycol, Propylenglycol-Ethanol) beispielsweise als 1 - 20%ige Lösung, bevorzugt 2 - 10 %ige, besonders bevorzugt 5 %ige Lösung aufgenommen und durch leichtes Erwärmen vollständig gelöst werden.

### Anwendungsbeispiel 7: Zuckerreduziertes Erfrischungsgetränk Typ Zitrone

| **Inhaltsstoff** | **Einsatz in Gew.-%** | | | | | |
|---|---|---|---|---|---|---|
| **Zubereitung** | **A** | **B** | **C** | **D** | **E** | **F** |
| Zucker (Sucrose) | 8 | 8 | 8 | 8 | 8 | 7 |
| Zitronensäure | 0,15 | 0,15 | 0,15 | 0,15 | 0,15 | 0,15 |
| Zitronenaroma | 0,1 | 0,1 | 0,1 | 0,1 | 0,1 | 0,1 |
| Verbindung **(17) + (18)** (Verhältnis 1:1) | 0,005 | - | 0,0020 | - | 0,0025 | - |
| Verbindung **(23) + (24)** (Verhältnis 1:1) | - | 0,005 | 0,0020 | 0,0025 | - | 0,005 |
| Balansin A | - | - | - | - | 0,001 | 0,001 |
| Balansin B | - | - | - | 0,002 | - | - |
| Phloretin | - | - | - | - | 0,001 | - |
| Hesperetin | - | - | 0,010 | - | - | - |
| 3',7-Dihydroxy-4'-methoxyflavan gemäß EP 2 253 226 | - | - | - | - | - | 0,0025 |
| Extrakt aus *Rubus suavissimus,* enthaltend 5 Gew.-% Rubusosid bez. auf das Gesamtgewicht des Extraktes | - | - | - | 0,010 | - | - |
| Extrakt aus *Hydrangea dulcis* enthaltend 8 % Phyllodulcin, bezogen auf das Gesamtgewicht des Extraktes | | - | - | - | - | 0,010 |
| Wasser | ad 100 | | | | | |

Die Zutaten wurden in der angegebenen Reihenfolge gemischt und mit Wasser auf 100 % aufgefüllt. Die Mischungen werden in Glasflaschen gefüllt und carbonisiert.

### Anwendungsbeispiel 8: Softdrink Typ "Cola"

| | Zubereitung (Einsatz in Gew.-%) | | | | | | |
|---|---|---|---|---|---|---|---|
| Inhaltsstoff | **A** | **B** | **C** | **D** | **E** | **F** | **G** |
| Saccharose | 0 | 8 | 7 | 7 | - | 7 | - |
| Glucose/Fructose-Sirup aus Mais, enthaltend 55 Gew.-% Fructose | - | - | - | - | 8 | - | 7 |
| Aromakomposition A aus Anwendungsbeispiel 2 | 10 | - | - | - | - | - | - |
| Aromakomposition B aus Anwendungsbeispiel 3 | - | 0,005 | - | - | - | - | - |
| Aromakomposition C aus Anwendungsbeispiel 3 | - | - | 0,0125 | - | - | - | - |
| Aromakomposition D aus Anwendungsbeispiel 3 | - | - | - | 0,015 | - | - | - |
| Aromakomposition E aus Anwendungsbeispiel 3 | - | - | - | - | 0,03 | - | - |
| Aromakomposition F aus Anwendungsbeispiel 3 | - | - | - | - | - | 0,01 | - |
| Aromakomposition H aus Anwendungsbeispiel 3 | - | - | - | - | - | - | 0,015 |
| Phosphorsäure | 0,07 | 0,07 | 0,07 | 0,07 | 0,07 | 0,07 | 0,07 |
| Citronensäure | 0,06 | 0,06 | 0,06 | 0,06 | 0,06 | 0,06 | 0,06 |
| Zuckercouleur | 0,14 | 0,14 | 0,14 | 0,14 | 0,14 | 0,14 | 0,14 |
| Coffein | 0,01 | 0,01 | 0,01 | 0,01 | 0,01 | 0,01 | 0,01 |
| Getränke-Emulsion Typ "Cola" | 0,05 | 0,05 | 0,05 | 0,05 | 0,05 | 0,05 | 0,05 |
| Wasser | ad 100 | | | | | | |

Die Zutaten wurden in der angegebenen Reihenfolge gemischt, in Flaschen gefüllt und carbonisiert.

### Anwendungsbeispiel 9: Zuckerreduziertes Erfrischungsgetränk Typ "Cola"

Zubereitung A: Vergleichszubereitung mit 10 Gew.-% Zucker

Zubereitung B-G: erfindungsgemäße zuckerreduzierte Zubereitungen mit 8 Gew.-% Zucker

| | Zubereitung (Einsatz in Gew.-%) | | | | | | |
|---|---|---|---|---|---|---|---|
| Inhaltsstoff | **A** | **B** | **C** | **D** | **E** | **F** | **G** |
| Saccharose | 10 | 8 | 8 | 8 | - | - | - |
| Glucose/Fructose-Sirup aus Mais, enthaltend 55 Gew.-% Fructose | - | - | - | - | 8 | 8 | 8 |
| Verbindung **(15) + (16)** (Verhältnis 1:1) | - | - | 0,003 | 0,0025 | - | 0,003 | 0,0025 |
| Verbindung **(23) + (24)** (Verhältnis 1:1) | - | 0,005 | - | 0,0025 | 0,005 | - | 0,0025 |
| Phosphorsäure | 0,07 | 0,07 | 0,07 | 0,07 | 0,07 | 0,07 | 0,07 |
| Citronensäure | 0,06 | 0,06 | 0,06 | 0,06 | 0,06 | 0,06 | 0,06 |
| Zuckercouleur | 0,14 | 0,14 | 0,14 | 0,14 | 0,14 | 0,14 | 0,14 |
| Coffein | 0,01 | 0,01 | 0,01 | 0,01 | 0,01 | 0,01 | 0,01 |
| Getränke-Emulsion Typ "Cola" | 0,05 | 0,05 | 0,05 | 0,05 | 0,05 | 0,05 | 0,05 |
| Wasser | auf 100 % auffüllen | | | | | | |

Die Zutaten wurden in der angegebenen Reihenfolge gemischt und mit Wasser auf 100 % aufgefüllt. Die Mischungen werden in Glasflaschen gefüllt und carbonisiert.

### Anwendungsbeispiel 12: Kaugummi

| **Teil** | **Inhaltsstoff** | **Einsatz in Gew.-%** |
|---|---|---|
| A | Kaugummibase, Company "Jagum T" | 30,95 |
| B | Sorbit, pulverisiert | 39,00 |
| | Isomalt^{®} (Palatinit GmbH) | 9,50 |
| | Xylit | 2,00 |
| | Mannit | 3,00 |
| | Aspartam^{®} | 0,10 |
| | Acesulfam^{®} K | 0,10 |
| | Emulgum^{®} (Colloides Naturels, Inc.) | 0,30 |
| C | Sorbitol, 70% | 14,00 |
| | Glycerin | 1,00 |
| D | Verbindung **(23) + (24)** (Verhältnis 1:1) | 0,05 |

Teile A bis D werden gemischt und intensiv geknetet. Die Rohmasse kann z.B. in Form von dünnen Streifen zu verzehrsfertigen Kaugummis verarbeitet werden.

### Anwendungsbeispiel 13: Zahnpasta

| **Teil** | **Inhaltsstoff** | **Einsatz in Gew.-%** |
|---|---|---|
| A | Demineralisiertes Wasser | 23,08 |
| | Sorbitol (70%) | 45,00 |
| | Solbrol^{®} M, Natriumsalz (Bayer AG, p-Hydroxybenzoesäurealkylester) | 0,15 |
| | Trinatriumphosphat | 0,10 |
| | Verbindung **(15) + (16)** (Verhältnis 1:1) | 0,02 |
| | Natriummonofluorphosphat | 1,12 |
| | Polyethylenglycol 1500 | 5,00 |
| B | Sident 9 (abrasives Siliciumdioxid) | 10,00 |
| | Sident 22 S (verrlickendes Siliciumdioxid) | 8,00 |
| | Natriumcarboxymethylcellulose | 0,90 |
| | Titandioxid | 0,50 |
| C | Demineralisertes Wasser | 4,53 |
| | Natriumlaurylsulfat | 1,50 |
| D | Pfefferminzaroma | 0,1 |

Die Inhaltsstoffe der Teile A und B werden jeweils für sich vorgemischt und zusammen unter Vakuum bei 25 - 30°C 30 min gut verrührt. Teil C wird vorgemischt und zu A und B gegeben; D wird hinzugefügt und die Mischung unter Vakuum bei 25 - 30°C 30 min gut verrührt. Nach Entspannung ist die Zahnpasta fertig und kann abgefüllt werden.

### Anwendungsbeispiel 14: Zuckerfreie Hartkaramelle

| **Inhaltsstoff** | **Gehalt (Angaben in Gew.-%)** | | | |
|---|---|---|---|---|
| | **A** | **B** | **C** | **D** |
| Palatinit, Typ M | 75,00 | 74,00 | 75,50 | 75,00 |
| Citronensäure | - | 1,0 | 0,5 | - |
| Wasser | 24,885 | 24,844 | 23,88 | 24,8815 |
| Farbstoff gelb | - | 0,01 | - | - |
| Farbstoff rot | - | - | 0,01 | - |
| Farbstoff blau | 0,01 | - | - | 0,01 |
| Pfefferminzaroma | 0,1 | - | - | 0,1 |
| Zitronenaroma | - | 0,1 | - | - |
| Rotfruchtaroma | - | - | 0,1 | - |
| Rebaudiosid A 98 % | - | 0,040 | - | - |
| Verbindung **(15) + (16)** (Verhältnis 1:1) | - | - | 0,010 | 0,005 |
| Verbindung **(23) + (24)** (Verhältnis 1:1) | 0,005 | 0,002 | - | - |
| Hesperetin | - | 0,001 | - | 0,001 |
| Phloretin | - | 0,002 | - | - |
| 3',7-Dihydroxy-4'-methoxyflavan gemäß EP 2 253 226 | - | - | - | 0,0025 |

Palatinit wurde gegebenenfalls nach Zugabe der Zitronensäure mit Wasser gemischt und die Mischung bei 165 °C aufgeschmolzen und anschließend auf 115°C abgekühlt. Das Aroma und die anderen Bestandteile wurden zugegeben und nach dem Durchmischen in Formen gegossen, nach dem Erstarren aus den Formen entfernt und anschließend einzeln verpackt.

### Anwendungsbeispiel 15: Zuckerreduzierter Kochpudding

Zubereitung A: Vergleichszubereitung mit vollem Zuckergehalt
Zubereitung B: Vergleichszubereitung mit reduziertem Zuckergehalt
Zubereitung C-F: erfindungsgemäße Zubereitungen mit reduziertem Zuckergehalt

| | **Zubereitung (Angaben in Gew.-%)** | | | | | |
|---|---|---|---|---|---|---|
| **Inhaltsstoff** | **A** | **B** | **C** | **D** | **E** | **F** |
| Sucrose | 7,8 | 5,4 | 5,4 | 5,4 | 5,4 | 5,4 |
| Stärke | 3,0 | 3,0 | 3,0 | 3,0 | 3,0 | 3,0 |
| Magermilch-pulver | 1,5 | 1,5 | 1,5 | 1,5 | 1,5 | 1,5 |
| Aubygel MR50 | 0,5 | 0,5 | 0,5 | 0,5 | 0,5 | 0,5 |
| Verbindung **(17) + (18)** (Verhältnis 1:1) | - | - | 0,01 | 0,005 | 0,005 | 0,003 |
| Extrakt (z.B. von PlantExtrakt) aus *Rubus suavissimus,* enthaltend 5 Gew.-% Rubusosid, bezogen auf das Gesamtgewicht des Extraktes | - | - | - | - | 0,010 | 0,005 |
| Hesperetin | - | - | - | 0,001 | - | 0,001 |
| Phloretin | - | - | - | 0,002 | - | 0,001 |
| 3',7-Dihydroxy-4'-methoxyflavan gemäß EP 2 253 226 | - | - | - | - | - | 0,001 |
| Vanilleschoten-Extrakt, sprühgetrocknet (Symrise) | 0,1 | 0,1 | 0,1 | 0,1 | 0,1 | 0,1 |
| Milch 1,5 % Fettanteil | ad 100 | | | | | |

Die festen Stoffe wurden vorgelegt und mit der Milch aufgerührt. Die Mischung wurde auf 95 °C für 2 min unter gutem Rühren aufgewärmt, abgefüllt und auf 5 - 8 °C abgekühlt.

### Anwendungsbeispiel 16: Fettarme Joghurts

Zubereitung A: Vergleichszubereitung mit vollem Zuckergehalt
Zubereitung B-D: erfindungsgemäße Zubereitungen

| | **Zubereitung (Angaben in Gew.-%)** | | | |
|---|---|---|---|---|
| **Inhaltsstoff** | **A** | **B** | **C** | **D** |
| Sucrose | 10 | 8 | 6 | - |
| Fruchtzubereitung Erdbeere | 10 | 10 | 10 | 10 |
| Rebaudiosid A 98 % | - | - | - | 0,050 |
| Verbindung **(15) + (16)** (Verhältnis 1:1) | - | - | 0,005 | 0,0075 |
| Verbindung **(23) + (24)** (Verhältnis 1:1) | - | 0,005 | - | - |
| Extrakt aus *Rubus suavissimus* (z.B. von PlantExtrakt) enthaltend 5 Gew.-% Rubusosid, bezogen auf das Gesamtgewicht des Extraktes | - | - | 0,010 | - |
| Hesperetin | - | 0,001 | 0,001 | 0,001 |
| Phloretin | - | - | 0,002 | - |
| Hopmoeriodictyol-Natriumsalz | - | - | - | 0,005 |
| Natürliches Erdbeeraroma | 0,1 | 0,1 | 0,1 | 0,1 |
| Joghurt, 0,1 % Fett | ad 100 | | | |

Die Inhaltsstoffe wurden gemischt und bei 5°C gekühlt.

### Anwendungsbeispiel 17: Milchmischaetränke

Zubereitung A: Vergleichszubereitung mit vollem Zuckergehalt
Zubereitung B-D: erfindungsgemäße Zubereitungen

| | **Zubereitung (Angaben in Gew.-%)** | | | |
|---|---|---|---|---|
| **Inhaltsstoff** | **A** | **B** | **C** | **D** |
| **Sucrose** | 10 | 8 | 7 | - |
| Fructose | - | - | 0,5 | - |
| Rebaudiosid A 98 % | - | - | - | 0,040 |
| Verbindung **(15) + (16)** (Verhältnis 1:1) | - | 0,005 | 0,0025 | 0,0025 |
| Balansin A | - | 0,003 | 0,001 | 0,001 |
| Extrakt aus Rubus suavissimus (z.B. von PlantExtrakt) enthaltend 5 Gew.-% Rubusosid, bezogen auf das Gesamtgewicht des Extraktes, | - | - | 0,010 | - |
| Hesperetin | - | 0,003 | 0,002 | 0,005 |
| Phloretin | - | - | 0,002 | - |
| Homoeriodictyol-Natriumsalz | - | - | - | 0,002 |
| Vanille-Aroma | 0,1 | 0,1 | 0,1 | 0,1 |
| H-Milch, 1,5 % Fett | ad 100 | | | |

Die Inhaltsstoffe wurden gemischt, mit Milch aufgefüllt, gut gerührt, in Flaschen abgefüllt und bei 5°C gekühlt gelagert.

### Anwendungsbeispiel 18: Zuckerreduziertes Tomatenketchup

Vergleichszubereitung mit Zucker (A)
Vergleichszubereitung mit reduziertem Zuckeranteil (B)
Erfindungsgemäße Zubereitungen (C-I)

| | **Zubereitung (Angaben in Gew.-%)** | | | | | | | |
|---|---|---|---|---|---|---|---|---|
| **Inhaltsstoff** | **A** | **B** | **C** | **E** | **F** | **G** | **H** | **I** |
| Kochsalz | 2 | 2 | 2 | 2 | 2 | 2 | 2 | 2 |
| Stärke, Farinex WM 55 | 1 | 1 | 1 | 1 | 1 | 1 | 1 | 1 |
| Sucrose | 12 | 9,6 | 9,2 | 8,4 | 9,6 | 9,6 | 8,4 | 4,2 |
| Tomaten-Konzentrat 2-fach | 40 | 40 | 40 | 40 | 30 | 30 | 30 | 30 |
| Glucosesirup 80 Brix | 18 | 18 | 18 | 18 | 18 | 18 | 18 | 18 |
| Branntweinessig 10% | 7 | 7 | 7 | 7 | 3 | 3 | 3 | 3 |
| Rebaudiosid A 98 % | - | - | - | - | - | - | - | 0,05 |
| Verbindung **(15) + (16)** (Verhältnis 1:1) | - | - | 0,01 | 0,005 | 0,005 | 0,01 | 0,005 | 0,01 |
| Extrakt aus *Rubus suavissimus* (z.B. von PlantExtrakt) enthaltend 5 Gew.-% Rubusosid, bezogen auf das Gesamtgewicht des Extraktes | - | - | - | - | - | - | 0,01 | - |
| Hesperetin 2,5%ig in 1,2-Propylenglycol | - | - | - | - | 0,1 | - | 0,1 | - |
| Phloretin 2,5% in 1,2-Propylenglycol | - | - | - | 0,2 | 0,2 | - | - | 0,3 |
| Wasser | ad 100 | | | | | | | |

Die Inhaltsstoffe werden in der angegebenen Reihenfolge gemischt und das fertige Ketchup mit Hilfe eines Rührwerkes homogenisiert, in Flaschen abgefüllt und sterilisiert.

### Anwendungsbeispiel 19: Zuckerreduzierte Eiscremes

Vergleichszubereitung mit Zucker (A)
Vergleichszubereitung mit reduziertem Zuckeranteil (B)
Erfindungsgemäße Zubereitungen (C-F)

| | **Zubereitung (Gehalt in Gew.-%)** | | | | | |
|---|---|---|---|---|---|---|
| **Inhaltsstoff** | **A** | **B** | **C** | **D** | **E** | **F** |
| Pflanzenfett, Schmelzbereich 35 - 40 °C | 20,00 | 20,00 | 20,00 | 20,00 | 20,00 | 20,00 |
| Zucker (Saccharose) | 12,00 | 8,00 | 8,00 | 8,00 | 8,00 | 8,00 |
| Magermilchpulver | 5,00 | 5,00 | 5,00 | 5,00 | 5,00 | 5,00 |
| Glucosesirup 72 % Trockensubstanz | 5,00 | 5,00 | 5,00 | 5,00 | 5,00 | 5,00 |
| Emulgator SE 30 (Grindstedt Products, Dänemark) | 0,65 | 0,65 | 0,65 | 0,65 | 0,65 | 0,65 |
| Aroma, enthaltend 0,1 % Diacetyl und 1 % Vanillin | 0,20 | 0,20 | 0,20 | 0,20 | 0,20 | 0,20 |
| Verbindung **(23) + (24)** (Verhältnis 1:1) | - | - | 0,01 | 0,0075 | 0,01 | 0,003 |
| Extrakt aus *Rubus suavissimus* (z.B. von Plant-Extrakt) enthaltend 5 Gew.-% Rubusosid, bezogen auf das Gesamtgewicht des Extraktes, | - | - | - | - | - | 0,010 |
| Hesperetin 2,5%ig in 1,2-Propylenglycol | - | - | - | 0,10 | - | 0,10 |
| Phloretin 2,5% in 1,2-Propylenglycol | - | - | - | - | 0,05 | 0,05 |
| Magermilch | ad 100 | | | | | |

Das Pflanzenfett wurde auf 58°C erwärmt. Magermilch und Glucosesirup wurden auf 55°C erhitzt und Zucker, Magermilchpulver sowie Emulgator und Aroma hinzugefügt und die Mischung ins Pflanzenfett gegeben. Die Mischung wurde mit Hilfe eines Durchflusshochdruckhomogenisators homogenisieret (180 / 50 bar). Die erhaltene Masse wurde 1 min lang bei 78°C temperiert, anschließend auf 2 - 4 °C abgekühlt und 10 h zur Reifung bei dieser Temperatur inkubiert. Danach wurde die gereifte Masse abgefüllt und bei -18°C eingefroren gelagert.

### Anwendungsbeispiel 20: Für Diabetiker geeignetes Eis

Es wurde ein für Diabetiker geeignetes Eis aus folgenden Zutaten hergestellt und in Portionen zu je 95 mL in Becher abgefüllt:
Eingedickte entrahmte Milch, Fructosesirup, Erdbeerstücke und Erbeerpüree (15 Gew.-%), Pflanzenfett, Diätschokoladensplitter (3,5 Gew.-%, mit Emulgator Sojalecithin), Molkenerzeugnis, Rote Bete Saft, Johannisbrotkernmehl, Guarkernmehl, Carrageen, Emulgator (E 471), Gelatine, Säuerungsmittel Citronensäure, 0,1 Gew.-% Erdbeer-Aroma (enthaltend insgesamt 5 Gew.-% an Verbindungen **(23)** + **(24)** (Verhältnis 1:1), bezogen auf das Gesamtgewicht des Erdbeer-Aromas), Farbstoff Carotin.

Nährwert (pro 95 mL):
Eiweiss 1,8 g, Kohlenhydrate 13,3 g (davon Fructose 9,5 g), Fett 4,2 g.

### Anwendungsbeispiel 21: Diätschokolade auf Basis von Maltit

Es wurde eine für Diabetiker geeignete Schokolade aus folgenden Zutaten hergestellt und in rechteckige Tafeln gegossen:
Maltit, Haselnussmasse, Kakaobutter, Magermilchpulver, Kakaomasse, Inulin, Butterreinfett, Emulgator Sojalecithine, 0,1 Gew.-% Vanille-Aroma (enthaltend Vanilleschoten-Extrakt, Vanillin und insgesamt 3 Gew.-% an Verbindungen **(15)** + **(16)** (Verhältnis 1:1) sowie insgesamt 3 Gew.-% an Verbindungen **(17)** + **(18)** (Verhältnis 1:1), bezogen auf das Gesamtgewicht des Vanille-Aromas).
Nährwert (pro 100 g):
   Eiweiss 8 g, Kohlenhydrate 43 g (davon Maltit 34 g), Fett 34 g.

### Anwendungsbeispiel 22: Diätschokolade auf Basis von Fructose

Es wurde eine für Diabetiker geeignete Schokolade aus folgenden Zutaten hergestellt und in rechteckige Tafeln gegossen:
Kakaomasse, Fructose, Magermilchpulver, Kakaobutter, Inulin, Butterreinfett, Emulgator Sojalecithin, Walnüsse, Speisesalz, 0,1 Gew.-% Vanille-Aroma (enthaltend Vanillin und insgesamt 3 Gew.-% Verbindung **(23)** + **(24)** (Verhältnis 1:1), bezogen auf das Gesamtgewicht des Vanille-Aromas).
Nährwert (pro 100 g):
   Eiweiss 8,8 g, Kohlenhydrate 34 g (davon Fructose 23 g, Lactose 7,5 g, Saccharose 1,4 g), Fett 36 g; Ballaststoffe 18,5 (davon 12,2 g Inulin); Natrium: 0,10 g. Kakao-Anteil mindestens 50 Gew.-%.

### Anwendungsbeispiel 23: Zuckerreduzierte Müslimischung

### Vergleichszubereitung mit Zucker (A)

Erfindungsgemäße Zubereitung mit reduziertem Zuckeranteil (B)

| Nr. | | A (Gew.-%) | B (Gew.-%) |
|---|---|---|---|
| 1 | Haferflocken | 17,00 | 19,00 |
| 2 | Knusprige Haferflocken Cluster | 10,00 | 12,00 |
| 3 | Reis Crispies | 16,90 | 17,80 |
| 4 | Cornflakes | 16,50 | 17, 50 |
| 5 | Korinthen | 3,50 | 3,50 |
| 6 | Haselnüsse, zerhackt | 2,50 | 2,50 |
| 7 | Glucose Sirup aus Weizen, DE 30 | 9,50 | 9,50 |
| 8 | Saccharose | 20,00 | 14,00 |
| 9 | Wasser | 4,00 | 4,03 |
| 10 | Citronensäurepulver, wasserfrei | 0,10 | 0,10 |
| 11 | Aromakomposition D aus Anwendungsbeispiel 2 | - | 0,07 |

Jeweils Bestandteile Nr. 1 bis 6 in einer Drehtrommel mischen (Mix 1). Jeweils Bestandteile Nr. 7 bis 9 erwärmen und Bestandteil Nr. 10 zugeben (sowie in Rezeptur B zusätzlich den Bestandteil Nr. 11 zugeben) (Mix 2). Jeweils Mix 2 zu Mix 1 geben und gut mischen. Zuletzt die resultierende Müslimischung auf ein Backblech geben und in einem Ofen bei 130°C für 8 Minuten trocknen.

### Anwendungsbeispiel 24: Zuckerreduzierte Fruchtgummis

### Vergleichszubereitung mit Zucker (A)

Erfindungsgemäße Zubereitung mit reduziertem Zuckeranteil (B)

| | A (Gew.-%) | B (Gew.-%) |
|---|---|---|
| Wasser | 23,70 | 25,70 |
| Saccharose | 34,50 | 8,20 |
| Glucosesirup, DE 40 | 31,89 | 29,89 |
| Iso Syrup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor GmbH) | - | 24,40 |
| Gelber und roter Farbstoff | 0,01 | 0,01 |
| Citronensäure | 0,20 | 0,2 |
| Kirscharoma, enthaltend insgesamt 4 Gew.-% an Verbindungen **(23) + (24)** (Verhältnis 1:1), und 0,3 Gew.-% Phloretin, bezogen auf das Kirscharoma | - | 0,10 |

Polydextrose ist ein selbst nicht süß schmeckendes Polysaccharid mit niedrigem Brennwert.

### Anwendungsbeispiel 25: Schoko-Cappuccino-Eiscreme

### Vergleichszubereitung mit Zucker (A)

Erfindungsgemäße Zubereitung mit reduziertem Zuckeranteil (B)

| | A (Gew.-%) | B (Gew.-%) |
|---|---|---|
| Glucose-Fructose-Sirup | 14,30 | 14,30 |
| Saccharose | 10,00 | 7,50 |
| Magermilchpulver | 5,00 | 5,00 |
| Sahne (36% Fettanteil) | 24,00 | 24,00 |
| Emulgator und Stabilisator Cremodan^{®} 709VEG (Danisco) | 0,50 | 0,50 |
| Kakaopulver | 5,975 | 5,975 |
| Carrageenan | 0,025 | 0,025 |
| Wasser | 40,20 | 42,50 |
| Cappuccino-Aroma, enthaltend insgesamt 2 Gew.-% an Verbindungen **(15) + (16)** (Verhältnis 1:1) und 1 Gew.-%.Homoeriodictyol-Natriumsalz, bezogen auf das gesamte Aroma | - | 0,20 |

## Patentansprüche

1. Verwendung eines, zweier oder mehrerer verschiedener Verbindungen der Formel **(I)** 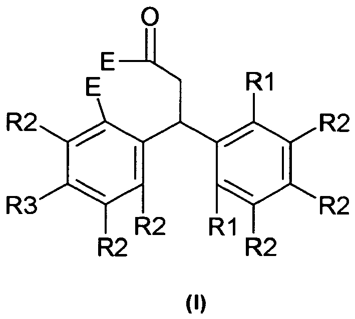 oder
eines, zweier oder mehrerer verschiedener physiologisch akzeptabler Salze eines, zweier oder mehrerer verschiedener Verbindungen der Formel **(I),**
oder
eines Gemisches eines, zweier oder mehrerer verschiedener Verbindungen der Formel **(I)** mit einem, zwei oder mehreren verschiedenen physiologisch akzeptablen Salzen eines, zweier oder mehrerer verschiedener Verbindungen der Formel **(I)**,
wobei
E entweder jeweils OH oder beide E zusammen ein O bedeuten,
R1, unabhängig von dem jeweils anderen Rest R1, Wasserstoff oder OR^{a} bedeutet, wobei R^{a} Wasserstoff, C1-C5-Alkyl oder C2-C5-Alkenyl ist,
R2, unabhängig von den anderen Resten R2, Wasserstoff oder OR^{b} bedeutet, wobei R^{b} Wasserstoff, C1-C5-Alkyl oder C2-C5-Alkenyl ist,
wobei gegebenenfalls zwei unmittelbar benachbarte Reste R1 und/oder R2 zusammen eine Gruppe OCH₂O repräsentieren,
und
R3 einen Rest OR^{x} bedeutet, wobei R^{x} C1-C5-Alkyl oder C2-C5-Alkenyl ist,
zur Erzeugung eines Süßeindruckes in einer oral konsumierbaren Zubereitung oder zum Beeinflussen der Stärke des Süßeindruckes süßer Stoffe oder Stoffgemische oder sowohl süß als auch unangenehm, vorzugsweise bitter, schmeckender Stoffe oder Stoffgemische.

2. Verwendung nach Anspruch 1, wobei ein, zwei, mehrere oder sämtliche der eingesetzten Verbindungen jeweils ausgewählt sind aus der Gruppe bestehend aus den Verbindungen der Formel **(I)** und deren physiologisch akzeptablen Salzen, wobei
E jeweils OH oder beide E zusammen Sauerstoff bedeuten,
R1, unabhängig von dem jeweils anderen Rest R1, Wasserstoff oder Hydroxy bedeutet,
R2, unabhängig von den anderen Resten R2, Wasserstoff, Hydroxy, Methoxy, Ethoxy, n-Propoxy, iso-Propoxy, n-Butoxy, iso-Butoxy, tert-Butoxy oder OR^{b} bedeutet, wobei R^{b} C5-Alkenyl ist,
wobei gegebenenfalls zwei unmittelbar benachbarte Reste R2 zusammen eine Gruppe OCH₂O repräsentieren,
R3 Methoxy, Ethoxy, n-Propoxy, iso-Propoxy, n-Butoxy, iso-Butoxy, tert-Butoxy oder OR^{c} bedeutet, wobei R^{c} C5-Alkenyl ist,
wobei vorzugsweise ein oder mehrere der Reste R1 oder R2 eine Hydroxygruppe bedeuten.

3. Verwendung wie in einem der vorangehenden Ansprüche definiert, wobei ein, zwei, mehrere oder sämtliche der eingesetzten Verbindungen der Formel **(I)** jeweils ausgewählt sind aus der Gruppe bestehend der Verbindungen der Formel **(II)** 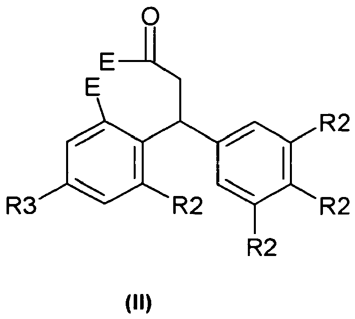 wobei
E jeweils OH oder beide E zusammen Sauerstoff bedeuten,
R2, unabhängig von den anderen Resten R2, Wasserstoff, Hydroxy, Methoxy, Ethoxy, n-Propoxy oder iso-Propoxy bedeutet, vorzugsweise H, OH, OCH₃ oder OCH₂CH₃,
wobei gegebenenfalls zwei unmittelbar benachbarte Reste R2 zusammen eine Gruppe OCH₂O repräsentieren,
R3 Methoxy, Ethoxy, n-Propoxy, iso-Propoxy, n-Butoxy, iso-Butoxy, tert-Butoxy oder OR^{c} bedeutet, wobei R^{c} C5-Alkenyl ist,
und deren physiologisch akzeptablen Salzen.

4. Verwendung wie in einem der vorangehenden Ansprüche definiert, wobei ein, zwei, mehrere oder sämtliche der eingesetzten Verbindungen der Formel (**I**) jeweils ausgewählt sind aus der Gruppe bestehend aus 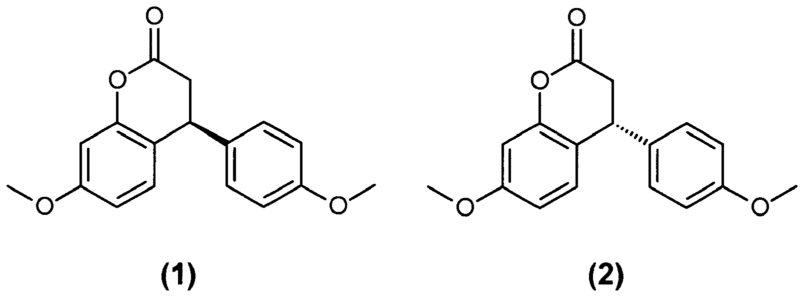 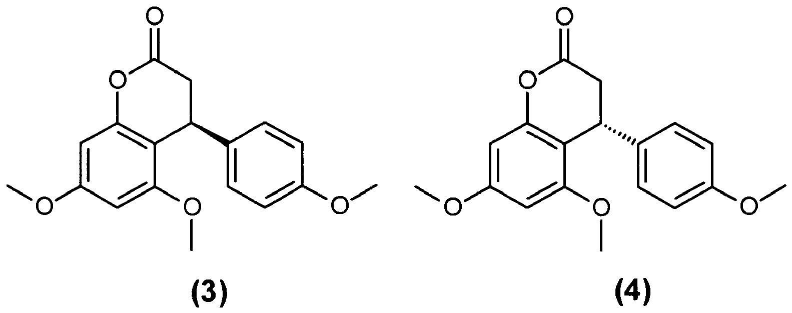 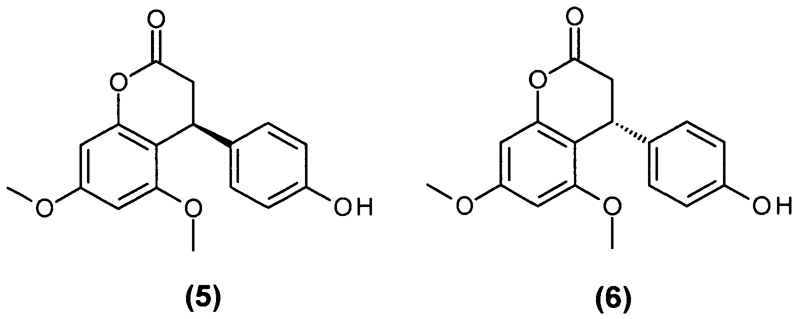 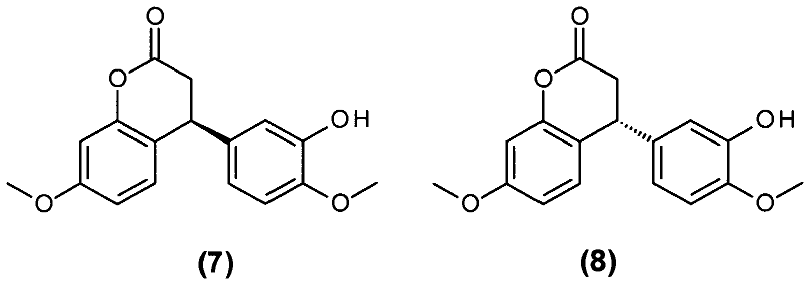 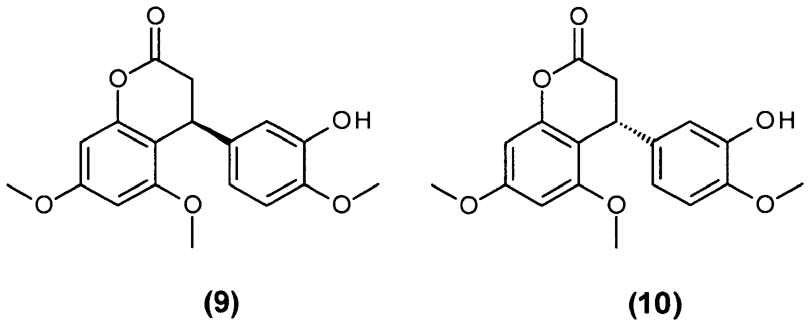 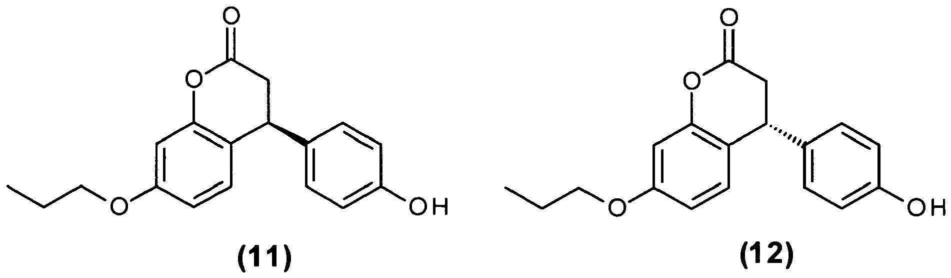 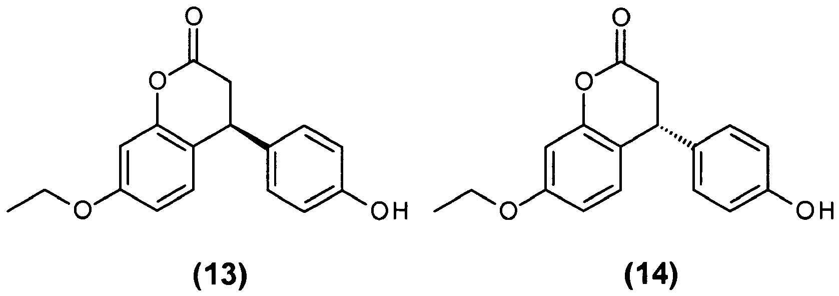 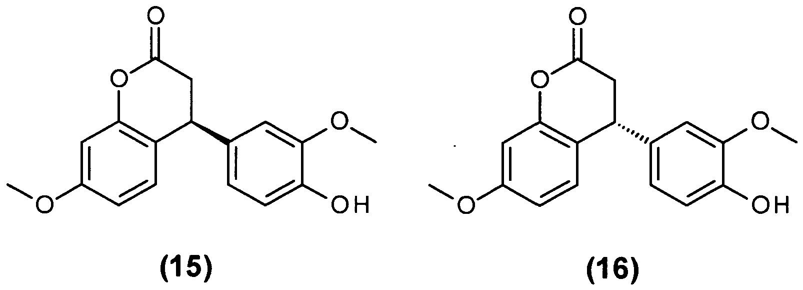 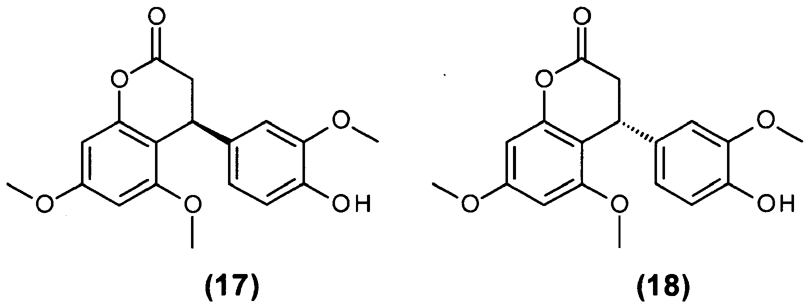 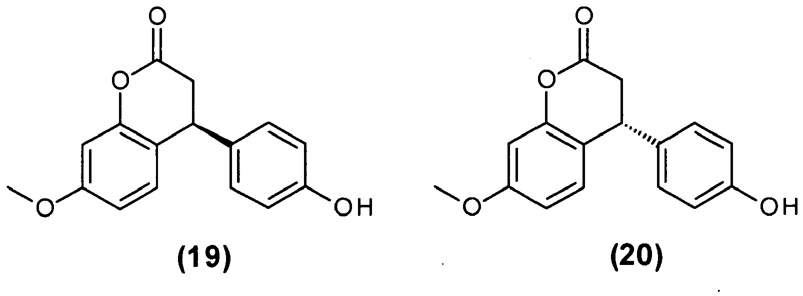 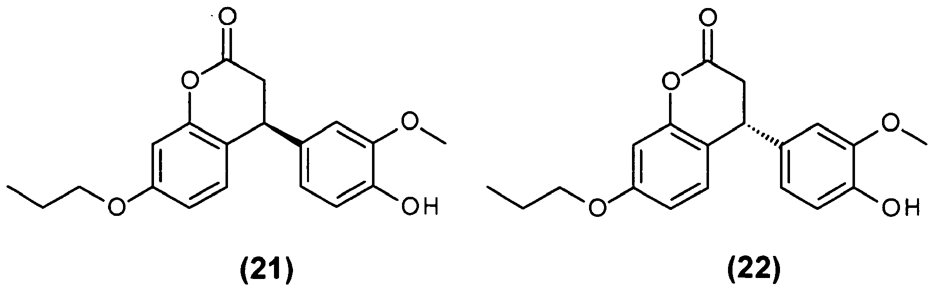 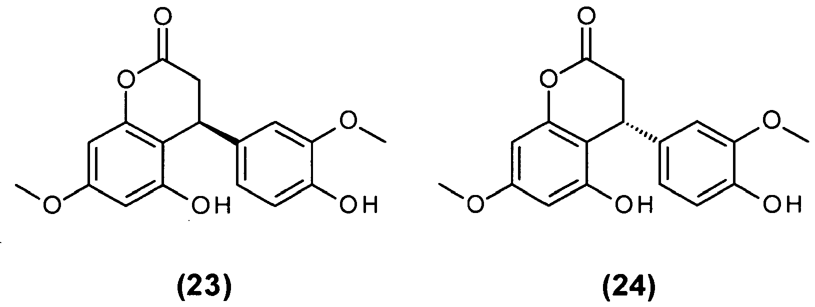 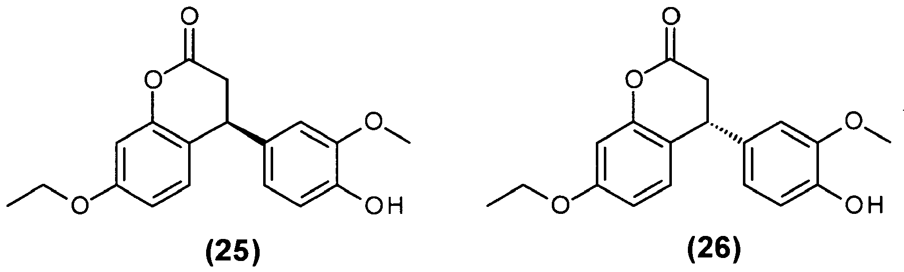 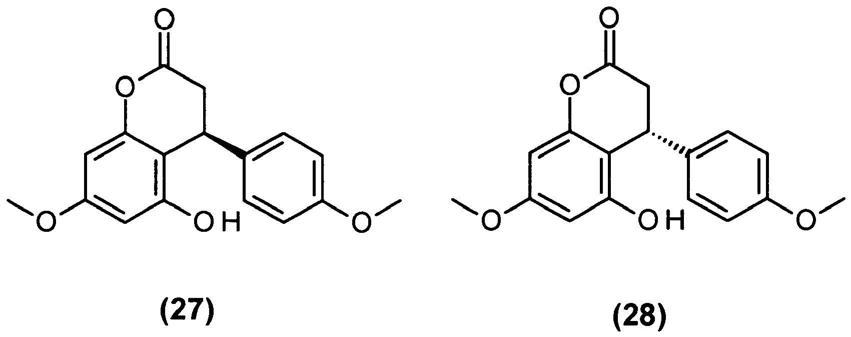 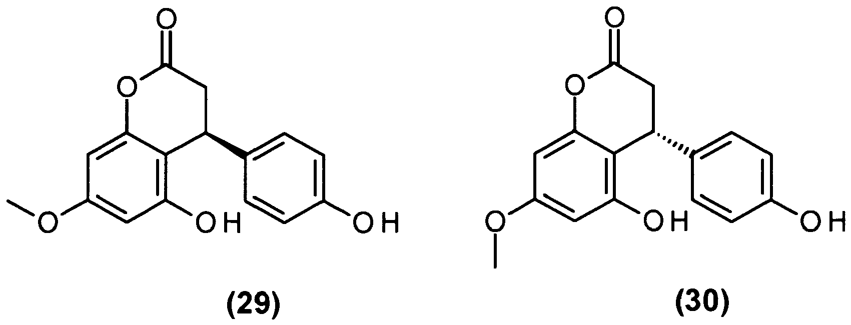 und deren physiologisch akzeptablen Salzen.

5. Verwendung eines, zweier oder mehrerer verschiedener Salze eines, zweier oder mehrerer verschiedener Verbindungen der Formel (**I**) wie in einen der vorhergehenden Ansprüche definiert
oder
eines Gemisches eines, zweier oder mehrerer verschiedener Verbindungen der Formel (**I**) wie in einen der vorhergehenden Ansprüche definiert mit einem, zwei oder mehreren verschiedenen physiologisch akzeptablen Salzen eines, zweier oder mehrerer verschiedener Verbindungen der Formel (**I**) wie in einen der vorhergehenden Ansprüche definiert,
wobei das bzw. die Gegenkationen des bzw. eines, mehrerer oder sämtlicher der physiologisch akzeptablen Salze vorzugsweise ausgewählt ist bzw. sind aus der Gruppe bestehend aus Na⁺, K⁺, NH₄⁺, Ca²⁺, Mg²⁺, Al³⁺ und Zn²⁺.

6. Halbfertigware umfassend oder bestehend aus den folgenden Komponenten:
(a) eine oder mehrere Verbindungen der Formel (**I**) und/oder deren Salze wie in den Ansprüchen 1 bis 5 definiert,
sowie
einen, zwei, drei oder mehrere Stoffe ausgewählt aus der Gruppe bestehend aus den Komponenten (b) und (c)
(b) einen oder mehrere süß schmeckende und/oder süß riechende Stoffe, und/oder
(c) einen oder mehrere sowohl süß als auch unangenehm, insbesondere bitter, schmeckende Stoffe,
sowie
(d) gegebenenfalls einen oder mehrere zum Verzehr geeignete Trägerstoffe,
(e) gegebenenfalls eine oder mehrere weitere Substanzen zum Maskieren oder Vermindern eines unangenehmen, insbesondere eines bitteren Geschmackeindruckes,
und
(f) gegebenenfalls eine oder mehrere weitere Substanzen zum Verstärken eines süßen Geschmackeindruckes,
wobei die Komponenten (b)-(f) weder eine Verbindung der Formel (**I**) noch ein Salz einer Verbindung der Formel (**I**) sind bzw. enthalten.

7. Halbfertigware nach Anspruch 6, **dadurch gekennzeichnet, dass** der oder die süß schmeckenden Stoffe der Komponente (b) ausgewählt sind aus der Gruppe bestehend aus
(b1) süßen Aromastoffen, wobei vorzugsweise ein, zwei, drei, vier, fünf oder mehrere der süßen Aromastoffe ausgewählt sind aus der Gruppe bestehend aus Vanillin, Ethylvanillin, 2-Hydrox-4-methoxybenzaldehyd, Ethylvanillinisobutyrat (= 3-Ethoxy-4-isobutyryloxybenzaldehyd), Furaneol^{®} (2,5-Dimethyl-4-hydroxy-3(2H)-furanon) und dessen Abkömmlingen (vorzugsweise Homofuraneol, 2-Ethyl-4-hydroxy-5-methyl-3(2H)-furanon), Homofuronol (2-Ethyl-5-methyl-4-hydroxy-3(2H)-furanon und 5-Ethyl-2-methyl-4-hydroxy-3(2H)-furanon), Maltol und dessen Abkömmlingen (vorzugsweise Ethylmaltol), Cumarin, gamma-Lactonen (vorzugsweise gamma-Undecalacton, gamma-Nonalacton), delta-Lactonen (vorzugsweise 4-Methyldeltalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Fruchtestern und Fruchtlactonen (vorzugsweise Essigsäure-n-butylester, Essigsäureisoamylester, Propionsäureethylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat), 4-(p-Hydroxyphenyl)-2-butanon, 1,1-Dimethoxy-2,2,5-trimethyl-4-hexan, 2,6-Dimethyl-5-hepten-1-al und Phenylacetaldehyd;
(b2) Kohlenhydraten, vorzugsweise ausgewählt aus der Gruppe bestehend aus Saccharose, Trehalose, Lactose, Maltose, Melizitose, Melibiose, Raffinose, Palatinose, Lactulose, D-Fructose, D-Glucose, D-Galactose, L-Rhamnose, D-Sorbose, D-Mannose, D-Tagatose, D-Arabinose, L-Arabinose, D-Ribose, D-Glyceraldehyd, Maltodextrine und pflanzliche Zubereitungen enthaltend einen oder mehrere der genannten Kohlenhydrate, vorzugsweise in einem Anteil von zumindest 5 Gew. %, bevorzugt zumindest 15 Gew.-%, wobei die Kohlenhydrate auch als natürlich vorkommende oder künstlich hergestellte Mischung vorliegen können, dabei insbesondere als Honig, Invertzuckersirup oder hochangereicherter Fructose-Sirup aus Maisstärke, und den physiologisch akzeptablen Salzen dieser Kohlenhydrate, insbesondere den Natrium-, Kalium-, Calcium oder Ammoniumsalzen;
(b3) Zuckeralkoholen, vorzugsweise natürlich vorkommende Zuckeralkohole ausgewählt aus der Gruppe bestehend aus Glycerin, Erythritol, Threitol, Arabitol, Ribitol, Xylitol, Sorbitol, Mannitol, Maltitol, Isomaltit, Dulcitol, Lactitol, und den physiologisch akzeptablen Salzen dieser Zuckeralkohole, insbesondere den Natrium-, Kalium-, Calcium oder Ammoniumsalzen;
(b4) natürlich vorkommenden Süßstoffen, vorzugsweise ausgewählt aus der Gruppe bestehend aus
(b4-1) Miraculin, Monellin, Mabinlin, Thaumatin, Curculin, Brazzein, Pentaidin, D-Phenylalanin, D-Tryptophan, und aus natürlichen Quellen gewonnene Extrakte oder Fraktionen, enthaltend diese Aminosäuren und/oder Proteine, und den physiologisch akzeptablen Salzen dieser Aminosäuren und/oder Proteine, insbesondere den Natrium-, Kalium-, Calcium oder Ammoniumsalzen;
(b4-2) Neohesperidindihydrochalkon, Naringindihydrochalkon, Steviosid, Steviolbiosid, Rebaudiosiden, insbesondere Rebaudiosid A, Rebaudiosid B, Rebaudiosid C, Rebaudiosid D, Rebaudiosid E, Rebaudiosid F, Rebaudiosid G, Rebaudiosid H, Dulcosiden und Rubusosid, Suavioside A, Suavioside B, Suavioside G, Suavioside H, Suavioside I, Suavioside J, Baiyunoside 1 Baiyunoside 2, Phlomisoside 1, Phlomisoside 2, Phlomisoside 3, sowie Phlomisoside 4, Abrusoside A, Abrusoside B, Abrusoside C, Abrusoside D, Cyclocaryoside A und Cyclocaryoside I, Oslandin, Polypodosid A, Strogin 1, Strogin 2, Strogin 4, Selligueanin A, Dihydroquercetin-3-acetat, Perillartin, Telosmosid A₁₅, Periandrin I-V, Pterocaryosiden, Cyclocaryosiden, Mukuroziosiden, trans-Anethol, trans-Cinnamaldehyd, Bryosiden, Bryonosiden, Bryonodulcosiden, Carnosiflosiden, Scandenosiden, Gypenosiden, Trilobatin, Phloridzin, Dihydroflavanolen, Hematoxylin, Cyanin, Chlorogensäure, Albiziasaponin, Telosmosiden, Gaudichaudiosid, Mogrosiden, Mogrosid V, Hernandulcinen, Monatin, Phyllodulcin, Glycyrrhetinsäure und deren Derivaten, insbesondere deren Glycosiden wie Glycyrrhizin, und den physiologisch akzeptablen Salzen dieser Verbindungen, insbesondere den Natrium-, Kalium-, Calcium oder Ammoniumsalzen; (b4-3) Extrakten oder angereicherten Fraktionen der Extrakte, ausgewählt aus der Gruppe bestehend aus Thaumatococcus-Extrakten (Katemfestaude), Extrakten aus *Stevia* ssp. (insbesondere *Stevia rebaudiana*), Swingle-Extrakten (*Momordica* bzw. *Siratia grosvenorii,* Luo-Han-Guo), Extrakten aus *Glycerrhyzia* ssp. (insbesondere *Glycerrhyzia glabra*), Extrakten aus *Rubus* ssp. (insbesondere *Rubus suavissimus*), Citrus-Extrakten und Extrakten aus *Lippia dulcis*; Extrakte aus *Mycetia balansae*;
(b5) synthetischen süß schmeckenden Stoffen, vorzugsweise ausgewählt aus der Gruppe bestehend aus Magap, Natriumcyclamat oder anderen physiologisch akzeptablen Salzen der Cyclamsäure, Acesulfam K oder anderen physiologisch akzeptablen Salzen, Neohesperidindihydrochalkon, Naringindihydrochalkon, Saccharin, Saccharin-Natriumsalz, Aspartam, Superaspartam, Neotam, Alitam, Advantam, Perillartin, Sucralose, Lugduname, Carrelame, Sucrononate und Sucrooctate,
und/oder
der oder die sowohl süß als auch unangenehm schmeckenden Stoffe der Komponente (c) ausgewählt sind aus der Gruppe bestehend aus aus Steviolglycosiden (insbesondere Steviosid und Rebaudiosid A), Rubusosid, Dulcoside, Mogroside, Phyllodulcin, Glycyrrhetinsäure, Extrakte aus Stevia ssp. (insbesondere Stevia rebaudiana), Luo Han Guo, Rubus suavissimus, Hydrangea dulcis, Glycyrrhyza glabra, Magap, Natriumcyclamat, Acesulfam K, Neohesperidindihydrochalkon, Naringindihydrochalkon, Saccharin, Saccharin-Natriumsalz, Aspartam, Superaspartam, Neotam, Alitam, Sucralose, Lugduname, Carrelame, Sucrononate und Sucrooctate.

8. Halbfertigware nach Anspruch 6 oder 7, wobei die Halbfertigware eine Gesamtmenge an Verbindungen der Formel (**I**) und deren physiologisch akzeptablen Salzen wie in den Ansprüchen 1 bis 5 definiert (Komponente (a)) im Bereich von 0,0001 bis 90 Gew.-% umfasst, bevorzugt im Bereich von 0,001 bis 50 Gew.-%, weiter bevorzugt im Bereich von 0,01 bis 40 Gew.-%, besonders bevorzugt im Bereich von 0,1 bis 20 Gew.-%, am meisten bevorzugt im Bereich von 0,2 bis 10 Gew.-%, bezogen auf das Gesamtgewicht der Halbfertigware.

9. Zubereitung, vorzugsweise oral konsumierbare Zubereitung, ausgewählt aus der Gruppe bestehend aus
(1) der Ernährung, der Nahrungsergänzung, der Mundpflege oder dem Genuss dienenden Zubereitungen,
(2) kosmetischen Zubereitungen, insbesondere zur Applikation im Bereich des Kopfes,
(3) zur oralen Aufnahme bestimmten pharmazeutischen Zubereitungen, umfassend
(i) eine oder mehrere Verbindungen der Formel (**I**) und/oder deren Salze wie in den Ansprüchen 1 bis 5 definiert,
und
(ii) einen oder mehrere weitere süß schmeckende Stoffe, einen oder mehrere süß riechende Stoffe und/oder einen oder mehrere sowohl süß als auch unangenehm, insbesondere bitter, schmeckende Stoffe,
wobei die Menge an Bestandteil (i) in der Zubereitung ausreicht, den Süßeindruck des bzw. der süß schmeckenden und/oder süß riechenden Stoffe des Bestandteils (ii) sensorisch zu verstärken.

10. Zubereitung, vorzugsweise oral konsumierbare Zubereitung, nach Anspruch 9, umfassend eine Halbfertigware wie in den Ansprüchen 6 bis 8 definiert.

11. Oral konsumierbare Zubereitung nach Anspruch 9 oder 10, ausgewählt aus der Gruppe bestehend aus
(1) der Ernährung, der Nahrungsergänzung, der Mundpflege oder dem Genuss dienenden Zubereitungen,
(2) kosmetischen Zubereitungen zur Applikation im Bereich des Kopfes,
(3) zur oralen Aufnahme bestimmten pharmazeutischen Zubereitungen,
wobei die Zubereitung (1), (2) oder (3) bezogen auf ihr Gesamtgewicht 0,1 mg/kg bis 1 Gew.-%, vorzugsweise im Bereich von 0,5 bis 1000 mg/kg, bevorzugt im Bereich von 1 bis 500 mg/kg, weiter bevorzugt im Bereich von 3 bis 300 mg/kg, besonders bevorzugt im Bereich von 5 bis 200 mg/kg, am meisten bevorzugt im Bereich von 10 bis 100 mg/kg, an Verbindungen der Formel (**I**) und/oder deren Salzen wie in den Ansprüchen 1 bis 5 definiert umfasst.

12. Zubereitung, vorzugsweise oral konsumierbare Zubereitung, nach einem der Ansprüche 9 bis 11, **dadurch gekennzeichnet, dass** die Gesamtmenge an Verbindungen der Formel (**I**) und/oder deren Salzen wie in den Ansprüchen 1 bis 5 definiert in der Zubereitung ausreicht, um im Vergleich mit einer Zubereitung, die bei ansonsten identischer Zusammensetzung weder eine Verbindung der Formel (**I**) noch ein Salz einer Verbindung der Formel (**I**) wie in den Ansprüchen 1 bis 5 definiert umfasst, eine Steigerung des in Sucroseäquivalenten zu bestimmenden Süßeindruckes um 10 % oder mehr, vorzugsweise um 20 % oder mehr, bevorzugt um 30% oder mehr und besonders bevorzugt 35% oder mehr zu vermitteln.

13. Verfahren zur Herstellung
- einer Halbfertigware wie in den Ansprüchen 6 bis 8 definiert
bzw.
- einer Zubereitung wie in den Ansprüchen 9 bis 12 definiert,
umfassend die Schritte
(1) Her- oder Bereitstellen der folgenden Komponenten:
(a) eine oder mehrere Verbindungen der Formel (**I**) und/oder deren Salze wie in den Ansprüchen 1 bis 5 definiert,
sowie
ein, zwei, drei oder mehrere Stoffe ausgewählt aus der Gruppe bestehend aus den Komponenten (b) und (c)
(b) einen oder mehrere süß schmeckende und/oder süß riechende Stoffe, und/oder
(c) einen oder mehrere sowohl süß als auch unangenehm, insbesondere bitter, schmeckende Stoffe,
sowie
(d) gegebenenfalls einen oder mehrere zum Verzehr geeignete Trägerstoffe,
(e) gegebenenfalls eine oder mehrere weitere Substanzen zum Maskieren oder Vermindern eines unangenehmen, insbesondere eines bitteren Geschmackeindruckes,
und
(f) gegebenenfalls eine oder mehrere weitere Substanzen zum Verstärken eines süßen Geschmackeindruckes,
wobei die Komponenten (b)-(f) weder eine Verbindung der Formel (**I**) noch ein Salz einer Verbindung der Formel (**I**) sind bzw. enthalten,
und
(2) Mischen der Komponente (a) mit einer oder mehreren der Komponenten (b) und/oder (c) sowie gegebenenfalls (d) sowie gegebenenfalls Komponente (e) und gegebenenfalls Komponente (f).

14. Verfahren zum Beeinflussen der Stärke eines sensorischen Eindruckes, insbesondere des Süßeindruckes, eines süß schmeckenden und/oder süß riechenden Stoffes oder Stoffgemisches oder eines sowohl süß als auch unangenehm, insbesondere bitter, schmeckenden Stoffes oder Stoffgemisches, mit folgendem Schritt:
Mischen von Komponente (a)
(a) eine oder mehrere Verbindungen der Formel (**I**) und/oder deren Salze wie in den Ansprüchen 1 bis 5 definiert
mit Komponente (b) und/oder (c)
(b) einen oder mehrere süß schmeckende und/oder süß riechende Stoffe, und/oder
(c) einen oder mehrere sowohl süß als auch unangenehm, insbesondere bitter, schmeckende Stoffe,
wobei die Komponenten (b) und (c) weder eine Verbindung der Formel (**I**) noch ein Salz einer Verbindung der Formel (**I**) sind bzw. enthalten,
wobei die Gesamtmenge an Komponente (a) in der Mischung ausreicht, um die Stärke des sensorischen Eindruckes, insbesondere des Süßeindruckes, des oder der süß schmeckenden und/oder süß riechenden Stoffe der Komponente (b) bzw. des oder der sowohl süß als auch unangenehm, insbesondere bitter, schmeckenden Stoffe der Komponente (c) zu beeinflussen.

## Claims

1. Use of one, two or more different compounds of formula **(I)** 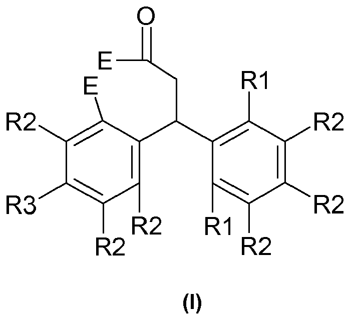 or
of one, two or more different physiologically acceptable salts of one, two or more different compounds of formula **(I),**
or
of a mixture of one, two or more different compounds of formula **(I)** with one, two or more different physiologically acceptable salts of one, two or more different compounds of formula **(I),**
wherein
E either denotes OH in each case or both E together denote an O,
R1, independent of the respective other residue R1 denotes hydrogen or OR^{a}, wherein R^{a} is hydrogen, C1-C5-alkyl or C2-C5-alkenyl,
R2, independent of the other residues R2 denotes hydrogen or OR^{b}, wherein R^{b} is hydrogen, C1-C5-alkyl or C2-C5-alkenyl,
wherein optionally two directly adjacent residues R1 and/or R2 together represent a group OCH₂O,
and
R3 denotes a residue OR^{x}, wherein R^{x} is C1-C5-alkyl or C2-C5-alkenyl,
for producing a sweet impression in an orally consumable preparation or for influencing the strength of the sweet impression of sweet substances or substance mixtures or of both, sweet and unpleasant, preferably bitter, tasting substances or substance mixtures.

2. Use according to claim 1, wherein one, two, several or all of the compounds used are in each case selected from the group consisting of the compounds of formula **(I)** and the physiologically acceptable salts thereof, wherein
E in each case denotes OH or both E together denote oxygen,
R1, independent of the respective other residue R1 denote hydrogen or hydroxy,
R2, independent of the other residues R2 denotes hydrogen, hydroxy, methoxy, ethoxy, n-propoxy, iso-propoxy, n-butoxy, iso-butoxy, tert-butoxy or OR^{b}, wherein R^{b} is C5-alkenyl,
wherein optionally two directly adjacent residues R2 together represent a group OCH₂O,
R3 denotes methoxy, ethoxy, n-propoxy, iso-propoxy, n-butoxy, iso-butoxy, tert-butoxy or OR^{c}, wherein R^{c} is C5-alkenyl,
wherein preferably one or more of the residues R1 or R2 denote a hydroxy group.

3. Use as defined in one of the preceding claims, wherein one, two, several or all of the compounds of formula **(I)** used are in each case selected from the group consisting of the compounds of formula **(II)** 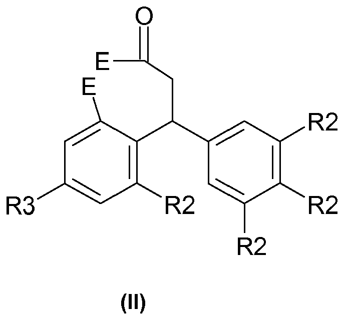 wherein
E in each case denotes OH or both E together denote oxygen,
R2, independent of the other residues R2 denotes hydrogen, hydroxy, methoxy, ethoxy, n-propoxy or iso-propoxy, preferably H, OH, OCH₃ or OCH₂CH₃,
wherein optionally two directly adjacent residues R2 together represent a group OCH₂O,
R3 denotes methoxy, ethoxy, n-propoxy, iso-propoxy, n-butoxy, iso-butoxy, tert-butoxy or OR^{c}, wherein R^{c} is C5-alkenyl,
and the physiologically acceptable salts thereof.

4. Use as defined in one of the preceding claims, wherein one, two, several or all of the compounds of formula **(I)** used are in each case selected from the group consisting of 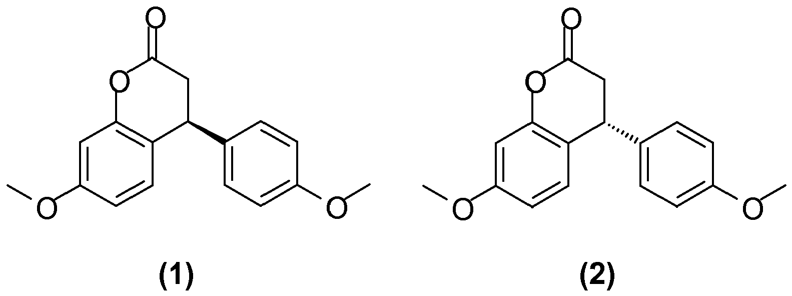 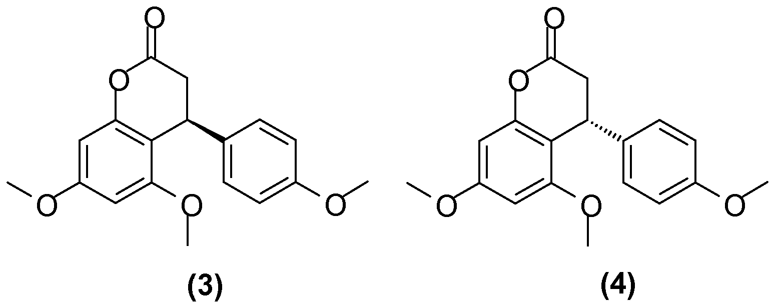 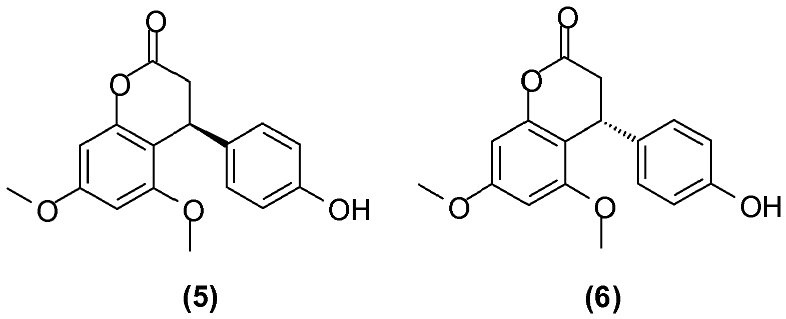 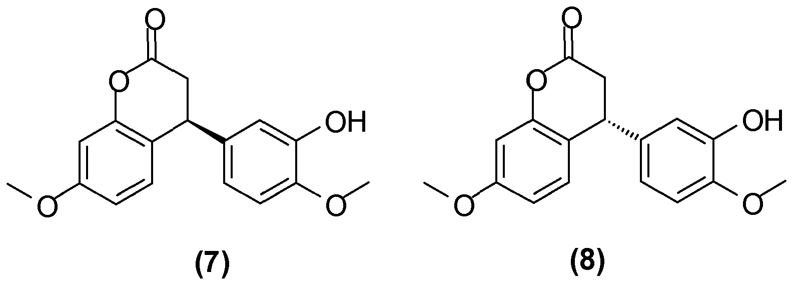 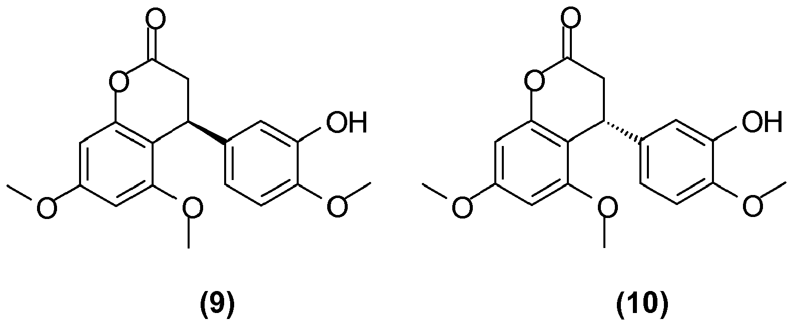 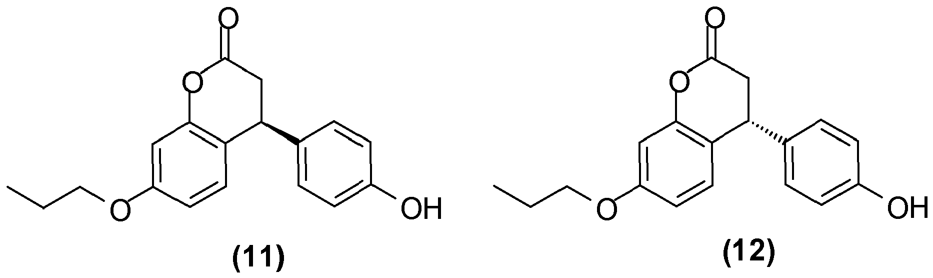 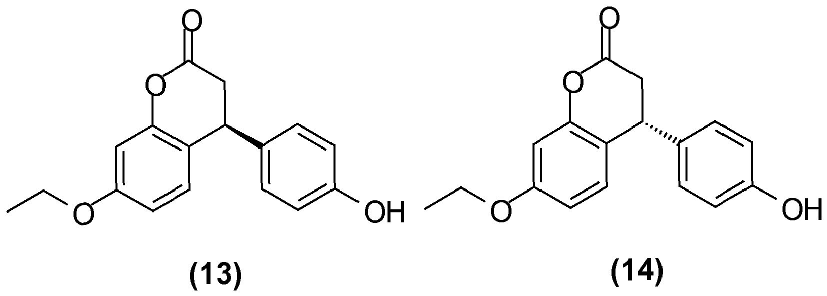 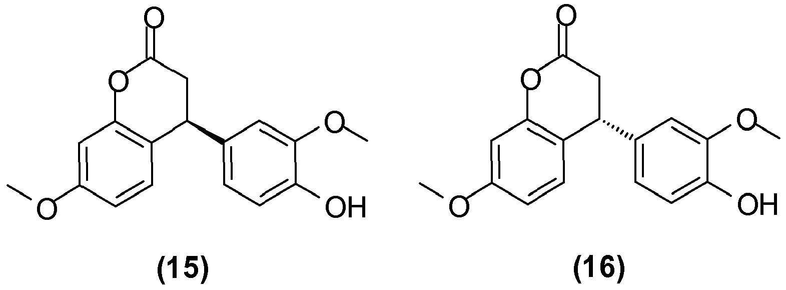 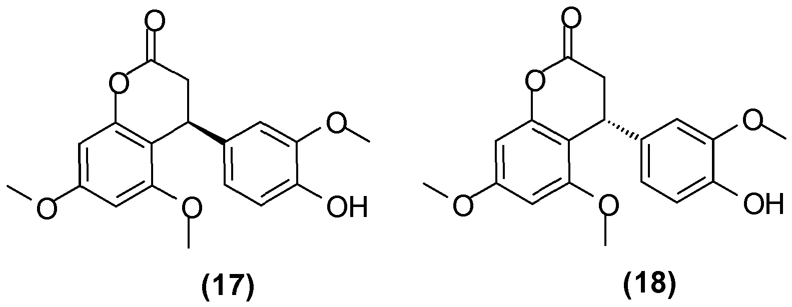 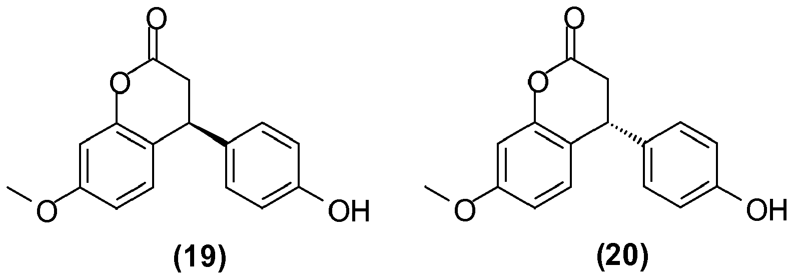 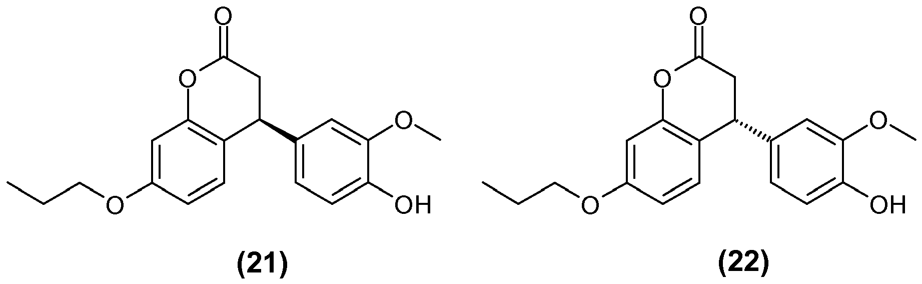 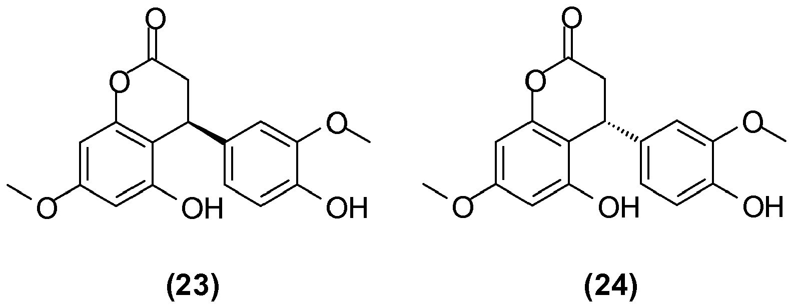 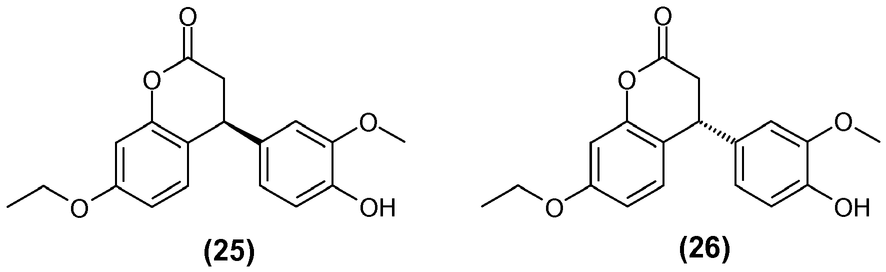 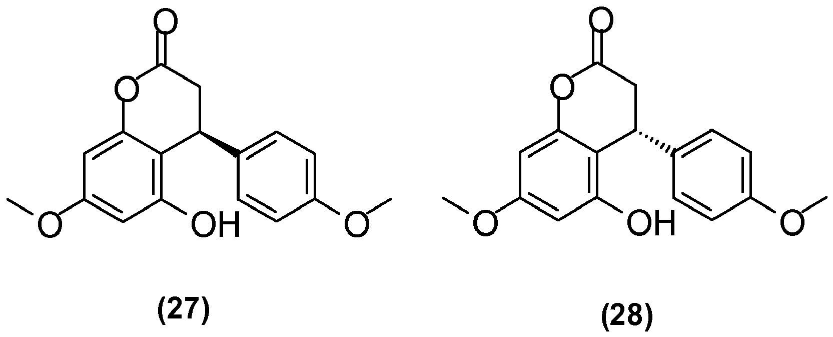 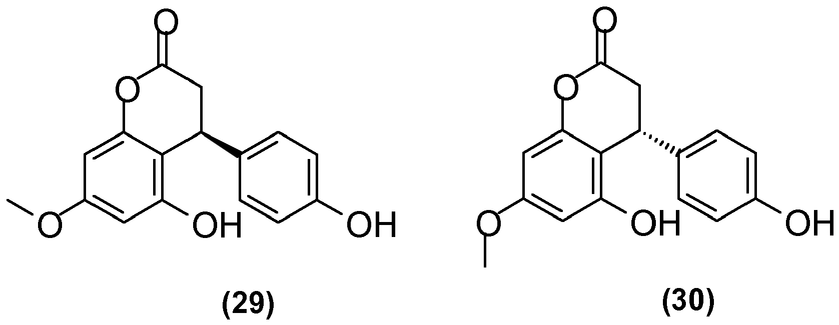 and the physiologically acceptable salts thereof.

5. Use of one, two or more different salts of one, two or more different compounds of formula **(I)** as defined in one of the preceding claims
or
of a mixture of one, two or more different compounds of formula **(I)** as defined in one of the preceding claims with one, two or more different physiologically acceptable salts of one, two or more different compounds of formula **(I)** as defined in one of the preceding claims,
wherein the counter-cation(s) of the or one, several or all of the physiologically acceptable salts is/are preferably selected from the group consisting of Na⁺, K⁺, NH₄⁺, Ca²⁺, Mg²⁺, Al³⁺ and Zn²⁺.

6. Semi-finished product comprising or consisting of the following components:
(a) one or more compounds of formula **(I)** and/or salts thereof as defined in claims 1 to 5,
as well as
one, two, three or more substances selected from the group consisting of the components (b) and (c)
(b) one or more sweet tasting and/or sweet smelling substances, and/or
(c) one or more both sweet and unpleasant, in particular bitter, tasting substances,
as well as
(d) optionally one or more carrier substances suitable for consumption,
(e) optionally one or more further substances for masking or reducing an unpleasant, in particular bitter taste impression,
and
(f) optionally one or more further substances for enhancing a sweet taste impression,
wherein the components (b)-(f) are not or contain neither a compound of formula **(I)** nor a salt of a compound of formula **(I).**

7. Semi-finished product according to claim 6, **characterized in that** the sweet tasting substance(s) of component (b) are selected from the group consisting of
(b1) sweet aroma substances, wherein preferably one, two, three, four, fife or more of the sweet tasting aroma substances are selected from the group consisting of vanillin, ethylvanillin, 2-hydrox-4-methoxybenzaldehyde, ethylvanillin isobutyrate (= 3-ethoxy-4-isobutyryloxy benzaldehyde), Furaneol^{®} (2,5-dimethyl-4-hydroxy-3(2H)-furanone) and derivates thereof (preferably homofuraneol, 2-ethyl-4-hydroxy-5-methyl-3(2H)-furanone), homofuronol (2-ethyl-5-methyl-4-hydroxy-3(2H)-furanone and 5-Ethyl-2-methyl-4-hydroxy-3(2H)-furanone), maltol and derivates thereof (preferably ethylmaltol), cumarin, gamma-lactones (preferably gamma-undecalactone, gamma-nonalactone), delta-lactones (preferably 4-methyldeltalactone, massoia lactone, delta-decalactone, tuberolactone), methylsorbate, divanillin, 4-hydroxy-2(or 5)-ethyl-5(or 2)-methyl-3(2H)furanone, 2-hydroxy-3-methyl-2-cyclopentenone, 3-hydroxy-4,5-dimethyl-2(5H)-furanone, fruit esters and fruit lactones (preferably acetic acid-n-butylester, acetic acid isoamylester, propionic acid ethylester, butyric acid ethylester, butyric acid-n-butylester, butyric acid isoamylester, 3-methyl butyric acid ethylester, n-hexanoic acid ethylester, n- hexanoic acid allylester, n-hexanoic acid n-butylester, n-octanoic acid ethylester, ethyl-3-methyl-3-phenylglycidat, ethyl-2-trans-4-cis-decadienoate), 4-(p-hydroxyphenyl)-2-butanone, 1,1-dimethoxy-2,2,5-trimethyl-4-hexane, 2,6-dimethyl-5-heptene-1-al and phenylacetaldehyde;
(b2) carbohydrates, preferably selected from the group consisting of sucrose, trehalose, lactose, maltose, melizitose, melibiose, raffinose, palatinose, lactulose, D-fructose, D-glucose, D-galactose, L-rhamnose, D-sorbose, D-mannose, D-tagatose, D-arabinose, L-arabinose, D-ribose, D-glyceraldehyde, maltodextrins and plant compositions comprising one or more of said carbohydrates, preferably in an amount of at least 5 wt.-%, preferred at least 15 wt.-%, wherein the carbohydrates may also be present as a naturally occurring or artificially produced mixture, in particular as honey, invert sugar syrup or highly enriched fructose syrup from corn starch, and the physiologically acceptable salts of these carbohydrates, in particular the sodium, potassium, calcium or ammonium salts;
(b3) sugar alcohols, preferably naturally occurring sugar alcohols selected from the group consisting of glycerol, erythritol, threitol, arabitol, ribitol, xylitol, sorbitol, mannitol, maltitol, isomalt, dulcitol, lactitol, and the physiologically acceptable salts of these sugar alcohols, in particular the sodium, potassium, calcium or ammonium salts;
(b4) naturally occurring sweeteners, preferably selected from the group consisting of
(b4-1)miraculin, monellin, mabinlin, thaumatin, curculin, brazzein, pentaidin, D-phenylalanine, D-tryptophan, and extracts or fractions obtained from natural sources containing these amino acids and/or proteins, and the physiologically acceptable salts of these amino acids and/or proteins, in particular the sodium, potassium, calcium or ammonium salts;
(b4-2) neohesperidin dihydrochalcone, naringin dihydrochalcone, stevioside, steviolbioside, rebaudiosides, in particular rebaudioside A, rebaudioside B, rebaudioside C, rebaudioside D, rebaudioside E, rebaudioside F, rebaudioside G, rebaudioside H, dulcoside and rubusoside, suavioside A, suavioside B, suavioside G, suavioside H, suavioside I, suavioside J, baiyunoside 1 baiyunoside 2, phlomisoside 1, phlomisoside 2, phlomisoside 3, as well as phlomisoside 4, abrusoside A, abrusoside B, abrusoside C, abrusoside D, cyclocaryoside A and cyclocaryoside I, oslandine, polypodoside A, strogin 1, strogin 2, strogin 4, selligueanin A, dihydroquercetin-3-acetate, perillartin, telosmosid A₁₅, periandrin I-V, pterocaryoside, cyclocaryoside, mukurozioside, trans-anethol, trans-cinnamaldehyde, bryoside, bryonoside, bryonodulcoside, carnosifloside, scandenoside, gypenoside, trilobatin, phloridzin, dihydroflavanoles, hematoxylin, cyanine, chlorogenic acid, albiziasaponine, telosmoside, gaudichaudioside, mogrosides, mogroside V, hernandulcine, monatin, phyllodulcin, glycyrrhetic acid and derivates thereof, in particular glycosides thereof such as glycyrrhizin, and the physiologically acceptable salts of these compounds, in particular the sodium, potassium, calcium or ammonium salts;
(b4-3) extracts or enriched fractions of the extracts, selected from the group consisting of Thaumatococcus extracts (katemfe shrub), extracts from *Stevia* ssp. (in particular *Stevia rebaudiana*), swingle extracts (*Momordica* or *Siratia grosvenorii*, Luo-Han-Guo), extracts from *Glycerrhyzia* ssp. (in particular *Glycerrhyzia glabra*), extracts from *Rubus* ssp. (in particular *Rubus suavissimus*), citrus extracts and extracts from *Lippia dulcis*; extracts from *Mycetia balansae;*
(b5) synthetic sweet tasting substances, preferably selected from the group consisting of magap, sodium cyclamate or other physiologically acceptable salts of cyclamic acid, acesulfam K or other physiologically acceptable salts, neohesperidin dihydrochalcone, naringin dihydrochalcone, saccharin, saccharin sodium salt, aspartame, superaspartame, neotam, alitam, advantam, perillartin, sucralose, lugduname, carrelame, sucrononate and sucrooctate,
and/or
the both sweet and unpleasant tasting substances of component (c) are selected from the group consisting of steviolglycosides (in particular stevioside and rebaudioside A), rubusoside, dulcoside, mogroside, phyllodulcin, glycyrrhetic acid, extracts from Stevia ssp. (in particular Stevia rebaudiana), Luo Han Guo, Rubus suavissimus, Hydrangea dulcis, Glycyrrhyza glabra, magap, sodium cyclamate, acesulfam K, neohesperidin dihydrochalcone, naringin dihydrochalcone, saccharin, saccharin sodium salt, aspartame, superaspartame, neotam, alitam, sucralose, lugduname, carrelame, sucrononate and sucrooctate.

8. Semi-finished product according to claim 6 or 7, wherein the semi-finished product comprises a total amount of compounds of formula **(I)** and the physiologically acceptable salts thereof as defined in claims 1 to 5 (component (a)) in the range from 0.0001 to 90 wt.-%, preferably in the range from 0.001 to 50 wt.-%, further preferred in the range from 0.01 to 40 wt.-%, particularly preferably in the range from 0.1 to 20 wt.-%, most preferably in the range from 0.2 to 10 wt.-%, with respect to the total weight of the semi-finished product.

9. Preparation, preferably orally consumable preparation, selected from the group consisting of
(1) food, food supplement, oral care or semi-luxury preparations,
(2) cosmetic preparations, in particular for application in the area of the head,
(3) pharmaceutical preparations for oral consumption,
comprising
(i) one or more compounds of formula **(I)** and/or the salts thereof as defined in claims 1 to 5,
and
(ii) one or more further sweet tasting substances, one or more sweet smelling substances and/or one or more both sweet and unpleasant, in particular bitter, tasting substances,
wherein the amount of component (i) in the preparation is sufficient to sensorically enhance the sweet impression of the sweet tasting and/or sweet smelling substance(s) of component (ii).

10. Preparation, preferably orally consumable preparation, according to claim 9 comprising a semi-finished product as defined in claims 6 to 8.

11. Orally consumable preparation according to claim 9 or 10, selected from the group consisting of
(1) food, food supplement, oral care or semi-luxury preparations,
(2) cosmetic preparations for application in the area of the head,
(3) pharmaceutical preparations for oral consumption,
wherein the preparation (1), (2) or (3) with respect to its total weight comprises 0.1 mg/kg to 1 wt.-%, preferably in the range from 0.5 to 1000 mg/kg, preferably in the range from 1 to 500 mg/kg, further preferably in the range from 3 to 300 mg/kg, particularly preferably in the range from 5 to 200 mg/kg, most preferably in the range from 10 to 100 mg/kg, of compound of formula **(I)** and/or salts thereof as defined in claims 1 to 5.

12. Preparation, preferably orally consumable preparation according to one of claims 9 to 11, **characterized in that** the total amount of compounds of formula **(I)** and/or the salts thereof as defined in claims 1 to 5 in the preparation is sufficient to impart an increase of the sweet impression determined in sucrose equivalents by 10% or more, preferably 20% or more, preferred 30% or more and particularly preferred 35% or more compared to a preparation, which, at an otherwise identical composition, does neither comprise a compound of formula **(I)** nor a salt of a compound of formula **(I)** as defined in claims 1 to 5.

13. Method for producing
- a semi-finished product as defined in claims 6 to 8
or
- a preparation as defined in claims 9 to 12,
comprising the steps
(1) producing or providing the following components:
(a) one or more compounds of formula **(I)** and/or the salts thereof as defined claims 1 to 5,
as well as
one, two three or more substances selected from the group consisting of components (b) and (c)
(b) one or more sweet tasting and/or sweet smelling substances,
and/or
(c) one or more both sweet and unpleasant, in particular bitter, tasting substances,
as well as
(d) optionally one or more carrier substances suitable for consumption,
(e) optionally one or more further substances for masking or reducing an unpleasant, in particular bitter taste impression,
and
(f) optionally one or more further substances for enhancing a sweet taste impression,
wherein the components (b)-(f) are not or contain neither a compound of formula **(I)** nor a salt of a compound of formula **(I)**,
and
(2) mixing of component (a) with one or more of the components (b) and/or (c) as well as optionally (d) as well as optionally component (e) and optionally component (f).

14. Method for influencing the strength of a sensory impression, in particular a sweet impression, of a sweet tasting and/or sweet smelling substance or substance mixture or of a both sweet and unpleasant, in particular bitter, tasting substance or substance or substance mixture, with the following step:
mixing of component (a)
(a) one or more compounds of formula **(I)** and/or the salts thereof as defined in claims 1 to 5
with component (b) and/or (c)
(b) one or more sweet tasting and/or sweet smelling substances, and/or
(c) one or more both sweet and unpleasant, in particular bitter, tasting substances,
wherein the components (b) and (c) are not or contain neither a compound of formula **(I)** nor a salt of a compound of formula **(I)**,
wherein the total amount of component (a) in the mixture is sufficient to influence the strength of the sensory impression, in particular the sweet impression, of the sweet tasting and/or sweet smelling substances of component (b) or of the both sweet and unpleasant, in particular bitter, tasting substances of component (c).

## Revendications

1. Utilisation d'un, de deux ou de plusieurs composés différents de la formule **(I)** 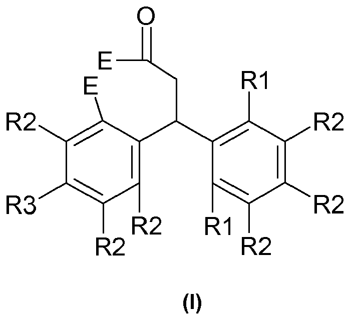 ou
d'un, de deux ou de plusieurs sels physiologiquement acceptables différents d'un, de deux ou de plusieurs composés différents de la formule **(I),**
ou
d'un mélange d'un, de deux ou de plusieurs composés différents de la formule **(I)** avec un, deux ou plusieurs sels physiologiquement acceptables différents d'un, de deux ou de plusieurs composés différents de la formule **(I),**
dans laquelle
soit E représente chacun OH soit les deux E représentent ensemble un O,
R1, indépendamment de l'autre radical R1 respectivement, représente de l'hydrogène ou OR^{a}, où R^{a} est de l'hydrogène, de l'alkyle en C1-C5 ou de l'alkényle en C2-C5,
R2, indépendamment des autres radicaux R2, représente de l'hydrogène ou OR^{b}, où R^{b} est de l'hydrogène, de l'alkyle en C1-C5 ou de l'alkényle en C2-C5,
le cas échéant, deux radicaux R1 et/ou R2 immédiatement voisins représentant ensemble un groupe OCH₂O,
et
R3 représente un radical OR^{x}, où R^{x} est de l'alkyle en C1-C5 ou de l'alkényle en C2-C5,
pour produire une impression sucrée dans une préparation consommable oralement ou pour influencer l'intensité de l'impression sucrée de substances ou mélanges de substances sucrés ou de substances ou mélanges de substances ayant un goût aussi bien sucré que désagréable, de préférence amer.

2. Utilisation selon la revendication 1, dans laquelle un, deux, plusieurs ou l'ensemble des composés mis en oeuvre sont choisis chacun dans le groupe constitué par les composés de la formule **(I)** et les sels physiologiquement acceptables de ceux-ci, où
E représente chacun OH ou les deux E représentent ensemble de l'oxygène, R1, indépendamment de l'autre radical R1 respectivement, représente de l'hydrogène ou hydroxy,
R2, indépendamment des autres radicaux R2, représente de l'hydrogène, hydroxy, méthoxy, éthoxy, n-propoxy, iso-propoxy, n-butoxy, iso-butoxy, tert-butoxy ou OR^{b}, où R^{b} est de l'alkényle en C5,
le cas échéant, deux radicaux R2 immédiatement voisins représentant ensemble un groupe OCH₂O,
R3 représente méthoxy, éthoxy, n-propoxy, iso-propoxy, n-butoxy, iso-butoxy, tert-butoxy ou OR^{c}, où R^{c} est de l'alkényle en C5,
de préférence, un ou plusieurs des radicaux R1 ou R2 représentant un groupe hydroxy.

3. Utilisation telle que définie dans l'une quelconque des revendications précédentes, dans laquelle un, deux, plusieurs ou l'ensemble des composés de la formule **(I)** mis en oeuvre sont choisis chacun dans le groupe des composés de la formule **(II)** 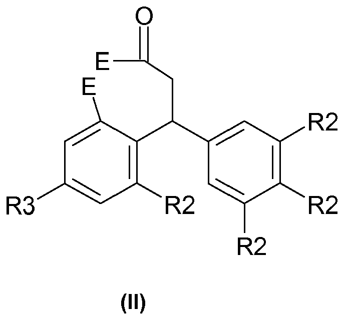 dans laquelle
E représente chacun OH ou les deux E représentent ensemble de l'oxygène,
R2, indépendamment des autres radicaux R2, représente de l'hydrogène, hydroxy, méthoxy, éthoxy, n-propoxy ou iso-propoxy, de préférence H, OH, OCH₃ ou OCH₂CH₃,
le cas échéant, deux radicaux R2 immédiatement voisins représentant ensemble un groupe OCH₂O,
R3 représente méthoxy, éthoxy, n-propoxy, iso-propoxy, n-butoxy, iso-butoxy, tert-butoxy ou OR^{c}, où R^{c} est de l'alkényle en C5,
et leurs sels physiologiquement acceptables.

4. Utilisation telle que définie dans l'une quelconque des revendications précédentes, dans laquelle un, deux, plusieurs ou l'ensemble des composés de la formule **(I)** mis en oeuvre sont choisis chacun dans le groupe constitué par 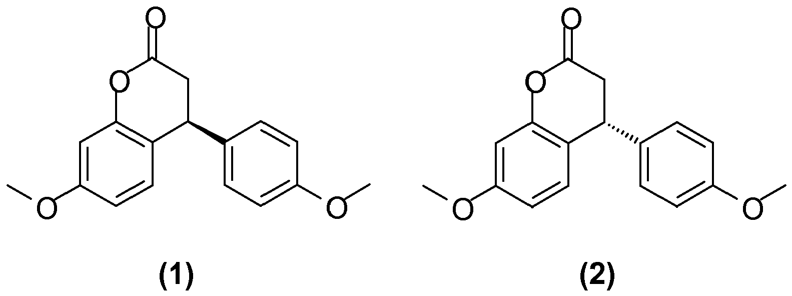 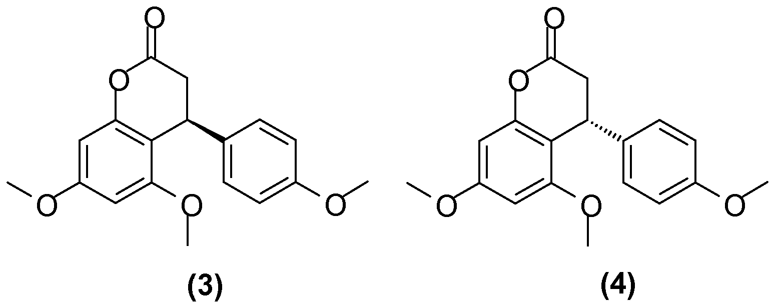 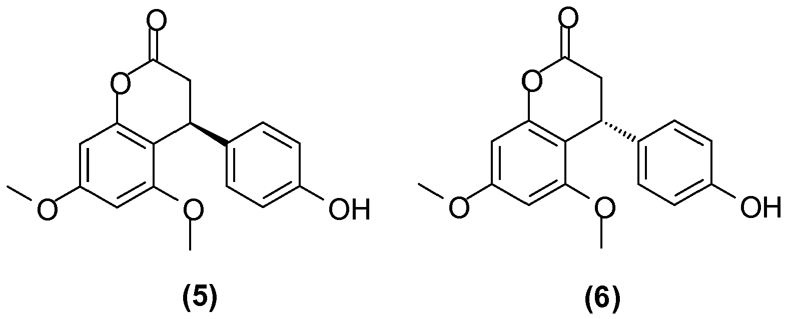 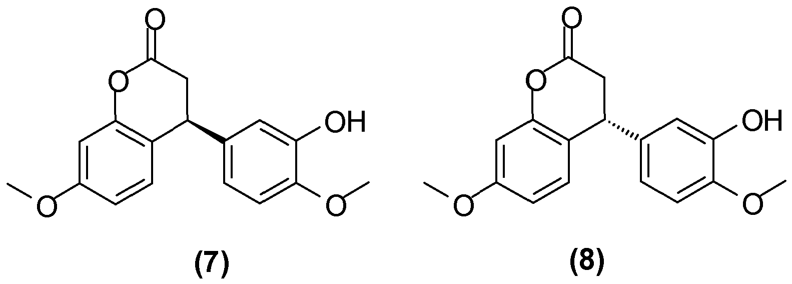 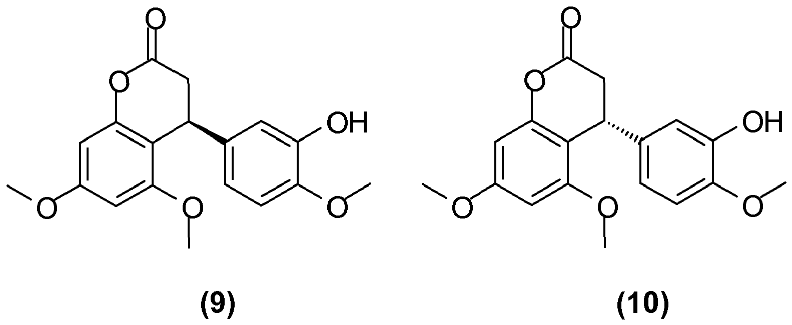 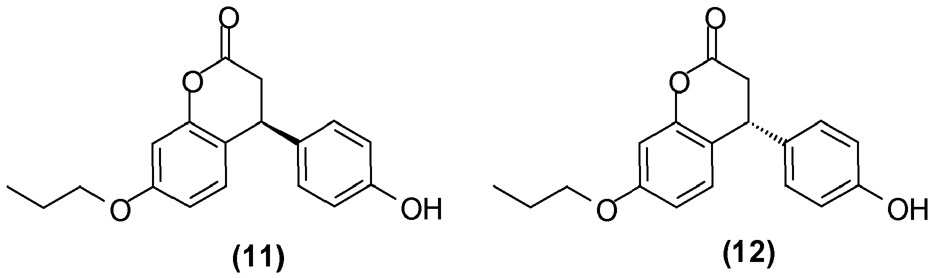 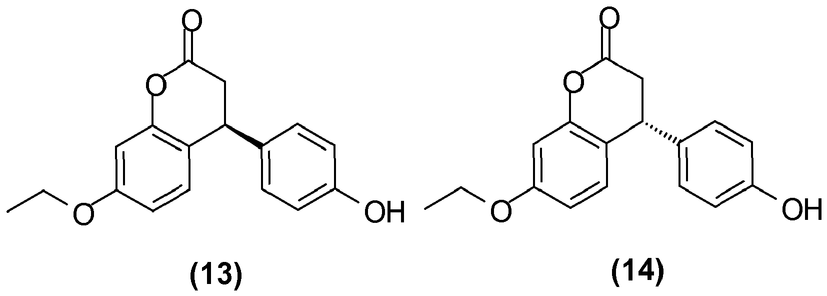 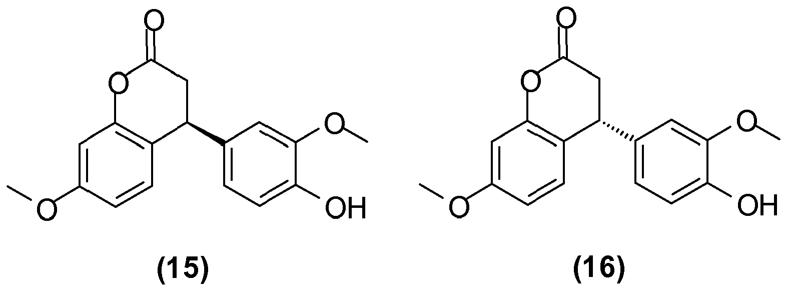 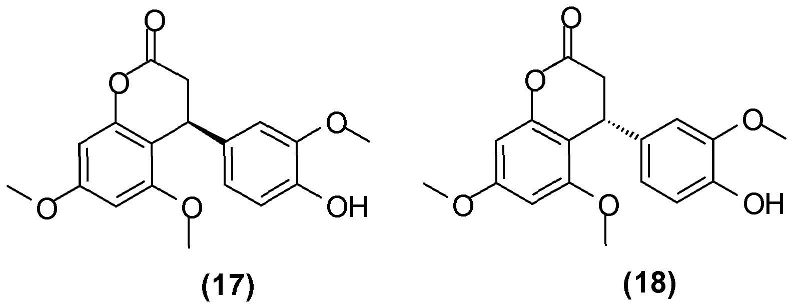 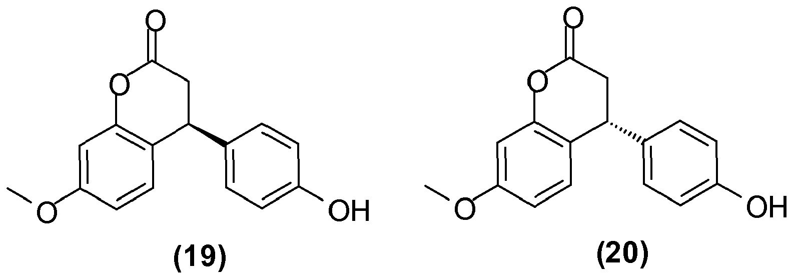 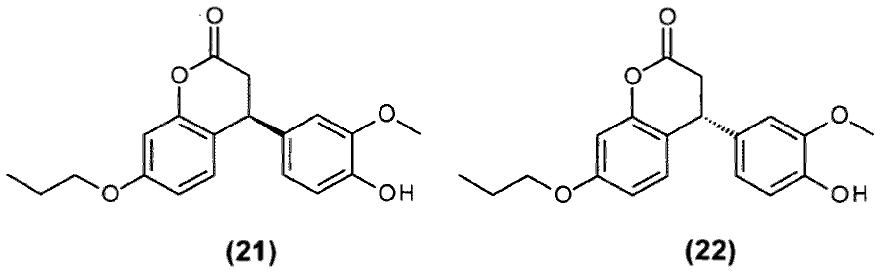 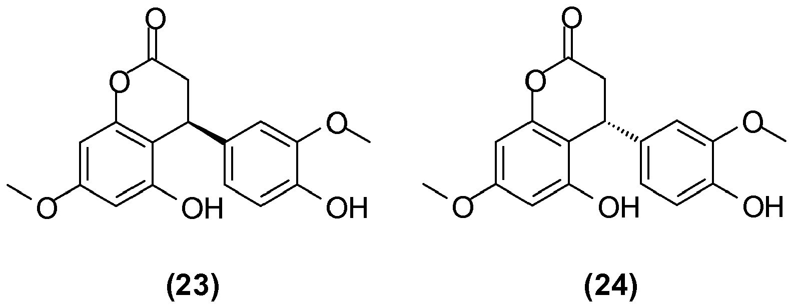 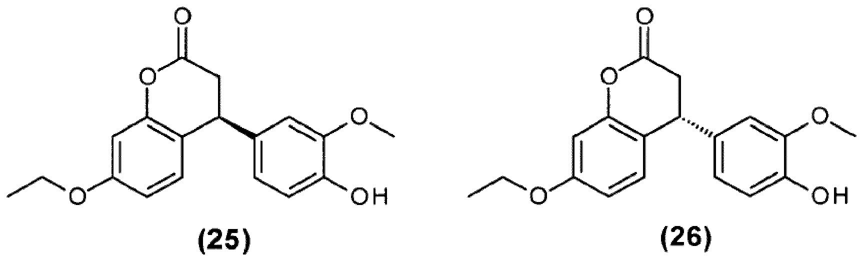 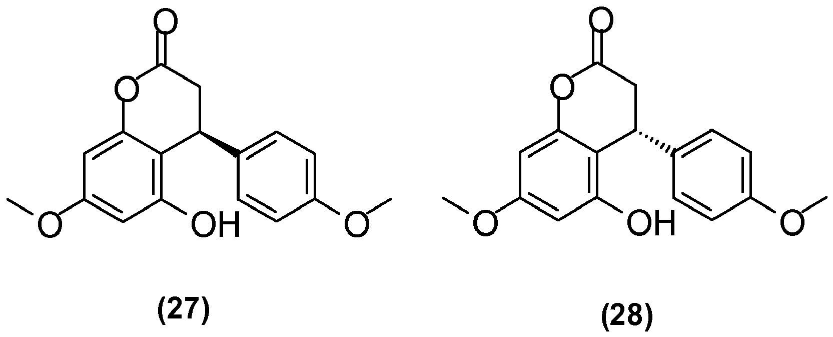 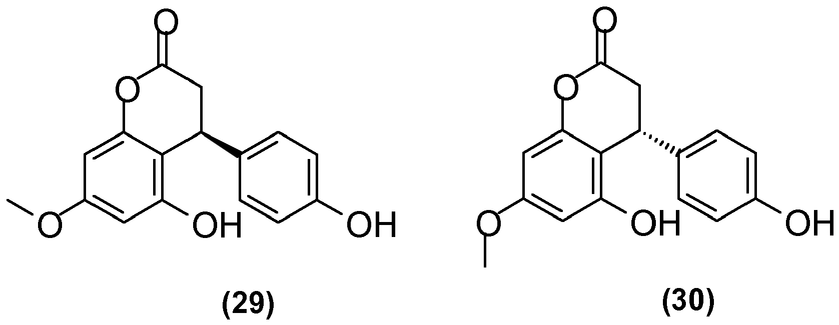 et leurs sels physiologiquement acceptables.

5. Utilisation d'un, de deux ou de plusieurs sels différents d'un, de deux ou de plusieurs composés différents de la formule **(I)** tels que définis dans l'une quelconque des revendications précédentes
ou
d'un mélange d'un, de deux ou de plusieurs composés différents de la formule **(I)** tels que définis dans l'une quelconque des revendications précédentes, avec un, deux ou plusieurs sels physiologiquement acceptables différents d'un, de deux ou de plusieurs composés différents de la formule **(I)** tels que définis dans l'une quelconque des revendications précédentes,
le ou bien les contre-cations du ou bien d'un, de plusieurs ou de l'ensemble des sels physiologiquement acceptables est ou bien sont choisi(s) de préférence dans le groupe constitué par Na⁺, K⁺, NH₄⁺, Ca²⁺, Mg²⁺ , Al³⁺ et Zn²⁺.

6. Produit semi-fini comprenant les ou se composant des composants suivants:
(a) un ou plusieurs composés de la formule **(I)** et/ou leurs sels tels que définis dans les revendications 1 à 5,
ainsi que
une, deux, trois ou plusieurs substances choisies dans le groupe constitué par les composants (b) et (c)
(b) une ou plusieurs substances ayant un goût sucré et/ou une odeur sucrée et/ou
(c) une ou plusieurs substances ayant un goût aussi bien sucré que désagréable, en particulier amer,
ainsi que,
(d) le cas échéant, un ou plusieurs excipients aptes à être consommés,
(e) le cas échéant, une ou plusieurs autres substances destinées à masquer ou à réduire une impression gustative désagréable, en particulier amère,
et,
(f) le cas échéant, une ou plusieurs autres substances destinées à renforcer une impression gustative sucrée,
les composants (b)-(f) n'étant ou bien ne contenant ni un composé de la formule **(I)** ni un sel d'un composé de la formule **(I).**

7. Produit semi-fini selon la revendication 6, **caractérisé par le fait que** la ou les substances du composant (b) qui ont un goût sucré sont choisies dans le groupe constitué par
(b1) les aromatisants sucrés, de préférence un, deux, trois, quatre, cinq ou plusieurs des aromatisants sucrés étant choisis dans le groupe constitué par la vanilline, l'éthylvanilline, le 2-hydrox-4-méthoxybenzaldéhyde, l'isobutyrate d'éthylvanilline (= 3-éthoxy-4-isobutyryloxybenzaldéhyde), le furanéol^{®} (2,5-diméthyl-4-hydroxy-3(2H)-furanone) et ses dérivés (de préférence homofuranéol, 2-éthyl-4-hydroxy-5-méthyl-3(2H)-furanone), l'homofuronol (2-éthyl-5-méthyl-4-hydroxy-3(2H)-furanone et 5-éthyl-2-méthyl-4-hydroxy-3(2H)-furanone), le maltol et ses dérivés (de préférence maltol éthylique), la cumarine, les gamma-lactones (de préférence gamma-undécalactone, gammanonalactone), les delta-lactones (de préférence 4-méthyldeltalactone, massoilactone, deltadécalactone, tubérolactone), le méthyl sorbate, la divanilline, le 4-hydroxy-2(ou 5)-éthyl-5(ou 2)méthyl-3(2H)-furanone, le 2-hydroxy-3-méthyl-2-cyclopenténone, le 3-hydroxy-4,5-diméthyl-2(5H)-furanone, les esters de fruit et les lactones de fruit (de préférence ester n-butylique de l'acide acétique, ester isoamylique de l'acide acétique, ester éthylique de l'acide propionique, ester éthylique de l'acide butyrique, ester n-butylique de l'acide butyrique, ester isoamylique de l'acide butyrique, ester éthylique de l'acide 3-méthylbutyrique, ester éthylique de l'acide n-hexanoïque, ester allylique de l'acide n-hexanoïque, ester n-butylique de l'acide n-hexanoïque, ester éthylique de l'acide n-octanoïque, éthyl-3-méthyl-3-phénylglycidate, éthyl-2-trans-4-cis-décadiénoate), le 4-(p-hydroxyphényl)-2-butanone, le 1,1-diméthoxy-2,2,5-triméthyl-4-hexane, le 2,6-diméthyl-5-heptène-1-al et le phénylacétaldéhyde;
(b2) les hydrates de carbone, de préférence choisis dans le groupe constitué par le saccharose, le tréhalose, le lactose, le maltose, le mélézitose, la mélibiose, le raffinose, le palatinose, le lactulose, le D-fructose, le D-glucose, le D-galactose, le L-rhamnose, le D-sorbose, le D-mannose, le D-tagatose, le D-arabinose, le L-arabinose, le D-ribose, le D-glycéraldéhyde, la maltodextrine et les préparations végétales contenant un ou plusieurs desdits hydrate(s) de carbone, de préférence dans une proportion d'au moins 5 % en poids, de préférence d'au moins 15 % en poids, les hydrates de carbone pouvant être présents également en tant que mélange présent naturellement ou préparé artificiellement, dans ce cas en particulier comme miel, sirop de sucre inverti ou sirop de fructose hautement enrichi à partir de l'amidon de maïs, et les sels physiologiquement acceptables de ces hydrates de carbone, en particulier les sels de sodium, de potassium, de calcium ou d'ammonium ;
(b3) les alcools de sucre, de préférence les alcools de sucre présents naturellement, choisis dans le groupe constitué par la glycérine, l'érythritol, le thréitol, l'arabitol, le ribitol, le xylitol, le sorbitol, le mannitol, le maltitol, l'isomaltitol, le dulcitol, le lactitol, et les sels physiologiquement acceptables de ces alcools de sucre, en particulier les sels de sodium, de potassium, de calcium ou d'ammonium ;
(b4) les édulcorants présents naturellement, de préférence choisis dans le groupe constitué par
(b4-1) la miraculine, la monelline, la mabinline, la thaumatine, la curculine, la brazzéine, la pentaidine, la D-phénylalanine, le D-tryptophane, et les extraits ou fractions obtenus à partir de sources naturelles, contenant ces acides aminés et/ou protéines, et les sels physiologiquement acceptables de ces acides aminés et/ou protéines, en particulier les sels de sodium, de potassium, de calcium ou d'ammonium ;
(b4-2) n la néohespéridine dihydrochalcone, la naringine dihydrochalcone, le stévioside, le stéviolbioside, les rébaudiosides, en particulier le rébaudioside A, le rébaudioside B, le rébaudioside C, le rébaudioside D, le rébaudioside E, le rébaudioside F, le rébaudioside G, le rébaudioside H, les dulcosides et le rubusoside, le suavioside A, le suavioside B, le suavioside G, le suavioside H, le suavioside I, le suavioside J, le baiyunoside 1, le baiyunoside 2, le phlomisoside 1, le phlomisoside 2, le phlomisoside 3 ainsi que le phlomisoside 4, l'abrusoside A, l'abrusoside B, l'abrusoside C, l'abrusoside D, le cyclocaryoside A et le cyclocaryoside I, l'oslandine, le polypodoside A, la strogine 1, la strogine 2, la strogine 4, la selliguéanine A, le 3-acétate de dihydroquercétine, la périllartine, le télosmoside A₁₅, la périandrine I-V, les ptérocaryosides, les cyclocaryosides, les mukuroziosides, le trans-anéthol, le trans-cinnamaldéhyde, les bryosides, les bryonosides, les bryonodulcosides, les carnosiflosides, les scandénosides, les gypénosides, la trilobatine, la phloridzine, les dihydroflavanols, l'hématoxyline, la cyanine, l'acide chlorogénique, l'albiziasaponine, les télosmosides, le gaudichaudioside, les mogrosides, le mogroside V, les hernandulcines, la monatine, la phyllodulcine, l'acide glycyrrhétinique et ses dérivés, en particulier ses glycosides tels que la glycyrrhizine, et les sels physiologiquement acceptables de ces composés, en particulier les sels de sodium, de potassium, de calcium ou d'ammonium ;
(b4-3) les extraits ou fractions enrichies des extraits, choisis dans le groupe constitué par les extraits de Thaumatococcus (*katemfe*), les extraits de *Stevia ssp.* (en particulier *Stevia rebaudiana*), les extraits de swingle (*Momordica* ou bien *Siratia grosvenorii*, Luo-Han-Guo), les extraits de *Glycerrhyzia ssp.* (en particulier *Glycerrhyzia glabra*), les extraits de *Rubus ssp.* (en particulier *Rubus suavissimus*), les extraits de Citrus et les extraits de *Lippia dulcis*, les extraits de *Mycetia balansae*;
(b5) les substances synthétiques ayant un goût sucré, de préférence choisies dans le groupe constitué par le magap, le cyclamate de sodium ou d'autres sels physiologiquement acceptables de l'acide cyclamique, l'acésulfame K ou d'autres sels physiologiquement acceptables, la néohespéridine dihydrochalcone, la naringine dihydrochalcone, la saccharine, le sel de sodium de saccharine, l'aspartame, le superaspartame, le néotame, l'alitame, l'advantame, la périllartine, le sucralose, le lugduname, le carrélame, le sucrononate et le sucrooctate,
et/ou
les substances du composant (c) qui ont un goût aussi bien sucré que désagréable sont choisies dans le groupe constitué par les stéviolglycosides (en particulier le stévioside et le rébaudioside A), le rubusoside, le dulcoside, le mogroside, la phyllodulcine, l'acide glycyrrhétinique, les extraits de Stevia ssp. (en particulier Stevia rebaudiana), Luo Han Guo, Rubus suavissimus, Hydrangea dulcis, Glycyrrhyza glabra, le magap, le cyclamate de sodium, l'acésulfame K, la néohespéridine dihydrochalcone, la naringine dihydrochalcone, la saccharine, le sel de sodium de saccharine, l'aspartame, le superaspartame, le néotame, l'alitame, le sucralose, le lugduname, le carrélame, le sucrononate et le sucrooctate.

8. Produit semi-fini selon la revendication 6 ou 7, le produit semi-fini comprenant une quantité totale de composés de la formule **(I)** et de leurs sels physiologiquement acceptables, tels que définis dans les revendications 1 à 5 (composant (a)), qui se situe dans la plage allant de 0,0001 à 90 % en poids, de préférence dans la page allant de 0,001 à 50 % en poids, encore de préférence dans la page allant de 0,01 à 40 % en poids, de manière particulièrement préférée dans la page allant de 0,1 à 20 % en poids, de manière la plus préférée dans la page allant de 0,2 à 10 % en poids, par rapport au poids total du produit semi-fini.

9. Préparation, de préférence préparation consommable oralement, choisie dans le groupe constitué par
(1) les préparations servant à la nourriture, de complément alimentaire, à l'hygiène buccale ou à la consommation,
(2) les préparations cosmétiques, en particulier destinées à être appliquées au niveau de la tête,
(3) les préparations pharmaceutiques destinées à être prises oralement, comprenant
(i) un ou plusieurs composés de la formule **(I)** et/ou leurs sels tels que définis dans les revendications 1 à 5,
et
(ii) une ou plusieurs autres substances ayant un goût sucré, une ou plusieurs substances ayant une odeur sucrée et/ou une ou plusieurs substances ayant un goût aussi bien sucré que désagréable, en particulier amer,
la quantité de composant (i) dans la préparation étant suffisante afin de renforcer sensoriellement l'impression sucrée de la ou bien des substances du composant (ii) qui ont un goût sucré et/ou une odeur sucrée.

10. Préparation, de préférence préparation consommable oralement, selon la revendication 9, comprenant un produit semi-fini tel que défini dans les revendications 6 à 8.

11. Préparation consommable oralement, selon la revendication 9 ou 10, choisie dans le groupe constitué par
(1) les préparations servant à la nourriture, de complément alimentaire, à l'hygiène buccale ou à la consommation,
(2) les préparations cosmétiques destinées à être appliquées au niveau de la tête,
(3) les préparations pharmaceutiques destinées à être prises oralement,
la préparation (1), (2) ou (3) comprenant, par rapport à son poids total, de 0,1 mg/kg à 1 % en poids, de préférence entre 0,5 et 1000 mg/kg, de préférence entre 1 et 500 mg/kg, encore de préférence entre 3 et 300 mg/kg, de manière particulièrement préférée entre 5 et 200 mg/kg, de manière la plus préférée entre 10 et 100 mg/kg de composés de la formule **(I)** et/ou de leurs sels tels que définis dans les revendications 1 à 5.

12. Préparation, de préférence préparation consommable oralement, selon l'une quelconque des revendications 9 à 11, **caractérisée par le fait que** la quantité totale, dans la préparation, de composés de la formule **(I)** et/ou de leurs sels tels que définis dans les revendications 1 à 5, est suffisante afin de conférer une augmentation de l'impression sucrée à déterminer en équivalents de saccharose, de 10 % ou plus, de préférence de 20 % ou plus, de préférence de 30 % ou plus et de manière particulièrement préférée de 35 % ou plus, en comparaison d'une préparation qui, avec une composition pour le reste identique, ne comprend ni un composé de la formule **(I)** ni un sel d'un composé de la formule **(I)** tel que défini dans les revendication 1 à 5.

13. Procédé de fabrication
- d'un produit semi-fini tel que défini dans les revendications 6 à 8
ou bien
- d'une préparation telle que définie dans les revendications 9 à 12,
comprenant les étapes
(1) préparer ou fournir les composants suivants:
(a) un ou plusieurs composés de la formule **(I)** et/ou leurs sels tels que définis dans les revendications 1 à 5,
ainsi qu'
une, deux, trois ou plusieurs substances choisies dans le groupe constitué par les composants (b) et (c),
(b) une ou plusieurs substances ayant un goût sucré et/ou une odeur sucrée,
et/ou
(c) une ou plusieurs substances ayant un goût aussi bien sucré que désagréable, en particulier amer,
ainsi que,
(d) le cas échéant, un ou plusieurs excipients aptes à être consommés,
(e) le cas échéant, une ou plusieurs autres substances destinées à masquer ou à réduire une impression gustative désagréable, en particulier amère,
et
(f) le cas échéant, une ou plusieurs autres substances destinées à renforcer une impression gustative sucrée,
les composants (b)-(f) n'étant ou bien ne contenant ni un composé de la formule **(I)** ni un sel d'un composé de la formule **(I),**
et
(2) mélanger le composant (a) avec un ou plusieurs des composants (b) et/ou (c) ainsi que, le cas échéant, (d) ainsi que, le cas échéant, le composant (e) et, le cas échéant, le composant (f).

14. Procédé destiné à influencer l'intensité d'une impression sensorielle, en particulier de l'impression sucrée, d'un(e) substance ou mélange de substances ayant un goût sucré et/ou une odeur sucrée ou d'un(e) substance ou mélange de substances ayant un goût aussi bien sucré que désagréable, en particulier amer, comprenant l'étape suivante :
mélanger le composant (a)
(a) un ou plusieurs composés de la formule **(I)** et/ou leurs sels tels que définis dans les revendications 1 à 5,
avec le composant (b) et/ou (c)
(b) une ou plusieurs substances ayant un goût sucré et/ou une odeur sucrée, et/ou
(c) une ou plusieurs substances ayant un goût aussi bien sucré que désagréable, en particulier amer,
les composants (b) et (c) n'étant ou bien ne contenant ni un composé de la formule **(I)** ni un sel d'un composé de la formule **(I),**
la quantité totale de composant (a) dans le mélange étant suffisante pour influencer l'intensité de l'impression sensorielle, en particulier de l'impression sucrée, de la ou des substance(s) du composant (b) qui ont un goût sucré et/ou une odeur sucrée, ou bien de la ou des substances du composant (c) qui ont un goût aussi bien sucré que désagréable, en particulier amer.
